# EUROPÄISCHE PATENTSCHRIFT

(11) **EP 2 882 858 B1**
(45) Veröffentlichungstag und Bekanntmachung des Hinweises auf die Patenterteilung: **10.10.2018**
(21) Anmeldenummer: 13759425.5
(22) Anmeldetag: 06.08.2013
(51) Int. Cl.: C12N 15/82

(54) **TRANSGENE PFLANZE DER ART SOLANUM TUBEROSUM MIT RESISTENZ GEGENÜBER PHYTOPHTHORA**
TRANSGENIC PLANT OF THE SPECIES SOLANUM TUBEROSUM WITH RESISTANCE TO PHYTOPHTHORA
PLANTE TRANSGÉNIQUE DE L'ESPÈCE SOLANUM TUBEROSUM PRÉSENTANT UNE RÉSISTANCE À PHYTOPHTHORA

(30) Priorität: 08.08.2012 DE 102012016009
(43) Veröffentlichungstag der Anmeldung: 17.06.2015
(73) Patentinhaber: KWS SAAT SE, 37574 Einbeck (DE)
(72) Erfinder: STAHL, Dietmar, Jürgen, 37574 Einbeck (DE); TEMME, Nora, 48146 Münster (DE)
(86) Internationale Anmeldenummer: PCT/DE2013/000446
(87) Internationale Veröffentlichungsnummer: WO 2014/023285

(56) Entgegenhaltungen:
- WO-A2-2006/047495
- WO-A2-2009/112270
- US-A1- 2011 167 514
- DATABASE EMBL [Online] 28. Januar 2005 (2005-01-28), "PE006G1 mycelium, carbon starvation Phytophthora infestans cDNA, mRNA sequence.", XP002717637, gefunden im EBI accession no. EM_EST:CV909836 Database accession no. CV909836
- PIETER VAN WEST ET AL: "Internuclear Gene Silencing in Phytophthora infestans", MOLECULAR CELL, Bd. 3, Nr. 3, 1. März 1999 (1999-03-01), Seiten 339-348, XP055092086, ISSN: 1097-2765, DOI: 10.1016/S1097-2765(00)80461-X in der Anmeldung erwähnt
- KOCH A ET AL: "New wind in the sails: improving the agronomic value of crop plants through RNAi-mediated gene silencing", PLANT BIOTECHNOLOGY JOURNAL, BLACKWELL PUB, GB, vol. 12, no. 7, 1 September 2014 (2014-09-01), pages 821-831, XP002738838, ISSN: 1467-7644, DOI: 10.1111/PBI.12226 [retrieved on 2014-07-18]
- LOKE S L ET AL: "CHARACTERIZATION OF OLIGONUCLEOTIDE TRANSPORT INTO LIVER CELLS", PROCEEDINGS OF THE NATIONAL ACADEMY OF SCIENCES OF THE UNITED STATES OF AMERICA, vol. 86, no. 10, 1989, pages 3474-3478, ISSN: 0027-8424
- CATALANOTTO C ET AL: "Involvement of small RNAs and role of the qde genes in the gene silencing pathway in Neurospora", GENES AND DEVELOPMENT, COLD SPRING HARBOR LABORATORY PRESS, PLAINVIEW, NY, US, vol. 16, no. 7, 1 April 2002 (2002-04-01), pages 790-795, XP002278529, ISSN: 0890-9369, DOI: 10.1101/GAD.222402

### SEQUENCE LISTING

<110> KWS SAAT AG
<120> TRANSGENE PFLANZE DER ART SOLANUM TUBEROSUM MIT
   RESISTENZ GEGENÜBER PHYTOPHTHORA
<130> KWS 0199 DE
<160> 43
<170> PatentIn Version 3.5
<210> 1
   <211> 1042
   <212> DNA
   <213> Phytophthora infestans
<400> 1
   cgtcaagatg ctgcggaggt ctgtgctgcc gttggcacgt cgtgccttcc cgcggtccgc      60
   ggctagccta gcgccgtcct gtgctgctcc caagtacttt tcacgtgcct tcagcgttgc     120
   ggagcagagt cagcggcatg tgatcgccgc gctggttgtc aaccagcctg gttgtctggc     180
   cgagatcgcc aacctcttcg ccgctcgagg taactatcga tacgcaaaga cgctggccac     240
   aggagatcct aaacatggtg tgatgcaggc tacaatattg acagtctggt tgtgggtcgc     300
   acggaggtcg aggagctctc ccgtatgacg gttgtcgtca acggcactgc gcagagtgtt     360
   gtcaacgtac gtatagtaat tacagagaca ggagcagtag gtgattgaca ctcaatgtgt     420
   ctttgcagat gaagaagcag ctcgaggatg tcgtgtatgt tgctgtggtc aatatcctga     480
   gcagtggcaa gaacgctgaa aagaactacg tcgagcgcga cctgatgctg gccaaggtgt     540
   ccacggccgg tacgtcggac gctgatctga taccctacca ccaccggaat tcgtgaacat     600
   ggacctgacg taaacatttg ctggtgtgtg tgctggctac agaggctgga tctcgcgctg     660
   aggtggtgga gcttgccaac ctgttcgacg ccaaggtgat tgacgtgcga ccgcaccagg     720
   ttatggtaca actggctggc acccctggtc gcattgaggc atttttggac ctgctaaagc     780
   cgctgggtat cacagagatc caccgcagtg gggtcattgc gatggctcgc agcaccagtg      840
   tgaccgacga cctgggcgat ctctcgacgt ttgagggcgc cacgcgaacg cttctggacg     900
   aggccgatga cgaagagttc gatgtctctc gccttccgcc tggataatat gttggtagag     960
   ttatgatcat gccatcacct ttttcttcga taggacttgt gcctgatgac aattgcagca    1020
   aggaacgtgc tagcgaagat tg                                             1042
<210> 2
   <211> 3072
   <212> DNA
   <213> Phytophthora infestans
<400> 2
   atggtgctcc gagcggttcg gctgatcgtc caggcatctc tgctcctgca agtgctccag      60
   tgtggcgcct ccgtaactgg tgctcaagta aatgaaaact ttgagaacac tgagcttacc     120
   tcgaacgata aactgggaac agtggctccg gccgacatcc cgtcttcaca agacgagaat     180
   ctcaagaaac aggaagagcg tggcttcttt gactggtttg gcaatggcga cgacacgcct     240
   gccccggctg ctgacgacaa ctccggtaaa tatgcgacta cgacaccgat ccctggcacg     300
   gctgcgcggc cgtctacgag tagtctgatg tcaaagtatg gtagtatgct cggtgacttt     360
   actaaagaca cgacgccggc ctcggaagcc gatgagcgca caacgagcgc acgtagcgct     420
   gccacccgtg ccacctcgga ctcctcttca ggtagtacct ctggcagcgc ggcagcagaa     480
   gtcaagccga agagaaagaa gaccaagaag cctgtggttg ccgccgcgtc tgaaagcgga     540
   gagagtgcaa gtgacgacga tgccagcgag gcgagcgctt cgggcagtga ggcaaagccc     600
   aagaagagga caaccaagaa gacgaggaag ccggtcgtag ctgctgcgtc gacgtctgaa     660
   tccgaggatg cctcgacgtc tgaatccgag gatgcttcgg gcagtgaggc aaagcctaag     720
   aagaagacaa ccaagaagac tagaaggccg gtagcagctg ctgcatcgac gtctgaatcg     780
   gaggatgctt cgggcagctc ggacgagtcg ggaagcgacg atctgtcgga tctgctcggc     840
   agctcggcgg gttccggaag cgaagatgac atgtcggccc ttctcggtag cgcgggaggt 900
   tccgggactg acgacgcctt atcagctctg ctggggtcgg gagtcggtgg ctcgggtagt 960
   atgcttagtt tcgaagactt tatgaagaat tacggtagca tgttcggttc gggaagtagt 1020
   gctgtcgacc tgttcggaga cccgagcacg ccccagaaca acacgattga cgacgaagat 1080
   atcatcctcg gcgaactgta cggtggcaag gagcacggcg acgccttctc ggacatcagg 1140
   aacatcaagt ttggtcagat gatcctgaac attacagttc gcggtcaaga gcgcgtggat 1200
   tccattggta ttacagtgat gactcaagaa gctgttggta accttgtgca cggtggtgaa 1260
   ggtggtaccg aaggattcat tgaaccggag atgggtgaca ctattgatac cgtcgaagta 1320
   cactgggaca agaacaaggg caagacgtgc atcttctacc tcaagatggc gacttctggc 1380
   ggtaagacga ttgcgacggg aacgaagacg gccaacagcg ccgtcatcaa gccgcccaag 1440
   ggctaccagc ttgctggatt ccatggtcgt gccagtagtt ctggtatctt ttgtattggt 1500
   ggaatcttca ccaagcaaga cgcgacggat ctcgcagtca cggacgtgat ggccatctcc 1560
   agtaagggct ctcctgatat ctacaactac gacaccacca ttcgtaactg ggtgggacct 1620
   ctagagacag cgagtgacaa cgcctgttac cagaagagag tcgacgtcag cagcaaggga 1680
   atgtgtccgt cgggtttcaa caaggacgac gacaggtgca tcacccaatg tcctctcaac 1740
   taccccattg actgcttgat ggagtgcatg cctcaaaaca gtgactgcac ccagttgatt 1800
   gtcgctaagg tttccgccgt cgttgctgtc gctttaaatg ccgctacgat gggtatcttc 1860
   ggtacgctgg tggctgccta cagaaccgct aactttgctc tcacctgcgc catcaacgtt 1920
   gtgaacgctg tcaagtcgct gatttactac ctgcgttaca agcagacctt gatcccgact 1980
   acggacacgg agaagttgat ggacaaggcg ttccagctgc agattgtcat tcttgacttg 2040
   cctctggcta tctgttcttg cctgggcatc aagattcctc ctaagctcca gttctcggct 2100
   accattctgg ctgtcgtgtc ggccattgtc atgatggctg tcatggtcgg tgaggccctc 2160
   ttcgcgtcgt cgaacaacgt catgctcatg cttcgtgagt cgggcgcttt taacacttct 2220
   gctctgaacg gagacaccat tgagcttgat acgttcctca acaccaagaa cggcacgtgt 2280
   ggttacgaga tgagaactct cactaaccgc gtcatgggca aggtctacga aatccgtaac 2340
   aacacgccga atgctgatgc cgatgatgta cgtgttgagg tgagcaagtc gtccatcatt 2400
   acggatgaca tccccattgt gaccaaccac tgcatgggtg atatctggac caacaagacg 2460
   ggcgcgtcgt cgtacaagac gcgcaacctg ctgcgtaaga ccctcagtgt aattgttgac 2520
   cagctcgttg aggacggtac gaccgatatg ggtaagcatg tgaccaagaa ggagaaggct 2580
   ctcgaatact cgaatatggg tcttttcgtg ctgtccatgt tcgatccgac gggtattgcc 2640
   tggatggctt ccgagttcgt gcagcccatt tgtggaccca ctgagtacct gggtgagatc 2700
   gatgatggta cgctgtacga cgctctgggt ctaaacacgg tcgaccaggc gttcctggga 2760
   agctacggtg tgtggaagaa gaagggtgat ggctccgtca cggtttactt cgagagtgtt 2820
   gacaagttcc ctgtgtctgt ggtgatcacg tccggtggtg acaagctcaa ggaggtcaag 2880
   gtgcctgcta acggcaacgt cacgtggaca tctacggtgg aggagcttgg tgacaagact 2940
   ctttaccttg accgctggcg tcctggtctc tttggcctgc ctggtacggg cggtggttcg 3000
   ctactgatgt ggatcccgcg atcgtctgag ggtggccagc ttgttcttca tgctcgcttg 3060
   aatgttagct aa 3072
<210> 3
   <211> 2405
   <212> DNA
   <213> Phytophthora infestans
<220>
   <221> misc_feature
   <222> (949)..(1048)
   <223> n is a, c, g, or t
<400> 3
   atgtcatccg tagacttgag tatcctgcgc aacggcatcc cggccgagct gccggcgcac 60
   ccgggcaacc accccgaccc gacgctgccc aaggctccgc accgcaacat cgacggtctg 120
   tccaaggacg atctcgtgct ggccgtgcag aacgcgttac ggtacttccc cgagaggttc 180
   cacgccactc tggctcccga gttcgcgcag gaactcaagg acgagggcca catctacatg 240
   caccgcttcc gtcccgtgca gtacgagatg aaggcgtacc ccattgacca atacccggcc 300
   aaatgcaaac aggcagctag tatcatgctt atgatcatga acaacctcga ccgtcgcgtt 360
   gcacagttcc ccaaccacct ggtcacttac ggtggcaacg gtagtgtctt ccagaactgg 420
   gcacagtact tggtggccat gcgctacctg tccgaactca cggaacagca gacgctcgtc 480
   atgtactctg gtcaccctat gggtttgttc cctagtcgtc ctgcagctcc acgtatggta 540
   gtgaccaacg gtctaatgat ccccaactac tccacacgcg ctcagtacga caaggcctac 600
   gctatgggta acacccagta cggtcagatg actgctggca gctactgcta cattggtccg 660
   cagggtattg tacacggtac gaccattact gtactgaaaa cctactgacg aggtctatgg 720
   cggttcagat gagagcaatc taccttcctt ctagaaaaat gctaaacaat tccagtggat 780
   gttgtatagg tcttgacaat caatattgcc ccctacgatt ttgtcaatat taacaatcag 840
   tttatttctt ccaatccctg attttacagt gaggatttcg agagtgggaa cgtggaaaca 900
   aaatcattgt caaatttgag cgagcctttc tcgccgatgc cagactggnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnngc gtcatctccg gtgtcatctc cgtcactgca 1080
   gagatcgacg aatctgcagt gaagaagcgt cacgaacagg gctgggttga cgaggtcgtg 1140
   agcgacctgg acgcctgtgt cgttcgtatc cgtgaggcaa aagccaaggg cgaggtggtg 1200
   agcttggcgt accacggcaa cgtcgtcact ctttgggaac gtctcgccga cgaggctgaa 1260
   gctacgggcg agctgctcgt ggagctcggc tcggaccaga cttccctaca caacccgttc 1320
   aacggcggct actacccggt ccagttgtcg ttcgaggagt ctcagcgcgt catggctgaa 1380
   gacccggagc gcttccagga actggtacag gagtctctgc gtcgtcacgc tgtcgctatc 1440
   aaccgtctga cagccaaggg catgcggttc tgggactacg gtaactcgtt cctattggag 1500
   gcacaacgtg ccggcgcgga cgtgttgcgt cccggtgtta agcccgagga ggctgctgtc 1560
   tcgactactg cctttaagta ccctagttac gttcaggaca tcatgggcga cattttctcg 1620
   cttggtttcg gtccgttccg ctgggtctgt acgtcgggtg accacgcgga cctgcagaag 1680
   acagacgcca ttgctgcccg tgtgatgcgc gagctgctgg ccgaaccgga agtgccggac 1740
   cgggtcgcgg ctcagctgcg tgacaacttg cgctggattg aggctgcaga ggagaacaaa 1800
   ctggtcgtgg gatcggaggc gcgtatcctg tacgctgacc gtgtgggtcg cggcacgatc 1860
   gccatggcgt tcaacgccgc tgtggctagc ggcgagctct cggcacccgt cgtgctcagc 1920
   cgcgaccacc acgacgtcag cggcaccgac agtccgttcc gtgagacgtc gaatgtgacg 1980
   gacggctcgg ctttctgtgc cgatatggcg gtgcagaacg cgctgggcga cgccgctcgc 2040
   ggagctacgt ggatcgcact gcacaacggc ggcggtgtcg gctggggcga ggtcatgaac 2100
   ggtggcttcg gaatggtact ggacggctcg gatgacgcgc gcgagaaggc ggcgtgcatg 2160
   ctcggctggg acgtcaacaa cggcgtggca cgtcgtgcgt gggcgcgcaa cgccaacgcg 2220
   cgcttcgcta ttgagcgcga gatgaaggcg gaccctttgg tacgtttgtt tggttacgag 2280
   cttgagactt tggaagcgtt agattttaat gtgtgtgtat ctttgtggta ttgcagctga 2340
   cggtgacgct ggctaacgag gccgacgacg cgagcgtgcg tgacgctgtg agcaagctct 2400
   tctag 2405
<210> 4
   <211> 1735
   <212> DNA
   <213> Phytophthora infestans
<400> 4
   atgctctcgc agcttaaacg cagattcacc ctgggacgac gttgtcagac cgaagaagaa 60
   gaccactcga ggatcaccat gagcctttcc aacagcaaca gtaccagctc cttgccccct 120
   gtggacgagc accacggctg tgagtacctg gatacggcgc tgacgatctt tgtaatcggc 180
   gcgtcgggcg atctggccaa gaagaagacg tatccgtcgc tctttgcgct ctacaccatg 240
   ggctacctgc ctgaacacgc ggtcatcgtg ggctacgctc gcagcgccaa gaatgatgcc 300
   gacttccgcg cgcaaattgc gccctggatc aagcctaaga cacccgaggc cgaggctcgc 360
   aaggaggcct tcctcaacaa gtgcatctac cgcagcggca aatacgactc aactgaagat 420
   gtgggcaagg taagcaagga gatggaggca ttggaagaag cccatggatc gcctgtggcc 480
   aaccgcctct tctacttcgc catcccgccc acagtcttcg tgcccatcgg cacgagcatc 540
   aagaaggcgg cactgaccac gcgtggttgg aaccgtctca tcgtcgagaa gccatttggc 600
   cacgatctcg actcgttcga caagttgtct caggacatgg gcgcgctgta cagcgaagac 660
   gagatctacc gtatcgatca ctacttgggc aaggaaatgg tgcagaactt gctcgtgttg 720
   cgcttcggca atgcaatctt cgagcccatt tggaaccgca actacgtgtc cagtgtgacc 780
   atcactttca aggaagacat cggcactcag ggccgcggtg gctacttcga ctcgttcggt 840
   atcatccgtg acgtcatgca gaaccacctg cttcaggtgc tgtcacttgt ggccatggag 900
   ccaccaatcc aagctgctgg tgacaactac tccaactata tccgtgatga gaaggtcaag 960
   gtgcttaact gcattgagcc tatcaagatc gagaacaccg tcctgggcca gtatgaaggc 1020
   agcaaggagc tcaacgagcc gggctacctc gaggacccga cggtgcccaa gggatcagtg 1080
   acccccacct tcgccacagc tgttatgtat gtcaacaacc cgcgttggtc tggtgttccg 1140
   ttcatcatga aggctggtaa ggccttgaac gaacgcaagg gtgagatccg tgtgcaattc 1200
   cgcccgcctc ctggagcgca gcacttgttc ccaggtgtca agatcccagt acaagagttg 1260
   gtgctgcgtc tacagccgga ggaagccgtc tacttgaaga tgaacgtcaa gagtcctggt 1320
   ctgcagaccc aggcgatctc aagcgagctg gacttgtcgt acgccgagcg ttacgagggt 1380
   gcagaggtgc cggacgccta cactcgcttg atcctggacg tgctgcgtgg taagcaggcc 1440
   gcattcgtgc gtgatgacga gctccgtgct gcatggaaga tcttcacgcc attgctgaac 1500
   gagattgaga cgcagaaggt gaagccgctg cagtatacgt tcggctcgcg tggccccaag 1560
   gagagcgacg agctggtgaa cagagctggc ttccagtacc accagggcga ataccagtgg 1620
   cagccgcgtg tgcgcactac cagtgcacta taggttgtct ctttggttga gcacaaagat 1680
   gcccggaagt ctggcataaa tatgatggtt gagccagcta gtcttcaata aggga 1735
<210> 5
   <211> 1245
   <212> DNA
   <213> Phytophthora infestans
<400> 5
   gggagcttaa aagtgctaca ggaagtcgct atcatggcgt caagaagtct cgtccgccgc 60
   tcggcgcgtc tcgccactgc catcgctact aagcgcagct tcgttcgcgc cgcttccgcg 120
   tcggtgaacc tccagtgctt tcagacagca cccgccactc gccagacccg tcttcagagt 180
   acgcagacaa gctccagcga tgagccgccc aaaacggagt ccccagtaga cccagagcag 240
   ctcattctgg ccaaggctct ggaccatgtg atttcccacg ggtacgttat ggtttaagtg 300
   ctataaaagc gaagatatcc taggttgtaa cgctctcgaa tgcaaggtgg gccattgagg 360
   ctctagcagc tggcgccacg gatcttggct acccttcggt ggctcacggc atgttccagc 420
   gcggagctat tgatttggtg gattatttta tggactcgtg tctcgccaag ctacgcgaga 480
   cgctgattgt caacacggaa aagttgcagg ccatgacggt gactgaacgt ctcaagtttg 540
   gcgtctgcac gcgtctgcag atgctggagc ctgtgttggc tacatggcca caggctatgg 600
   ccattggcgc tctaccgcag aacgcacctg gtactgcaaa gagactggcc cagctctcgg 660
   atgaaatctg gtacttcgct ggtgacaagt ccacggatct atcatggtac accaagcgcg 720
   ctattctcac gggcatctac gctagcacgg agctgtttat gctgaatgac aagtcgccga 780
   acttccagga cacgtgggat ttcttggacc gccgtgtgga cgaaacgatc caactcggag 840
   aactgcctca gaacgtacgt gaagctgttt gagtctctta taaggcttag ctgttgacgt 900
   gactaactac ttgtgtgctg gtctgtgtct gagcagctga acgatgtggc tgggatggcc 960
   agtattgggc tgcagtcggt gttttcggct gtgacgtcgc ttgcgggtcc tctggccagt 1020
   cagatcatct cgaactcgcc tttgagtcaa gttccgaacc cgatttcagc tgtgggcagt 1080
   gtggttcctc cctcggttgt atcggctgtt gcctctggaa tgccatttag caatcctact 1140
   tctgctggac atgacggtat ggcgttcaag tcgaaggatc tggacgaaat taaccaagag 1200
   cttgagaagc tcggcggcct tgacgcgagt gaacgacgga actaa 1245
<210> 6
   <211> 1461
   <212> DNA
   <213> Phytophthora infestans
<400> 6
   ccacgtccag ctcaagccaa ttgccatgct gtccgctact cgccgcctta cgcgatctct 60
   gcgtcgccct tctgcactgg gatgccgcct cgagtcctca ttcgctcccc tcactcagct 120
   atccgaggag gaaaccatgt tcaaggacac agtcgcccgg ttcgcggctg acgtggtagc 180
   tccgaatgtc cgcgccatgg acacagcggg ggagatggac catgctatca cacgaggact 240
   attcgagaat ggactgctgt cggtagagat cccagcagac tacggaggta gcgaggcctc 300
   gttcatgaac ctctgcttga ctattgagga gctgtccaaa gtggatcccg tcgtgggact 360
   gctcgtggat ctacagaaca cagttgtgaa caacgtcttc ctggtgagtg aagagtacga 420
   ttggtattct aaattctgta gctgaccatt gtgttgcagg tgcacggcac ggacgagcag 480
   aaagaaaagt acctgccgag actcagcgcg gacatggtgc gtgcatcaat atttggaaat 540
   attgtgataa tactaatatt gtacggtgtt gtagatcggc agtttctgtt tgtcagaggc 600
   tggatcaggt agtgacgcgt tcgcgctgaa gacacgggcg gaggcctcgc cggacgggag 660
   ctactactcc atcactggtc agaagatgtg gatctccaac gccgagtact ctggcgtgta 720
   cttggtgttc gccaatgtgg acccgtccaa gggctacaag ggtatcacgt gctttatcgt 780
   ggaccgagac atggaaggac tggagatcgg taagcccgaa gagaagctcg gcatccgcgc 840
   atcgtcgaca tgtcccgtca cactgacgga tgtgaaggtc ccgaaggaga acattctggg 900
   agagctgggc aagggataca agatcgcaat cagcacgttg aatgaaggac gcattggcat 960
   tgcgtcgcag atgctgggac tggctcaggg agtctacgac cagacgttgc cgtacttgtt 1020
   cgagcgccaa cagttcggct ctcccatcgg agagttccag gcgatgcagc accaatatgc 1080
   ggaggcagcg ctcgatatcg agacggcgcg tttgttggtg tataacgcag cgcgtctcaa 1140
   ggatgctggc caaccgttcg tcaaacaggc tgctatggcg aagcttcatg cctcgcgtgt 1200
   ggcagagaag acggcatcca agtgcatcga gctgcttgga ggcattggct ttaccaagta 1260
   cttgcttgcg gagaaattct atcgtgacgc aaagatcgga gctatctacg aaggaaccag 1320
   taacatgcag ctgacgacga ttgcgaagct tgtgtcggag gaatacaaga ggtaattgaa 1380
   tgtcgtgatc ctgtactcag acttgatcct tgtagcaaac caaacaagta acatttccgg 1440
   agaacgagcg ttagaaccag a 1461
<210> 7
   <211> 1580
   <212> DNA
   <213> Phytophthora infestans
<400> 7
   atgctgcgtc gtcttgccct ccgtaccgtg aagccttctg tgcgcaactt cgcctccgcc 60
   tcggagctgt acaagaaccc gcgcagctcc aagttcagca tgaccaagat cgtggccact 120
   atcggccccg tgtcggagca gctggacatg ctacagaaga tcacggacga aggtttgcgt 180
   atcatgcgca ttaacttctc acacgccgag tacgacgagg ccttgctgcg cgtcactaac 240
   cttcgtgcct gtcgcggcgt gcacgccgag ggtcaggcca aattcaacct acgtgccgtg 300
   ctacacgaca ccaaaggacc cgagatccgt acgggtaaga tgcgtgacgg caagaaaatc 360
   acgttagaga gtggcaagtc ggtggtgctc acgaccgacc cggctttcga gcttgagggt 420
   accgccgaca agttctacgt tacgtatcag cagttagccg aaaccgttaa ggtgggcgat 480
   acagtgttac tatctgacgg ccttatccgt ctcacctgta cgtcagtggg taaggatgag 540
   attacgtgcc acatccacaa taccgaggaa atcggtaacc gtaagggtgt aaacctcccg 600
   ggtttgatcg tcgagttgcc tgctctatct gataaggaca aacgcgacct ggattggggt 660
   gtcgagcacg atatcgactt tatcgcggcc tcgttcatcc gtaaggccag cgacgtgaac 720
   tcgatccgtt cgttcgtggg agagtgcgtt aagaaacact ggggtaacca acctggttac 780
   attgccccta agatcatctc taagatcgag aacctcgaag gtatccagaa ctttgaggag 840
   atcctcgagg cctcggatgg catcatgtgt gcccgtggcg atttgggtgt cgaagtgccg 900
   gcacagaagg tcctgacata ccagaagatg atggtggacc gctgtaacgc cgtcggaaag 960
   ccggttattg tggcgacgca gatgctcgaa tccatgcaga acaacccgcg ccccacgcgt 1020
   gccgaagtgt cggatgtcgg caatgccgtg ctggacggcg cggactgcgt catgctcagt 1080
   ggcgagtcgg ctcagggcaa gtacccgatc gagtctgtgg ccacgatgaa cacggtgatc 1140
   aaggaggccg accagctcct actcaagcct aactaccagg ccaagttcca gttcgagccg 1200
   cccacgtcgg acgtcgagtc agccgtgtcg tcggcagtca agacggccaa cgagatgcac 1260
   gcgcagctcc tgattgtgct cacccgtacc ggttacacgg cccgaaaggt cgccaagtac 1320
   aaaccgactg tgccggtcat gtgcttcacc accgacctta aggtcggccg tcagctccag 1380
   atccaccgcg gtctgtaccc ggtggtgccg gactaccttg accgtgcccc gactaccgcg 1440
   gaggccatcg ctcacgccaa gaagatggga tggttgtctg ccggcgaccg tgtcgtcgtg 1500
   atcagcggcg acaagctgtc ggacgatctc ggccgggaaa tcctcatcgg cgtcgctgac 1560
   gtccagtaag acggagacaa 1580
<210> 8
   <211> 900
   <212> DNA
   <213> Phytophthora infestans
<400> 8
   atgtggtcga cgatgatcat gcggttccct acgcaggcca agtccgtgtc gcgcagcgcg      60
   cttgtggcgg caggtatggc tgcgttcgct gccttctcca gtgtttcctc tactcgctgc     120
   gaagaggaga agtccaaggt ggcgttgagc cccaaggagt tccgttcttt cactgtgcgt     180
   aaagttgaga ccgtgaacta caacacgaag cgcgtgacgt tcgctcttcc cacacccgag     240
   cacgagatgg gtctcacgac gcctagttgc cttatggctc gtgccaaggt agacggcaag     300
   acagtggtgc gcccctacac gcctgtcaac gtgaacgacg agaagggttt cttggagctc     360
   gtagtcaagg gttacccaca gggaaagctc agcaagcaca tcgtgcagct caaagaagga     420
   gactctcttg acatgaaagg tccctttccc aagttcaatt actaccccaa caggtacaag     480
   agcatcggca tgatcgctgg cggctccggt atcaccccca tgctgcagct catcaaggcc    540
   atttgccgca acccggagga ccgcaccgag atcacgctgc tgtactgcag tgtctcggaa     600
   gaagatatca tcctgcgtga agaagtggag gccatgatgt acctgtaccc gcagatctcc     660
   gtgatccacg tgctcagcaa cccgtccgcc gagtggaagg gtcttacagg cttcgtgtcc     720
   aaggagatga tcgaaaagta catgccggag ccgtcagacg acaacctcgt gtgtgtgtgc     780
   ggtcctccgc caatgatgta ccacgtctcg ggtgacaagg cgaaggacag gtctcagggc     840
   gaactgcaag gtctgctgaa agacatgaac tacacctcca ctcaagtgtt caagttttaa     900
<210> 9
   <211> 1245
   <212> DNA
   <213> Phytophthora infestans
<400> 9
   gtcgagcaca agaccaacac tcctgcacca tgactaccct caagatcgtc gtttccggcg      60
   ccgccggcca aattgcgtac tcgttgctgc ctctcagtac gtccgtctcc ttccctcttg     120
   tgtcttctcc ataccctaac gtctcaccat gttgcagtct gcatcggcca cgtcttcggc      180
   cccaaccagc gcgtggagct gcgcctgctg gacatcgaac ccgcccagga agcgcttgag      240
   ggcgtcaaga tggagctcca ggactgtgcc ttcaacctag tggacgctat catccccaca      300
   gccgatctgg agactgcttt caaggacgcg gacgtcgcaa tcctcgtggg cggcttcccg      360
   cgcaagcaag gcatgcagcg caaggatctg attgagaaga atgtagccat ttttaaggct       420
   cagggagctg ctatcgacca gttcgccagt cgcgacgtta aggtgcttgt tgtggccaat      480
   ccagccaata cgaactgcct cattgccatg gagaacgcac ccagcatccc gcggcgcaat      540
   ttctcggcac tgacgcgtct ggaccatgag cgtttgcgct cgttcctagt ggagaaggtc      600
   aacgagaccc aaagcccgaa agttacgtcg aaggacgtca acaaggtcgt gatctggggc      660
   aatcactcga gcacgcaagt gcccgacgtc acgaacgcag aagtgaaggg ccagccgctt      720
   gataagatcg tctcggacaa ggattgggct gagaagaagc tcgtcaagga cgtgcaggag      780
   cgcggtgcag ccatcattaa ggctcgcaag ctctcgagcg ccatgtcggc tgccgccgct      840
   atcggcgccc acctgcgcga ctggttcaat ggctccaagg acggcgagct cgtgtctatg      900
   gccatttgct cggacggcaa caagtacggt gtgcccgagg ggctcattta ctcgttccca      960
   gtcaagtgcg cgggcaacgg cgcgtacgag gtggtgaacg gtcttcccat ctcgccgcgt     1020
   atcgacgcaa tgatgaaagc cactgcgcag gagctgacag aggagaaggc cgacgctgtg     1080
   gagatcctgt cgcgccagtg agcagtattc agctaatagc tggacgatgg caacgtcgtc     1140
   tacaccccat tcgatttttt tctcccatta tgagctgctt caatcgttta tttgaccatc     1200
   taaaccaatt aatttcttgt tattacgatt tcttattgaa aaaaa                     1245
<210> 10
   <211> 1280
   <212> DNA
   <213> Phytophthora infestans
<400> 10
   atgtcgtctt cattttgtca gctatccaag cgcgtgctgc agtatcttcg acatgagcat 60
   ccacatcgtc tatttccctc catcggtcga agacgcaaac tcccaggcat aaacctcgac 120
   ggaagatctc agcggtgatg gcgcgtggtt agccgcaagg tgttccgatg cacaccacac 180
   actactcgcg aaaacgacca tacttctgtc ggccagggta acctctgggg agctgcacaa 240
   cggccgtctt tttagcttat tttagcgagc caacaggttg tgcctcattg gccacgtgtt 300
   cacgcacgta cgtcacgtgt gtgctcattt taatattaaa gatcccgttg aagacgaaaa 360
   gcttctttgc tggcatcatg cgcttccaga cgttgttgct cgtccttgtc gcactagtca 420
   tcgccccgtt tcaggccctg tctccaccca gcgagtccaa cgtgccgttc actcttcggt 480
   acgtcccggg cttctttaaa caaggtaccc cgtccgtgga gattccacct tcaaattcaa 540
   ggcatctcgg gctacttgac aacgtcacgt ggacggatgt agacgcctac attgctgcac 600
   gagccgctca aggcgtggac gtcaagttgt tcctcttctt acgtcatgga gaaggcctcc 660
   acaacgtcgc cgaagccact tacggcaccg aagcctggga cagattctac agcaaactag 720
   cgaaatatac tgacgccaag ttgacgaaac ttgggatgca acaggctgtc aaagcgtcgg 780
   aaaggatcga cgaggagctt aagagaggac tgagcctgga ggaagtcgtg gtttccccgt 840
   tggagcgtac actacatacg gcaatgatcg cgtgccagaa tcaccacgag atcccgaagc 900
   ggtcgatgga atggccccgc gagaccatcg gcgtctgcac gtgcgactta cgcggcacca 960
   tctccgccaa ggccgagctg tacccaagta tcgacttcag tgatatctgg agtgatgcag 1020
   acccgtggtg gacgcctgat catcgtgaga ccgagctgca catcaacgac cgcgctcgca 1080
   tcttcctgaa ccgcgtcttc tacggtcaca agtcagtgcg tgttggtgtg gtgacgcaca 1140
   gtggactaac caccgccgcg atgcgtgtca ttggccatcg taagtacagt gttgcgacgg 1200
   cggaagtgat accgttcctg cttgaagaca ccacggtgca cacgatcctg tcgatcttct 1260
   ctggtaaaga agacatgtag 1280
<210> 11
   <211> 1016
   <212> DNA
   <213> Phytophthora infestans
<400> 11
   atgtcgtcct atcgtgctgt ccaagttgta aagttgacta aggacttccg cgccgctacc 60
   agaatcgtga gtgtccccga actacccacc gctgggcccg acagcatcgt tgttaagaac 120
   cgcttcttgg gaattaactc gaccgacgtc aatcttacga atggcatgta tcatgacaag 180
   ctgccgtttc tcgcgggcgt tgaaggtgca ggtctcgtca ctgaggtcgg gagtaacgtc 240
   acgactgtga aagtgggcga cgctgtcatg taccagacgc tgggagccta tgcggactac 300
   gtacaagtgc ccgtgtcggc cgccatcaag atcccagagc tgtcgaagaa cgctctaccg 360
   gttgcagtgg gcggcgtctc tgcgtccatc tgccttgaat gtctcggtga gatgaagtcg 420
   aatgagaccg ttcttgtgac tgctgctgct gggggaaccg gacagtttgt cgtacagctc 480
   gccaaacttg ctggcaacca tgttatcggc actaccagct ctgacgagaa agtggaggct 540
   ttgacgaaac tgggctgcga ccgtgtaata aattacaaca aggagaatgt cggcgatgtg 600
   ctaaagaagg agtatccgaa tggagtagac atcgttttcg agtccattgg aggcgacatg 660
   ttccgtgctg ctgtcgataa cattgcagtc cgtggtcgta tcatcaagat tggcttcatc 720
   tcgcgagtat tggagaccaa agacaagacg actccccaag gccgcctccg ctgctatcgt 780
   gggaagcgaa caagaagatt ctgatgaagt ctgcttccat ccgagggctc ttcatgtatc 840
   actttgagga acacattcga gagcacacgg agcgattgct gaagctcatt agcgagggca 900
   cgttgaagcc tggcgttgac cccactacgt acaagaaatt cgagtccatt ccgaatgcaa 960
   tcgaccgcat gttcgcccgt gaaaatgtag gcaaactcat cgtagagctc gagtag 1016
<210> 12
   <211> 267
   <212> DNA
   <213> Phytophthora infestans
<400> 12
   atgggcaagc acacggagcg agccaaggag agtccattca agcagcagtc gatgatcctc        60
   gtgcctatgg acacaccagg cgtgcaggtc gtgaagccca tgcatgtctt tggctacgac       120
   gacgcaccgc acggacacat ggatatgctg ttcaaggacg tgcgtgtgcc gttcagcaac       180
   gtgttgctcg gtgaaggtcg cggctttgag atcgctcagg atcgtcttgg acctggccgt       240
   atccaccact gtatgcgagc catctga                                           267
<210> 13
   <211> 1147
   <212> DNA
   <213> Phytophthora infestans
<400> 13
   atgcgcaaaa tattgaatat tgattggtta aaacctggtc ttggcatcct tatgaccaat        60
   tacagttatt gagtgcttta agcggggcag acgtccacta cgtcacactc agaatctcca       120
   cacaagcaac agaccgacac aatgagcagg tgcgagtggc atcccagcaa cttcgtgcaa       180
   atttagcctt gttatattaa ttgtataacg tggcatgttg cagaccgacg atctcagagg       240
   ttatcgccga gcggaaggcc aacaacactg cggctttcat caccttcgtg ccctgcggct       300
   tcaagactaa ggctgacaca gtggacattc ttctgggact gcagcgcggc ggcgcgaaca       360
   tcatagaggt gggcattccg tactcggacc cacaggccga tggcccgacc atccagcgcg       420
   cacaccaggt gggcgtggac cagggcatca cgctgcatga cgtcctggcc acagtgagcg       480
   aggcgcgaac taagggcctt actacgcctg tggtgcttat gggctactac aacaacattt       540
   tgcagtacgg cgaagtcaaa atctgccccg acgcccagaa aggtacgcct gtaatcgacc       600
   acaggagaga tttgtgggct caaaaactga caataaacgt atttgtagct ggggtggacg       660
   gattcattat tgtggacctg ccgcctgagg aggcaaagac gctcagtgac gacgcagcta       720
   agcacggact tgcgtacatc ccgttggtgt ccccaacgac gacggaggag cgtatgaagc       780
   tcatcgactc ggtggcacat ggttttgtgt attgcgtgag tctcaccggt gtcacgggcg 840
   ctcgtaacga gctgcctccg aacctcgacg cgttcatggc caagagtaag ttgcttttcg 900
   cgctttagtt tattttaatt gtcgagctaa cgtggtccct tgattcgctg cagttcgtgc 960
   caacgtgaag caccctctcg ctcttggctt cggtctgtcg acccgccagc acttcgttca 1020
   agccagcgcg cttgcggacg gcgttgtgat cggctcgaag attgtcaaga tcatcgagga 1080
   cgcgcctgac acagccacgc gtgctgccaa tgtggaggca tttgccaaga gcatcgtgaa 1140
   cccttag 1147
<210> 14
   <211> 1917
   <212> DNA
   <213> Phytophthora infestans
<400> 14
   atgggtaaca tcctggagga gatcgcagcg cagcgccgtc tggacgtggc ggccgccaag 60
   caggtcatat ccgccgatga tctggccaag aagatcgagc acgcggaggt cgttttcggc 120
   tcgaccctgc ccgtgcttga ccgccttaac gcacccacgg tgcgtccggc agtgcaggga 180
   atctggagaa gaatctgcgt ctaacgcagc taccgctgta ctgtattttt tccttgtatt 240
   tatggcagag agaggggtgg tccgacgtgg ctctggctgc cgagtttaag cgtgcgagcc 300
   ccagcaaagg tgacatcgcc acggagctca atctgcgtga gcaagtgcag gcctacgcca 360
   acgcaggtgc cagtatgatc tccgtactga cagagcccaa gtggtttaag ggatcgctag 420
   acgacatgag ggcagcccgg gaggtggtcg agggcatgag tcagcgtcct gccatcctgc 480
   gcaaggattt catcatcgac gtgtaccagt tgctggaggc ccgcgcttac ggagcggatt 540
   gcgtgttgct catcgttgcg ctgctgtccc aggagcagct tatcgagctc attgacgtac 600
   gtcgccccgc ttgcactaca tgtagtgggt ttactgtaca ctgacaagcc tcttgttggt 660
   ttgtaggcca cccacaatct cggtatgtgc gctctggtcg aggtgaacag catccaggag 720
   ctggacatcg ctctggctgc gagggctcga ctcattggcg tcaacaaccg cgatctccgc 780
   acgtttaagg tagacatgaa cacgacggct cgcgttgcag acgctattcg tgagcgtggg 840
   ctttcgctgg gacgtgacgg cgttacgctc tttgctctca gtggcattcg ctcgcacgca 900
   gacgtggtca agtacgagaa gtgcggcgcc cggggcatct tggtgggcga gtacttgatg 960
   aagagtggtg atattgccgc gacggtgaag gatctcttgc agaatgtgac gcgtcacacc 1020
   gagtcgggcg aattcgcttt ggctcctccg cttgccaaag tgtgcggcgt cacgacagtg 1080
   gagtacgcgc tggcggcctt gcgtaatggt gccaatatga ttggtatcat catggcggaa 1140
   cactcacccc gctatgtcca agtggaggaa gccaaggcca ttgcccaggc tgtgtgggag 1200
   tatggggagc gcacgggccc tattctctca gacattgtgg agactcactt ggacggcaag 1260
   aacgactggt ttcatagaaa tgtccttgcg ttgcgtgaag cttgctcgcg tgcacctctc 1320
   gtggtcggag tgtttgtcaa caagacagct gccgagatga acgcgactgc aaaggaaatc 1380
   ggactggact tggtacagct acacggcgac gaggggtttc agatctgcag ggacattaag 1440
   taccccacca ttcgcgcgtt gcatctgccc gacaccacgc tctgcgacgg cgtggacgca 1500
   gaagccgttc tacagcaggt tcaggaaggc cttgccaact acattctgct tgatacgacc 1560
   gtgaagggtc agcagggcgg cactggcgtc acattcgact ggaagattgc agccatcttt 1620
   gcgcaggcac gacttccctg cctcatggct ggtggcctta cccctgagaa cgtggtgaag 1680
   gctctatcgg tcggtcaccc cgttggggtg gatgttagca gtggagtgga agtgaaaggt 1740
   tcacctggtg tgaaggatat ggacaaggtg actgcgttcc taaaggctgt gaaggattac 1800
   ctctcgattg ctacgcttaa gatcgaggag gagacggaaa cctagagaga gaccctcgga 1860
   gtgttctaat caatgagggc cactaatgaa gtgcttttac gttacgagct tggatgc 1917
<210> 15
   <211> 1494
   <212> DNA
   <213> Phytophthora infestans
<400> 15
   atggtgcact atcgtagtac tcgcggcggc gtccgcggcc ttagctttga gcaggcggtg      60
   ctgacgggct tggcttccga ccgtggcctg ctgatacccg aagctgacga gttccctacg     120
   cttccagctg atgcgctgga gaagtgggcg tctctttcgt accaggaatt ggccgtggag     180
   gtcatgcgcc ttttcattga cgaaagcgag atctcgcgtg accagctgcg tgagcttgtg     240
   accaagagtt acaacgcgac cacgttccgc tccgatgagg tggcgccggt ggtcaaggtg     300
   acggatcaaa tgctggtgct tgagctcttc catggcccca cgtttgcctt taaggatatc     360
   gctctccagt ttctaggcaa ccttttcgag tttttcttaa agcgcaagaa cgaggcgctg     420
   ccttctgacg ctcctaaaca ccagatcaca gtcgtgggcg ccacctcagg cgacaccggt     480
   agctcggcca tatacggtct ccgcggcaag gagaacgtcg aggtgttcat cctcttcccc     540
   gagggtcgtg tgagcgccat tcagcagcga cagatgacta ctgtattgga ccagaacatc     600
   cacaacgtgg ctgtgaaggg cacgttcgac gactgccagg ctatcgtcaa ggaccttttt     660
   gccaacgctg acttcaaggc caagtacaac ctaggtgccg tcaactcgat taactttgcg     720
   cgtatcttgg cgcagatcgt gtactacgtc tgggcctact tccgtgctca cgacgagggt     780
   gttagcggcg aggttgcctt ctccgtccca acaggcaact tcggtgacat ccttgccggt     840
   ttctacgcca agaagctcgg tgttcccatt ggcaagctga ttgtggctac caacgagaac     900
   gatatcctgc accgcttttt ctccacagga aaataccacc gccgcgacat cgagcacacg     960
   atttccccgt ccatggacat ttgcgtgtcg agtaactttg aacgttatct cttcgctctg    1020
   tccggcgaga accacgacat tctacgcggc tggatgcagg ctttcgagca gactaatgag    1080
   ctcacgatct cgggcgagct gctctccaag gcacaagacg agatggcatc gtatgcggtg    1140
   cttcaggagc aggtgcgctc cactattgcg gagtacaaaa cgatgcatca gtacctcttc    1200
   gacccgcaca gcgctattgg cgcagcagct gccatgcact ttgtccagga taaccttgca    1260
   gacaagccaa actcggcggt agttgtagtc ggcactgccc actacggcaa gttcctcccc 1320
   gtggtgtcga aggcactagg tgtcgctgag tcagagattg agcaacaccc gatcctcaag 1380
   gcgctggaat cactgcccac ccggctttcc gttgccagca actcgagtga aactgtggct 1440
   gagcatattc gcaagattat tgctgagaag aacgacgaag cctgtagtga ttga 1494
<210> 16
   <211> 1299
   <212> DNA
   <213> Phytophthora infestans
<400> 16
   atgagctcgt tccgtcttcg tattgccaac gcgcgccaga tcgttcaagt ttgtgcgaac 60
   ggcgagccgt ttaagaccgg cgcggcccaa aaggatctgg ctgtgctcga gaatgcgtca 120
   cttgtcgtgg acaaggcagg taagatcgcc ttcgttggtc ctgccgctga cgtagagaag 180
   tggctgagca cgcagctgca gcccgtgagc tttgagaagg acgtggacgc tcgtgacatg 240
   gtcgtactgc cgggttttgt ggatgcgcac acgcacccag tgtggagcgg caaccgtgtc 300
   aacgagtttg ccatgaagct ggctggcgct acctacatgg aagttcacga gagcggcggc 360
   ggcatcaaca acacggtgcg tcacaccaag aagagttcag aaactgagct gttggagctg 420
   ctgcgtacgc gactagaccg catgttgcgt agcggtacga cgcttatcga agcgaagagc 480
   ggatacggtc tcgagactga cactgagctc aagatgctgc gtgttttaca ctccgcatcg 540
   acgggtgaca acgcacaccc tgtggagatc gtgtccaact acctgggtgg tcactcggtt 600
   cctgacggca tgactgctgc tgaggccacg gaggatatca tcacgaagca gattcctgct 660
   cttgtgcagg ccaagaagga cggcaccatc tcgcccgaat tcatcgacgt gttctgcgag 720
   aagggtgtgt tcgagcacaa cgactctcac cgtattctag aggcaggcaa gaaggcggga 780
   ttggacatta acttccacgg tgatgagatg tgtccgatgc agtccggtac cttggcggca 840
   actctgggcg cccgtgcaat ctcgcactgt gagatgctga ctgctgagga tcttcaggct 900
   atggcagcgc acaagccaga gcctgtattc gccgtgcttc tgcctactac caagtatatc 960
   ctgaagctgc ccaatccgcc agcacgcgac atgatcgctg ccggtgttcc tgttgctctg 1020
   ggcagtgact acaacccgaa cgctcattgc ctttccatgg cactgaccat gaacatggca 1080
   tgtgtgctgt tcggtatgac catgaaggag gctcttgtag gcgccaccat caacgccgct 1140
   gcctccatca accggtcggc tactcacggc agtctcgagg tcggcaagca gggcgacttg 1200
   gtcctgatgc gtgctacgca gtgggagcag atcatctacg aaatgggcga ccctcctatt 1260
   gaacacgtag tgaagaaggg agttgtctac acgaagtag 1299
<210> 17
   <211> 1846
   <212> DNA
   <213> Phytophthora infestans
<400> 17
   gcaacgaaca agtctcttcg ttccacttcc gtttcacctc gtcgtcatgg ccatcgccaa 60
   ttcgaacacg aagtctgagc tcgttctgga cggcgagtct ctctgtgctg aagacctggt 120
   gcagctgtcc aagggagaca cgagaatctc gctaagtcaa gaagcctgga agcgcgtggc 180
   ttgtggccgt gaggtcgtgg acaatatcct caaggacaag actcgtgtgg cgtacggtat 240
   taacacgggc ttcggactct tctccaacgt cattatcggc cccgagaagc tcacagagtt 300
   gcaggaaaac ctcatccgct cgcactcgtc tggcacaggc gagccgttga cattcgctca 360
   gacgcgtatg ctgctcgcgc tgcgtatcaa tgtattggct aagggacact cggggatccg 420
   tgtgcacact ctggagcagc taatagacgc ctttaacgcc gactgtctgt ccgtggtgcc 480
   agccagaggc actgtgggcg cctcgggcga cttggctccg ctagcacacc ttgcactcgg 540
   tatgatgggt gaaggaccca tgtgggataa ggtggatggt cagttcgtca tcagcgaggc 600
   ctccaaggtc ctggccaagc acggactgaa gcctgttcag ctcggagcaa aggaaggtct 660
   ggctatgatc aacggcacac aactcatcac gtcggtgggt gctgaggcgg ttgtccgtgc 720
   tcagaacgtc gctaactgcg ctgatatcgc cgtcgcattg acactcgagg tgctctgcgg 780
   tactgtcaac gccttccacc cgcgtattca tgcagcccgt ccgcacactg gccagatgct 840
   cgttgcctcg cgtattcgca cactactacg tgctgacaac ccgtccgagc tgttccgtag 900
   ccacaactac gaaggaaagg tgcaggacgc ctacactcta cgttgtgcgc ctcaggtgca 960
   cggcattgtg cacgacacga tcaacttcgt acgtggtgtg ctggacgtcg agatgaacag 1020
   tgccacagac aaccccatgg tcttcacggg tagtgccgag gtcacgacgg atctgtctcc 1080
   ttcaattgac acgaatcaag tcaagcccaa catcgaacag gtagaacacg agatcacgga 1140
   tctgaatgac gccaaggagg agatcaagcg tcttaaggct cttgttgcac agaagcagaa 1200
   gcctgtggaa cacccggcgg cgggtatgaa gcgcacatcg gacacgttct accgtggcgg 1260
   cggtggcttt gtcatctctg gcggcaactt ccacggcgag tatccggcta aggtgctgga 1320
   ctacctggca attggtatcc acgagattgc tagcgttagt gagcgtcgta ttgagcgtct 1380
   agtgaaccca acgctcagca acctgccggc tttcctcgtg ccagagggcg gtctgaactc 1440
   gggttttatg attgctcact gtacggcagc cgctctggtt tcggagaaca aggtgctcac 1500
   acacccgtcg tcggtggact cgatctctac gagtggagcc aaggaggacc acgtgtcgat 1560
   gggaggcttt gctgctcgta aggcgcttac ggttgtggag catgtcgaga ctgtagttgc 1620
   tattgagatt ctggcagcgt gtcaagccct tgacttgctg cgtccgctgc gtacgacgga 1680
   ggctcttgaa gctgtccacg gactggtacg cacccgcgtg gctaggtttg acaaggatcg 1740
   cttcatgaag cccgacattg acgccgtgct ggacctcgtt cgtagtggag ctatctgcga 1800
   cgttgtagct ccgttcttgt caaaactgca cgtgtcagga ctttaa 1846
<210> 18
   <211> 1218
   <212> DNA
   <213> Phytophthora infestans
<400> 18
   ccgcacagag caactgccac taggcatcat gctgcaccga cttggccgcc gctcgactgc 60
   cacttccgcc cagcggtcac tgcgccgatc agccatgacg ccaccgcagc gccaatcctt 120
   tcatttcagc tcgtcagcac gcaagccggc atctttggat gctcagaacc gcgtagctgg 180
   tgctgcctcc tttttcctgg acgccaaggc cgtttgcaag ggcaagcgct gtgcattggg 240
   caagaacaac agcggtattg gagctaaccc ggccacatgc ggggaggatt cgttcttcct 300
   cacacctgac gtggtgggcg tggctgacgg cgtcggtggt tggaacgaga acggcgtgga 360
   tccaggcaag atatcgcgct cgctaatgcg caacgcggca ttatttgtgc agcagcagac 420
   ggccaatagc gagagtgcca ccacgcaaca ggttctggct cacggctaca agcaggcgct 480
   gctggatgac gaggtggagg cgggtagcac gacagcctgt atcgtgagac tgaagcagtc 540
   gtctgagggc aaacccgtgc ttgagtactc gaacctcgga gattctggct tcgtggtcat 600
   ccgcaacggc gagataatct tccgcagtaa atttcaggtt cgaattgact gttgggagtg 660
   acagtagaag agttactgac ggcgttgctt tagtattatg gacgtgctcc gtaccaactg 720
   gcaaagatcc ctctgcggtt taagcagtat ggtgctattg agaatcaccc tgatgatgta 780
   agtctgcttc gcatttggat actatttatg ctggagacag tacaatgcta acggttattg 840
   ctccaggcgg actcgggtga gattgacgtg caagacggag acctcgttgt actagccact 900
   gatggtgttt gggacaactt cgcccccgac ctccagaaag ctccttcgca gttcccggta 960
   agattcttta ccgccgtgtt cgagctacct gtaattactg actttgtttc tcgttattgt 1020
   ctgctatttc taccgtagcc gatgctctcc tggcgaagat attggtcagg tgaactcgca 1080
   tcttttgtgg atgttattgc agagaacccc gacaaggctg ccgaaaatat cgtcctggcc 1140
   tcgctccgcc acaaccttaa gcccgacgac atcactgtcg tcgtggcaaa ggtagtcgtg 1200
   atggcatcaa agctgtag 1218
<210> 19
   <211> 735
   <212> DNA
   <213> Phytophthora infestans
<400> 19
   atgaagcagt tactgcagcc gatctcgttc tctggtgctg cgagtgtgtt cgtcgtggtg 60
   gcaaagcaac gcaagaaacg ctgccgtgcc aatgacaagg aagatgacgc tgttaaggac 120
   gacgaagaga ctgagacagc agaggacgaa ctggacgagg aagaccaacc cacgcgactg 180
   tacgtggcca atgtgggcga ctgtcgagct gttttgtgca ctgcagacgc cttagcagtc 240
   gacatgacga cggatcacaa ggcgtcgcta ccggctgaac aggagcgtat cgaggcgtca 300
   ggtggcttcg tgcacaacgg ccgactggac ggcattctgc agatctcccg tggcttcggt 360
   gacttggcgc acaaacagga tggccacttg gtggtcacgc cagatgtggt ggagcatctc 420
   gtgaacccgt cggaccagtt cttgctgctt gcaagtgacg ggctcttcga cgtgttgacg 480
   tcgcagcaag ctgtcaactt tgtgctgcgt aaacttcaga cccatggaga cgtccaactc 540
   gcagctcagg agctggtgct caaggcgcaa gcttacttcg cacacgataa catcagtgtg 600
   gtcattgtgg cgttgaacca gaaaggtgac gcgtgaaatg aacagcggct tctattgact 660
   acggaagatc ggagaccgac gaagaagcag aaaacccgga gatcaagctc gagatccaag 720
   aaattatgaa caaga 735
<210> 20
   <211> 1845
   <212> DNA
   <213> Phytophthora infestans
<400> 20
   atgcgacgca aggagaccaa ggtgttgttg acgctcgaag aggtgcgcga ggccttcgag 60
   cagcagacgc cgctggccgt caaggacgcg ctgggtgtca ttcatgaggc gcagttcatc 120
   atgaacctgg agccgaacct tgtggccgtg cggcaaagag cttcaacata tgtttttgga 180
   gatatccatg gccagttcta cgacctgatg cagctgatgg acgccgttgg cgttgccgac 240
   ttggccgagc gtgatgtcca gctggtgttt ctgggagact acgtggaccg cggggccttc 300
   tcgtgcgaag tgatgctcta tctgctactg ctcaagatcc gattttccga caaagttgtt 360
   ctcctccgtg gcaatcacga gtgcgaatcg atttcatcct tctacggatt tcgcaacgag 420
   tgtaagggga agtacggcat ctcagcctac taccacttct tgtcctgttt ccaatcgatg 480
   ccagtggctg cgctgctgtc cacgtcgcgt gggcaagttc tgtgtgtgca cggtggcctc 540
   tcacccgagc tgaaaacaat cgaagacatt caaactatgg accgacggcg ggaaatccct 600
   accacggggc tgttatgcga ccttttatgg tcggacccga agacttcgca cactcgcgat 660
   gtggacgcag aggtggacac tcagccagga tgggagccca atcaggcccg tggatgctcc 720
   tactacttca attcggcggc attgttcgag tttttaggca ccaacaagct gctgtcgatg 780
   cttcgtgcac atgagttcga ggatgaaggc ttcacgtatc acttcaactc gcaggagtat 840
   caagagctgg atactcgagt ggacaagtcc atgcctccac tcattaccgt tttttcggcg 900
   ccgaactact gcgacagcta cggcaacacg gccgcctatt tgctcttccg gaatgaacct 960
   ttctcgtggg aaatccagca aattaactcc gcgggacatc cagccccacc gattgcgagc 1020
   gcagaacgag gatccgacat gtggcgacta ttcaatcaaa cgttgccatt cctcccagca 1080
   agcaaggaat tctttgagga agttttgtgg ttagcagaag gacgacgacg tcttacaagt 1140
   gaaatgcacc cacaagctat ccgaaagatg ttggatgacg aagtgaacaa atgggaattg 1200
   gtagaagaaa cgccaacgcg cagctcgggg ccagcgtctc ctcctcgaat ccaccgcaga 1260
   ccttctttga gtaatctgga cgagacccaa acggacgagc gtccaagtct gcttactccg 1320
   caagagatgg acacaattaa gctgatgttt tcattaatgg acacggatgg cagcttggaa 1380
   ctcagctcga ctaaagtgtc tcagttcatt ctcaatattc tgggtgaaaa aatcagcact 1440
   gcggacgcgg aggcttattt ggatgctttg gactacgacc gcaacggagt ggtggatttc 1500
   gcggatattc tgtcgtgggt tgccgtgatg aaggcgaatc gcaacaagca cgagtcgagc 1560
   cgactcctga gttggccaac tgtacagagc ggtgtgcgca tgttgactcg tgggattttt 1620
   tccagtaaag tcttgctatg gctcgcattc ggctgcttac tacgcgatat tatcgtccca 1680
   aagcagcgaa aagtgatcaa gtcgtcgtcg atccgagtgc tgggctcagc gtcgctagtc 1740
   ctgtacatgg tgggcgtatt gtcagggcga gagcgtggct ggctgcggct tttctctctg 1800
   caacgcgctg tcggaattat gacgcagcga ttcgtatcaa agtag 1845
<210> 21
   <211> 1489
   <212> DNA
   <213> Phytophthora infestans
<400> 21
   atgatgccgc ggggcttacc accacgaggc ccacctggca gtttcccacc tactagtgtt 60
   gcggctgtac cccctgcagg cggtgccgct gcagctcgtt tcggcaagct ctctgtgaag 120
   gtgttgcgtg cgtttgacct aaaaaagctg ggaatgctgg acactgcaga cccgtatgtg 180
   aagctcacga tcggcacgca gaacgtacag actaaggtcc aagcaggcgg tggcaagacg 240
   cctgagttta atgagacctt cgacttcaat attgcaaccg agaaggagct cgtggtcgaa 300
   gtttgggacc aagaaaaagg aggacaagac cggttcatgg cgcaggcgaa ggtggagatc 360
   gtgtcgtggc tatcaaaggg tggctttgaa ggtgacgtcg agctacggga tcgcgagaac 420
   agccctgccg ggaagctggc gatcgttgcc aagttcacga agcccgaaat tggcgcgaca 480
   ggacccgtca aggctccacc catggcccct ccgatactct caggtcctgc agtggctccg 540
   ttgcccccag gagcgaatgc cgtatctgtt cctggtgcgc tgccattggc accgtccgaa 600
   ccgcctcgag acccgaacgg caagttcacc gacaaggaga ttctcgaggc atttaaagcc 660
   tttgatctgg atcacaataa ctatgtgggt gctgcggaaa tccgccatgt gctaataaat 720
   attggcgagg cgcctacaga cgaagaagta gatgaaatga tcaagatggt tgacaaggat 780
   ggcgatggcc aggtgagctt tgccgagttt tacgcgatgg ttacgaaagg gaagcagccc 840
   ccacccggat tgggtgtcac tacagctctt ccggagaaag ctgcagctcc tggaggtgca 900
   gtttcgggcg ctcaggccat tcagcttcgt aatcagcgca aaatggcgct ggaagagttt 960
   gcacgcgata acggtatcaa acccgagagt gtgaagaagg cgtacaagcg attccaagcc 1020
   acagataaag atggatcggg ccagatcgac tactcggagt tctgcgaggt gctgcaggtg 1080
   gatccatcgc cgcagtgcga gaaggtgttc cagttgttcg acaatgacaa gacgggtcgg 1140
   atcgacgtcc gggagttcat gattgcgttg tctaatttca cgggtgctga gaaggaggag 1200
   aaactaaagt tcgcgttcct cgtgtttgat gaagatggta acggtgtgat cacgcgacaa 1260
   gaactgatga agatcctgaa ggcgaaccac atggcctcta gcgaatcgga agttgcgcgc 1320
   aaggcggaca ctattatgtc ccaaggggat aaggacggcg acggagttat ctcgttcgac 1380
   gagttctcag ttgtgagcaa gaagtttcct aatattttgt tcccagccta cacgctaggt 1440
   acggccaaag attagaatac ccgatgaata caatatcctt atctctcgt 1489
<210> 22
   <211> 2862
   <212> DNA
   <213> Phytophthora infestans
<400> 22
   atggctagca agctcaacgc gttcctggcc gcggtgcctg cgaacgccaa cgtgcccagc 60
   ccgcccaaga cagcgccggc cgtgatggcg ttcccgcagc ttgagttcaa ggaagcgccc 120
   gagaacgtgg gcgcattcgc ccccaccacg ccgcagccgc ccgtggtgcg tcgtcgccag 180
   cgcaaaaata accccagcct caagctggag ctggactcga accaggaaca tgacagacct 240
   gaggatgatc gagatatgtc gcgtccttcc cctctaaaga gcttcatggc cgcccaagca 300
   gccgccgtta agctgaaggc acagacggag aagcacgata tcccaacacc agacttggcg 360
   cgcacaagca gcgggtccga gctggacgcc aagtcggagg cgggacccat gcgcatggcc 420
   agcgtacaag gctcgcacca ccgtcgcgtg tccaccgaca caacgctatt taaccggtat 480
   ctgcgtcaac aagcagacct ggatattagt cacgacacgg tgcagtattt cgcaaatgtc 540
   ccggtcatta ccgccactgc agctgccgtt gatgctctgg aatgcgaaat gagtgtggat 600
   aataacgccg acgaggagca cgcgatcatc gtacaggaac ccgaggagat ccttgctgtc 660
   ttccgactcg gaggcaccat ccctgtgtcc agtgcgctgg aaattgtacg acgagctacg 720
   aacctcatgg cactagagca gaacgtcatc tcgattcgtg cgccgtacac tctagtgggg 780
   gatctccacg gccagttcca ggacttgctc gaactcttcc gagtacacgg ctctcctgcc 840
   gtggacaacc cgttcttgtt cctaggtgac tatgtggacc gtggcgtgtc ttcgtgcgag 900
   ataatcttat tgctcttggc cttcaaagtg gctttcccgg acagtgtcca tttattgcga 960
   ggcaatcacg agtgtcggag tttgagtaca ttttacggtt tccgtgccga atgcctcaag 1020
   aagtacggac ctgtgctcta taaccgtatg atcaagtgtt tcgagagtat gccactggct 1080
   gcacgactcg agacagcgca cggcacgttc ctggctgtac atggcggact gtcgcccgat 1140
   attcagttcg tgggggacat taacggacaa gtgaaccgct ttatggagcc tgaaccaaac 1200
   ggagctctgt gcgacttgtt gtggtcagat cctgcgaagg gcgaagctca ggagcaggag 1260
   tgggcaccca atgggatgcg tggctgctcg ttcacattta atgaacgcgc ctgtcgcgaa 1320
   tttctgaagc gcaacaacct cctggccatt gtacgtgctc atgaactgga agaaaatggt 1380
   tataaggagc actttcgaca cgaaggagac cgcacagaag aagacgagga cgggaaactg 1440
   gctctacctg cggttgtgac ggtgttttca gctccggagt actgcaacac gaaccacaat 1500
   gtcggcgcga cgctgaaaat tccctgggaa agacaaaatg gtcgtctgct gcagtatcag 1560
   cagcataaac gcagtcaatg ttccgagttt gagttcacgc gagccagcga ggagacggcg 1620
   gccaaggcgt ttctggagga gaatttgccg ttcttgccga ttgactttta cgacttggtg 1680
   aatgtgtgtc ggcagctgcg gctcactctt gagagagcag cgagtatcac cccagcacct 1740
   gcttcagaac ctattcgcaa gctgtcgctt gtgtcgtcgc tgtgtgcgcc agcaactagt 1800
   ctggaaacga ggatcgagga ggaagaccca gagcttcccg tgtcgccacc tgcttctgca 1860
   ccagcaaatg aagtggagaa ggaagctaca gcaactctga aggcagtggg cgagtcgttg 1920
   caggattggg aacttgttga gacgaagagt gtcgaggttg acactaggaa ggtgaagaaa 1980
   gacaagaaga aggagaaaaa ggagaaaaaa ctgaaggaga agatcgaaaa gaagaagcaa 2040
   aaggacaaga agaagtggga tacgagtcga attgctagcg gttggaagct ctgccctggg 2100
   tttgtacgct tctacgaccg ctacttctca agagacagta tgaagaagag cgagccggag 2160
   atcacctcag ctcctggcca tctaggcaag cggccgtcgt ggatctcaaa ctacaaaaca 2220
   ccttttagac gctcgacttc gaccggcgag gcccccaccg acactgaaac tgtcgacacc 2280
   gtcttggact ctaccggtcc tgctcgacgg aagagtatga cggattggat gcccatgccg 2340
   tcgttcgagc aggtggcgca gctgacgaac acgcttcagg gtcacattcc cgtgcagtcg 2400
   aacggcgtca atgtattcac gaaggcacag tggcaagctc tgaagttgta cttctcgatc 2460
   ctggatctgg atggcaatgg cgttttgatg gaagagagct tcgttgttct cctcgcagag 2520
   caagacagcg gtgggttgag ttaaagtgaa gtgaggatgt gaatttgttg ctaatttaaa 2580
   ttgttgttgg tgttgtgtgc agatgcatac gcgactgaag acgaactgag tctgttgatg 2640
   gaagtcatgg actgcaacgc cgacaatatg atcactgagc aagatttctt gctattcgca 2700
   taccgagcct tactgcgatg gaaaaaagct ctacatggct ccgactagag tgtgcaggag 2760
   cggaatcgaa ctgacgtagc gaagtatgaa caatgccaga ttagaatcca cagtagcgcg 2820
   gcatatgccc ataatgaaga acgagaaatc tattagttga aa 2862
<210> 23
   <211> 984
   <212> DNA
   <213> Phytophthora infestans
<400> 23
   atgtccgcgc cgaccttcca tcgatcattc cgcaatttgc agcttccaag tcaacagtcc 60
   aagctccaaa ccgaaacaag agtgatgatc gcaatttcga cagctgagga tttccactct 120
   gccgtggccg ctgcgactca gaagccgttg gttgcctgtt tctccgcgcc gtggtgcggc 180
   ggttgcaagt tggtggcgcc caaggtggac aagctagccg aggagcttgc cgagaccgtc 240
   tcgtttgcca gggtgagtgc tgaggagcac gagacgttat gcgaagaagt ggaagtggac 300
   agcttcccgc acttccgcgt gtacagggac ggcaagattg tgggtgacta cacgagctcc 360
   aagttcgaca aggtggagtc ctttatccgc ggccatgtgg ctcctaatac tgtgcaggag 420
   accgaggaaa cacctgaagg cgaggttacg gaggaaacgg cggagaagga tgctgcgaaa 480
   gaggaggaag tgtcagctga ggctgcgacg gaaggctcaa agaagcgcca ggagcgtgaa 540
   gaggaggaga cgaatgacga gcaggtggct aagaaggcca agacggagga ggaggcgtcg 600
   acggaggctg agaaggctgc aactacggag ccagctgaga aggttgagga agcggcaacg 660
   gaggagaccg agacggctga aaaggattca tcagaagaga agatcgaagc tgctcccgtt 720
   gagaagtctg aaacggccgg gaaagacact gctccagtcg atgctgctgc tgcttaaacg 780
   acactgctct gctttgcatg atgtctaaat cgacggacat cttctgtggg ctagctgggg 840
   gctgattgat ccagctgaaa tcaagatgaa acaataaaaa aagaagtatt ttatggcgat 900
   tcttttgttt gtgcccgata tcctcgacat gttctgaata catgtacatg tgctctgcct 960
   tcttgtcaga acaatgtgga atgc 984
<210> 24
   <211> 501
   <212> DNA
   <213> Phytophthora infestans
<400> 24
   caagcagtga agctaaggaa caacaagatc aaacaaacca tacacctatc tacgatgcct 60
   gtcgagatca acacccccga agagttcaac gccgctatcg gcgagaagaa gctgacggtc 120
   gtgcagttct ctgcgccgtg gtgcggcggc tgcaagatgg tggcccccaa ggtgaccaaa 180
   ctgatggagt cggacttcgc tgacgtcaag ttcctcaagg tgagcgcgga ggagctagag 240
   gatttctgcg aggagatcga cgtcgacagc ttcccaacgt tccgcgtgta caaggacggc 300
   gaagtggcgg cgtcttacgt gagctccaag tttgagaagg tagagcagtt catccgtgag 360
   aatgcgaagt aagcgtgacg gcgatttgcg accgtctatc aaagctcgcg catggtgatc 420
   tacaggcttg atgacctacg ctaagatggt ggagagcgag tgagtttggt ccccggcatt 480
   gcgtatcggc agtggactga a 501
<210> 25
   <211> 734
   <212> DNA
   <213> Phytophthora infestans
<400> 25
   gacaagcagt gaagctaagg aacaacaaga tcaaacaaac catacaccca tctaagatgc 60
   ctgtcgagat caacaccccc gaagagttca acgccgctat cggcgagaag aagctgacgg 120
   tcgtgcagtt ctctgcgccg tggtgcggcg gctgcaagat ggtggccccc aaggtgacca 180
   aactgatgga gtcggacttc gctgacgtca agttcctcaa ggtgagcgcg gaggagctag 240
   aggatttctg cgaggagatc gacgtcgaca gcttcccaac gttccgcgtg tacaaggacg 300
   gcgaagtggc ggcgtcttac gtgagctcca agtttgagaa ggtagagcag ttcatccgtg 360
   agaatgcgaa gtaagcgtga cggcgatttg cgaccgtcta tcaaagctcg cgcatggtga 420
   tctacaagct tgatgaccta cgctaagatg gtggagagcg agtgagtttg gtccccggca 480
   ttgcgtatcg gcagtggact gaaggatgct actaatatat tgaaatagga aaccatttta 540
   acgaagaccc tgagcctcag gctcattctc aaccggaaat atagaccgca tgcggtccca 600
   ggaggacaaa tagagtcttc taattggctt acggggtagg tggtcgcatg cgacgaagtt 660
   cataaaaaga cgctcatgcg gcaatttgcc cgcatgcgct aaacttgccg cacagtagag 720
   tgtaggggtc gagt 734
<210> 26
   <211> 1448
   <212> DNA
   <213> Phytophthora infestans
<400> 26
   ctcgagccca agcgacaatg gcggcggtga ctggcagtgt ggtaagcgtt cagagtgtgg 60
   cgcagttcga cgaggcaacg gcgcgcgagt cgacgctatc cgtgagtttc ttctgggccg 120
   aatttcacga ggcctgccgt cccaatggac agctggacgt ggtggtgcgc cagctcgcga 180
   ctctgcaccc tcgcatccgc ttcctcaagg tggcggccga ggagattccg gagctctcgg 240
   agcgttttca gatcgccgta gtgcccactt tcgtcgtcgc acagggtcgc gccgtgctgg 300
   acaaactcga gggcgccaat gtggccgaac tggccaagcg cgtggatgtt ctgagcaaga 360
   gcgtggccaa gagcagctcc acatccgcgt ccacagccga ggacgccccg aaaccgctgg 420
   atgacgcgct cgaataccgt ctcaagaagc tcatcagcgc gtcacccgtc atgctcttca 480
   tgaagggcaa tccgacggag cccaagtgcg gctttagtcg acagatggtg gcgctgttga 540
   acgaagaaaa gatccaattc ggcacctttg acatcctcaa tgacgacgaa gtgcgtcaag 600
   gactcaagca gttttccaac tggcccactt accctcagct ctatgtgaat ggctcgctca 660
   ttggcggact ggacatcgtc aaggagatga agtccgaggg atccatcgtc gagcaactag 720
   gactcacgaa gaacgtcgag gaggctgaag ctgccttcca agagagtctc cgtgcactcg 780
   tcaactccgc tccagtgctg ctcttcatga aaggccatcc gtccgagccc aaatgcggct 840
   ttagcaagaa gacagtcaag ctcctacgtg accaccagat tggcttcagt tcgttcgaca 900
   tcttgagtga cgagcaggtg cgtcaaggtc ttaagaagtt ctccaactgg cctacgtacc 960
   cgcagctgta cgtcaagggc aaactcgttg gtgggctgga tatcctgaac gagatggcgg 1020
   aggacggcga cctcagtgaa cagcttggcg tggagaagaa ggccaagaag gagaacaaat 1080
   acgaacagct aatcaaccgc gctcgcgtca tgatcttcat caagggaacg ccccagcagc 1140
   cgcagtgtgg cttcagtcgc aagctcgtgg acatcctgga cgccgagggc ttcaagtacg 1200
   actactttga catcctcacg gacgacagcg tgcgtcaggg actgaaggag cactcgaact 1260
   ggcccacgtt cccgcagctt tacgtcaatg gcgagttgat cggcggactg gacatcgtgc 1320
   agcagctgca ggaggacgga gaactcgccg agctcaagga gtagacgacc aggtcatcct 1380
   tcgcgtaaaa cgaatacaga aaatcatttc tcttatcctg catgtgtatg ctgggtttga 1440
   tggtttaa 1448
<210> 27
   <211> 1106
   <212> DNA
   <213> Phytophthora infestans
<400> 27
   gtcggaagag gtcgtcggtc gtccatgcag ctgcagacgt tggcgtacgc catctccggc 60
   gccttcacgc tgctttccat tatcctttcg ggatggctca tctggacaca cttgctgtac 120
   aatccgtcag ccggcatccg caagcacgtg atccgcatcc tcatgatggt ccccatttac 180
   gcgctaacgt cctacatggc gctggtattc aacgagtcca aactgttgtt cgagactgtg 240
   cgcgatctgt acgaagcctt cgcgctctat tcgtttcact gcttcctggt cgagtatctg 300
   ggtggccaat ccgttctagc aagcaccatg cgctccaagc cgcagatgac acacgtcttc 360
   cctttctgct gtgtacagcc gtggtccatg ggcggcaagt tcctgcgtca aaccaccatc 420
   ggcatcttgc agtacattcc cattaagctg cttatgagca tcgtcatgct catcacgagc 480
   ttggcaggtg tatacggaga aggagagctc atgaaccccc tagtgagcta cggctacgtg 540
   tgctttatcc tcagtgcgtc gcagacgtgg gcactttact gtctgctgat attcttccac 600
   ggagctcacg aggagttgca gcccatgcgg ccatggccca agttcctggc cattaaggca 660
   attattttct ttacgtactg gcagtcgatc atgattagtg gacttgtaag tgtcggggtc 720
   atctcggaga agtggcatat cggctgtccg gactgctggg acgctcagaa aatcgcctcc 780
   gcactgaatg actttgtcat ctgcgtcgag atgctgggct ttgccattgc ccaccactac 840
   gccttcgcga ttgaagactt tttgtcgccg tcaggtacgg caggtgttag tgtgccgtct 900
   tcgaacgtca aggcaccact gctggccaat tttatggacg ctatcaacgt gacggacgtg 960
   tcgacagacc tcaagaactc ccggaacgag attctcacca agaaacaggc gctggctgcc 1020
   aagttcgagc gcatgaactc gacttcacct ggtggtggca tgttttaata gcgtcagtta 1080
   aaagggagcg atacagtaag cacacg 1106
<210> 28
   <211> 2007
   <212> DNA
   <213> Phytophthora infestans
<400> 28
   atgagctcac aggacggcgc tggtcgcgca tctgaggaca agaatggaga cggcgtgtac 60
   gtgacgaaga accgctcgct cttctccatg tggctgcacg gcaaggcgat tccaagccgt 120
   tctggtccgg ccgtggtgtt ccgctcggcc gacgtgatac aggaagggta tttgctcaag 180
   cagggcctgc gacttaaaat gtggtcccgc cgctacttta tactgcgact cgaggagcga 240
   cacatgactc tagggtatta taccagcaag gactctctga ctttatgctc cgagacgccc 300
   atcggaccag gacacttgtt gggacacgtc aacacgacca aatacccgcg tcgtctcgag 360
   ttgcgctgcg gtacaaaggt catggtactc gaggcggagg accaaaagtc gtatgaagcc 420
   tggaagaacg ccctccaaga agcgatacgc tggaaccacg ccatggtgcc ttcaaaagac 480
   ggcagttttg tcacgtacgg gaagcaagcg accgaggata taaaacaaga ggaacgcagt 540
   cgcgccgagg cggccaagaa gctgcgagag aagcagcgag cggacgaggc cgccgccgcc 600
   gccaacgccg ccaataaacc caaatatctg cccgcgacac gccccgggac gcaatgcttt 660
   atgacatcga atacaagatt tgagattccc tctcatttcg aatatgtcaa aaccatcggc 720
   tcgggcgcct acggagtcgt catctccgct acgagctcgc aaacaggcac cacggtggca 780
   attaagaaca tccagcgcgc gttcgacgac ctgacggacg ccaagcgcat tgtacgcgag 840
   attaagctca tgcgccactt gaaccacaag tgcgtgcttg gggtggagga cattttcgag 900
   cccgtggcgc tgtccaagtt cgaagacgtg tacattgtgt cccaattgat ggctacagat 960
   ctccaccgcg tcatctactc gagacacgcg ctgtcggacg aacacatcgc cttcttcatg 1020
   taccagatgc tgtgtgccat gaagtacgtg cactcggcca acgtgatcca ccgagacctg 1080
   aagccgtcca acgtcctggt gaacgccaac tgcgagctca agatctgcga cttcggactg 1140
   gcgagaggcg ttttccccga agaagagctg gaattgacag aatacgtagt cacaagatgg 1200
   taccgagcgc cggagatcat gctggggtgt atgaagtaca ctcgggaggt ggacgtctgg 1260
   tccatgggct gtatcttcgc cgagatgatg tcgcgcaagc ctcttttccc gggacaggac 1320
   tacattgatc agctgcatct catcatgaac gcgctggggg ctccaaacga ccaggatctc 1380
   tactttttga gcaacgcgcg tgctaggaag ttcatgaacg ccgagttcca gaagcgcgga 1440
   cccaacccga cgaagcctct ggcgcacatg ttcgcagatt cgcctccaga cgctctggat 1500
   ttactgcaga agatgctggt gattgacccg aataagcgga ttagtgtgga cgaggcgctg 1560
   gcccatccgt acttggctgc gatccggaat gtggaggacg agacgaccgc cacttcgagc 1620
   ttcgattttg actttgagaa cgagaaattg acgaaacccg tgctgcagag actgatctgg 1680
   gacgagatga ggcatttcca ccctgaagtg ggcgacgaga cagcgacgga gggagatgac 1740
   agcagtgtcg ctaccacgca ggcttctatc acacccgtga cacctgtgac ccccgctacg 1800
   gtagagcaag acacaacaga gacgacaagt gacagctcgg acgcccctgt caaggtatcg 1860
   acgccaacag cgtcagagga agccaagcca gaagacgaag acggggaaca acacagcacc 1920
   aatagcgaca agatccatag gacagacaaa ctgacagacg cacagacgcg acaagaggcc 1980
   ggcgaaccag ctcgggaagt cgcataa 2007
<210> 29
   <211> 2053
   <212> DNA
   <213> Phytophthora infestans
<400> 29
   gctcaagcca gagtccaaac gctcgttcaa cgcccattca acgctcgagc aacggctgat 60
   accgacatga accgtaagct ggagctcatg gagctggaca gcggcggcgc cagccctaac 120
   agcagcaaca gtggaagccc cagtccgact ctcgacctgc tgcatcgctc tgattcgagt 180
   agcagtaacc aggggggtga gatgcctctt gtcggggttg cccacaaccc agacgtacgt 240
   cgtctgcctt tctctatttg tagtgatagt attttcacgt agacgtttgt tacagctgag 300
   ttatttgagg gcaactgcaa cacatggagg cagcagcacg accagcagca agtatggcag 360
   ccccgaggag tcatttggtg aagaggacga ggacaacaac aagaagacgg cgcgtcgcaa 420
   gaccgactcc aacagcaagc gtccgtggac acgcgaggag aatgacaagc tcatgcaact 480
   cgtcaagcag tacggcgcca agcgctggtc tctcatcgcc atgcacctgc caggccgtgt 540
   cggtaagcaa tgccgtgaga gatggcacaa ccacttgaat ccgtcggtcc gcaaggacgc 600
   atggacggct gaagaggact atgtcatctt cgagtgccac aagaatgtgg gcaaccaatg 660
   ggccgagatt tccaagatgt tgcctggcag gacggacaat gccatcaaaa accgctacta 720
   ctcgaccatg cgtcgcatgc agaggcagtc aatccgcaaa aaaggtccca tgcgtgatgg 780
   caagagcatc cgcgtggcgt cagtcacgtc gtctcccgtg caaaacaaca accaaatggg 840
   tcctgctccc agccagcgat ctttgactgg catgcaacac cagttgccac cgcaacaaca 900
   gcttccgcac cgaggggtga gtttccaatc cacatatcaa cggctcttct cggaggcctc 960
   tggggacgcc gcacgcgtga gtcccgctgc ttcgaattaa ttggagagga tcgatactaa 1020
   ctattatggt gttgatatat aatgcaggat ggcaactcga tagtggactt tgaacgttca 1080
   aacatgatgt ccgcatcact acgacaaccc acgatggtct acccgctaaa cggaggtagc 1140
   ttttcaaacg ggatgaaccc ggactatagt cgtcccatgt ccatgacgtc gtctccctgc 1200
   tcggtgtccc ccgccgatga tctgaatcat aacagccaga acgagacctt tgactacgtt 1260
   ccgatgcaat cttcgatcca gcgtgtacgg tcatctagtc cggttgtgat gggcactcct 1320
   ccttcgacgt caataggcat gatgaactca ccttacggct cgcctgcaag tcacatccaa 1380
   cagcaacagc ctggcaacta catgatggca ggaaacccat attctaacaa caacgtgcgt 1440
   gggatgtact ttgggacgtc tgggcttgct cagcaatacc gtcctgatgc ccctgtaaac 1500
   gtcccacaca agcgcctcct cgacgtccag ggttcacaac gtgatatgtg gaagaatgat 1560
   agccctgtat cggtagcagc accgatattc cgcggtatgc cggcgacgca catgcagcaa 1620
   ggtcaagtca gcgaaccgat ggcattgcag cagtccaagc cagcttccat gccactgtat 1680
   aggcaagcgc cgaatatggg tcacttcaac agcatggagc aggtatggac ggatgatgca 1740
   tatctgtgat tggaccgcca ttgtggcatg ctcctgatag actgcatgaa acaaatttaa 1800
   agttattact taccaaagac agtggcttct cttactcttt acttggttaa cttttttcgc 1860
   ttgatctaca gcggtttcac gttaggtagc tgtagctcct gatcgagttg cttattaaat 1920
   agcaccactt gttgacaggg attgatatta aaatgaattt ttttcagact aatatattag 1980
   ctgatattac atgtaccaaa atgtcaaact aatagctgac tattagtttg aaaaaggcgc 2040
   caaaacaagc taa 2053
<210> 30
   <211> 1173
   <212> DNA
   <213> Phytophthora infestans
<400> 30
   atgcttggcg acgtagacag tttcggcggt ctgggtccca ttccgtccat gaacgcggtg 60
   ttggagaagc gcacgtcaag tcagttttcc attagctcgc tgctgccccc gtccgtgagt 120
   gtctcgagta ctggtgtgtt aaacacaact gttacacgga gcgagagcgc agtgacagat 180
   agtgttgaag caactaccga taatgaaggc caaggatctg caccaagggt gtctaccaca 240
   actgcagagg ggtctggtgg caacaccaag tacatggacc ccactacggg cgactacatg 300
   tacccggaca acgtcaagcc catgagcttc tacgacaaac aacgtcttgt gcagaagatt 360
   acgatgcttc ccagtcaata tctgcgtggc ctcatggacg tcatcagcaa gtaccagcca 420
   gatactgtac gtcagatcga cgacgatggc tacgcgttcg atctgggtca gatgaacgag 480
   aatactgtgt gggctatcag tgactacgta aaggactcga tgattgagct ggatggatac 540
   attaaatcac tcaaccaaac tgctattgac cttgctgccg gtgaggatcg acatggagaa 600
   actgttctga cggccgcaag tgcggctaca cccaccagca gtgcggttcg ccacttgacc 660
   gacactctgg cgatgaacac gtccaagatg tctgtcatca ctaataacga gaatcagaat 720
   aaattcctgg aacaggtgga gatgtattcc aaacccaaag tgaccaagtc gcgtgtaaag 780
   cccagcaaga agcaccagtg tccgacgtgt aacaagcaat tccgcggacg ctccgagctg 840
   cagaaccata tcagaactca cacaggcgag aagccactca aatgctcgta cgcgggatgc 900
   acgaagcgat atgcacacag ctcgaatctg cgtgcacacg agcgaacaca cgccggaata 960
   aagccgtata catgtcacta cgacggctgc gggaagagct tcgcccactc tgtatcactt 1020
   aaggaacata tttggatgca tgcaggattc cagccctacg tgtgtccgta cgagggatgc 1080
   cagaagaagt ttacgcaggt ctcaaatttt gcccgacaca agaagacgca cgagaaggaa 1140
   gacaacgaac actcgattga gagcgataat tag 1173
<210> 31
   <211> 754
   <212> DNA
   <213> Phytophthora infestans
<400> 31
   atgattaagc tcgcggccgg tgtgtccaag aaatccgtcg tcgatgtgta tgtcactctc 60
   agtgtgcccg acagccccgt acttagcacg gcacagaaga acgtggagct gaacgtcgag 120
   aagttcttcg tggtcagcaa gactctgcct gaattgcctt ccaggtagaa gacgctgccc 180
   gccccgacgc cttcgtcaag gcaacggatc ctcctacgtg aacgtcggtc tggaggatcg 240
   tctcaactcc cgtccgcttg acctgcatac acctgccaac cagtgtatca agcgcatcca 300
   ggccgctgtg ggccagctgt tccgtgcatt cctcatccag cgcgacttcg tggagaccca 360
   cacacccaag ctggtcgctg gcgcctcggg gagcggagcc aactgcttca cactcaagta 420
   cttcgattag gacgtcatct tggctcagag tccgcagaag tacaagcaga tggcgtgtgc 480
   tgctgctggt ctcgagcgcg tgttcgagat cggcccggtc ttctgtgctg agaactcgag 540
   cacgcaccgt cacatgtgcg agttcgtggg tctggatctg gagatgacta ttaaggagca 600
   ctaccacgag gtcctggagg tgttctcgga cctgtacatc tacattttcg atggcttgaa 660
   ggaacgttac gccaacgaat tggctactat caacaatcag tacccgttcg agccgctcaa 720
   gtacattaag ccgtcgctta tcatcaactt ctag 754
<210> 32
   <211> 659
   <212> DNA
   <213> Phytophthora infestans
<400> 32
   gggctccatg tcatctgcga ctttggacgg agctctctat tgtagaattc aactgactct 60
   agacaagtaa ccatgcagaa cgaggccggc cagtcgatcg acatctacat cccgcgcaag 120
   tggtgcgtat tgcgctggtg actttaatac gctcagagct actggagcta actggtgctt 180
   gctattgtct gtcatcgtta tatagctcgt ggaccaaccg catcctggcg gccaaggacc 240
   acgcctcggt gcagatcaac gtcggccgcg tgaacgccaa cggcgtcttc acgggtgagt 300
   cggacacctt cgctcttgct ggctacattc gccaccacgg tgagggcgac atggccatca 360
   ccgagctggc tcgccaggcg gacgccaaga actaagcaga ccaagttgtc gtgatcgcgc 420
   gctgctgctg cgaagtggag tgtgcgttca tctacgtcta gctggcagtt caggaggctg 480
   gtgattacga tatgtatcaa cagatacgtc tgtgatcgct ggcaggaatt gcggtaccgt 540
   cgcttatgga gtcggttgtt tgcgggttcg acctcgcctt ttgatttgat cgggtgtagg 600
   cttagtattt aactctggcc aacggacgag caagaataaa gtagttcaaa cccgtcaga 659
<210> 33
   <211> 1049
   <212> DNA
   <213> Phytophthora infestans
<400> 33
   ctcgagcgca agacgatctc cgctatcgcc ccaccatgtt cgccagcttc gccactgccc 60
   tcatcgcttc cactctccgg ccctcgagcg cagccgccac caactatgcc ggtggctggc 120
   ttccgtcgga ccccaccaac cagtgtgtgg atatttgctc cgctccgtcc aagacgtgcc 180
   tcacttcgga cgctgcctgc ctcgccaagt cgatggtccc gggcgacttc gactatatgg 240
   tcctggagca gctcttcgtg ccgcagttct gccgtgacct gctaaagggt gtcgactcca 300
   ctatttcgca ccaaaacatc gatatgtacc cgaacggcac agcctgcgtg gagagtgtgg 360
   tcaagagtga gctcacgatt cacggtctgt ggcccaatta caacgatggc tacgtgagct 420
   gctgcaatcc gagctcagcg gtggccaacg acccttataa cgccgctgac ttcgctgccg 480
   cccaaagcag cctactcacc accatgggtg agaagtgggt ggacgctacg caagccacta 540
   cgtacgaatc gctatgcgag atctacaacc acgagttcca gaagcacggt ctctgctatg 600
   ccgctgacga cgctgattac atctcggctg ctgtcacgta cttcactgct acgctgagca 660
   cggcggaccg catcagttca gccaccgagc agatcaacaa gtgggcagct cagtcgacgc 720
   ctcagacgac tctggccgag attgaggctc tctacggcca cagtgtcatg gtgttgtgct 780
   cggctgtgga tggcaacaac caactgtcgg ctatccgtac gtgctacgag aagccgacaa 840
   acatcacgag cgagggagcg tccgcgcaga ttgactgcgc agccgcgacg gccacctcct 900
   cgttctccgt atgctccggt gactcgccga tcactctgac tgcatacaca gcccccacct 960
   cggcttcgat gcaataagct ccgacgatag agcgacggtg cagtctgcta gtataaagtt 1020
   caatgcacca gttacttaaa aatgtacca 1049
<210> 34
   <211> 1203
   <212> DNA
   <213> Phytophthora infestans
<400> 34
   atgaccaagt ggggggttgt agtggcacta acactgctgt tagcgacgcc tagtttagtg 60
   ctaggcgcct gccccaacaa gtgttccgga cacggcaaat gcggtctcaa tgacgtctgc 120
   caatgcatgc agaactgggt cggcggtgat tgctcgggtc ggcaatgtgc gttcactcgc 180
   gcgtggcaag atacagcaca gcgcgacgac gacgcacact accacgcaga atgtggcagc 240
   cgcggaacgt gtgatcgagc tactggagaa tgcacatgtg acgcaggatt catcggcagc 300
   ggctgtcgac gaatgcagtg tcccaatgac tgcagtggcc acggcacgtg tgagtttatt 360
   gaggaattgg cgacggatac ggcccacaag cggattgggg gagtggcagg tcgtaaatac 420
   acgctttggg accaagagaa gatcatgggc tgcgtatgtg acgccaacta cgaaggtcac 480
   gattgctcga tgcgctcgtg tcctagaggc gacgatcctc tgaccccgaa tcaatacgac 540
   atggtgcaag ctattattct tgataaggct ggcggtgagg ggtacttgac gtactatgat 600
   ccgtacggca atgcgtatac tacggagaaa atcacgcttg gtggaacgct gagtgtcgct 660
   tttcaaccaa cagacgatga taccacatgt gcaaacattc agaaggcgct ccgccgttta 720
   ccgaacaacg tgctcaacac agtcactgtg gtagctgttg acaggtttta tgccttcaag 780
   cgctctgacc caacggactc gttggggtat ggaacgttaa acaaaattgt aaatgacgac 840
   gctgcagcgt acgcaggaac cggaactcaa attaaagcga tgtgcgaggt gatttttacg 900
   tcggagccgg gcactacggg gtatcagaat ttgctggatt gcaatgttgc cgcgcatggc 960
   gatactaagg gccagcaccc gcttactact ggtgtaacgt ctggcacttg cgtcgttaaa 1020
   gaagtctatc cggtgacgct tggcacgagc aacatgcttg ccgaagatac tccagcgtat 1080
   cgtccattga ccgagctcac cgagtgtgct ggtcgcggca cctgcgacta cgacacagga 1140
   acatgcgagt gcttcgcggg ccacatgggc ttggcatgcc agaagcagga agcgctcgtc 1200
   tag 1203
<210> 35
   <211> 7947
   <212> DNA
   <213> Phytophthora infestans
<400> 35
   atggcgcgcg ctccggcaca attggcgcag cttttactag cggcgctgct gctgtcagcg 60
   atgtgtgagg cgttattgcc tatcacgaac gcgctgcagc gtgcccgcca aaacctcacg 120
   gccttgcagc ggcaatggat cgatccggac acggacccca agttctacaa catatcagtc 180
   cccaagggtg ccttcaactt cggtggcaac tcgagctaca acaacgagta caagctcatc 240
   ttctcggacg agtttaatag ctccaagcgc acgttcgagt cgggtttcga ctccaaatgg 300
   acggcagtca acctccgcga taccaccaac atggggcagc actacttttt gccccaggct 360
   gtgcagatcg acaagggcaa cctcatcatc acgacctcca agcccaaaca gcgctaccgt 420
   ggcactaagt acgtcagcgg tgctgtgcag acatggaaca agttctgtta caccggcggc 480
   tacgtcgagg taagggccat tctgcccggc aagtggggta tccccggcac gtggcctgct 540
   atctggatca tgggcaatat cggccgtgcg ccattcctcg gatcccagga cggtacgtgg 600
   ccgtggagct tcgactattg cgccccctac gtggagaagg ccgagaaggt caagcagaag 660
   atcaatgcct gtggcaatct gaccaacaaa cacgacaagg aatcgtatcc agagatgtac 720
   ggcttgaacc cctttcaggg tcgtggagcc acggaaatcg atgtgattga agcgcagatt 780
   cgtgctcgtg atgagcctgc attcatctcc acgtccctgc agatccgtcc cagtctgtac 840
   gatgatatgc gcccggcttc agagtcgctc cctgctcctg gacaatggta ccagggtctc 900
   aagtttggtg agttcacgaa aatcaatagc gactactacg gtgagatggg cctcgactcc 960
   atttcggctc tgacacagct cgagtccaat gcgttcaagt cgttccactt gttccgcctg 1020
   gactggtctc ctggtcccga aggttacatt cgatggtgga tggacaacag cttcgtgttc 1080
   gaaatccctg gatccgcgct gaataagtgg gtaggtgctg tcccccctcg ccagatcccc      1140
   gtggagccta gttatctcat tctgagtacg gctgtttcgg agaagttctc tccaccgtgc      1200
   gatgggcaga tttgtaactc gctatggccc tccaacttta cgatcgacta cgtacgcgtc      1260
   tatcaaggca acccgaaccg gtatacttcg gtgggatgca atccggaagc ctacccgacg      1320
   aaagattgga tctacgccca cccagtggaa tacggtctcc cgtggtatgt ttcactacgt      1380
   gtggacatcg gtctgctgca tcttttcgct gtggttaatg cgctgctggg tctcttcatg      1440
   gcgttccgtg gcacgtccca accgaagatg ttgtcagcgt acgccagctc gttgtggctg      1500
   actgcagcct tttatggagc tcttagttca agcgcccccg tagaccttgc ttggattcag      1560
   actgcgcttg cgtgtctgtg cgggctggta cttggcggtc tttgttgctt ggtgtacccg      1620
   gtttcgcttg gtgcaatgct tggactgtat ggcggggtga tagctagcca attcgtgcct      1680
   ttgctgtcta cgcgcgtgat cactgcatta ctggttggtg tgggaatcgc gctaggatct      1740
   gctccacaga tcgacacgaa gcacgttgtg attctgtcga cctctttgct tggtagtttg      1800
   gcgtttctgc tctctgtatc gctgtgggtg agtgaaggcg atattgctga gaatgcgtgg      1860
   aatctcgctg gcttcatttt caatggtaac aacgatgacc acgtgggttt ctgcacgaaa      1920
   tattgcctgg cgatgtatat tctgcttgtc gtactcagtg cggtgagcac tacgtatgga      1980
   tacctccgta tgcgtggcgt gacgctgcgc accaacccgc gtgaggccaa gttccccgcg      2040
   gtgtcagcaa gttctgacgc gcgggcttgg agacctgatg atgatgcggg cgtggagaag      2100
   actacggtga attttttctc gccatccaag ctgccgtaca acatgcagca gttcagtacc      2160
   atcttccgta tcgctgtgaa cgtgcagcgc tcgtttggct tccaacttga caacttccgc      2220
   aaccagacgg aacacgttgt ggtgcttctc accaacaact cgcgaaagag cggaaatcct      2280
   taccgcaagc tgcacgattt ggtgttttcc aactacaaca actggtgctg caagcttaag      2340
   atccagcctc tgaactgggg cgagcagcga ccaccgcagg gtggtctcac aatggtggac      2400
   gagatgtcgg tggacttgtg tctgttcttc tttatctggg gtgaggccag caacctgcgt 2460
   cactcgcctg agttcctgtg tttcctgttt cacaagatga aagaagagtt cccgtccgtc 2520
   cgtcactcag agcgcgaggc tggatacttt ttggatacgg tggtgacacc tgtctacggc 2580
   ttgctgaagg ctgagatgac ttcaaagtac gaccatgagg accgtcacaa ctacgacgac 2640
   ttcaacgagt tcttctggac gaagagatgt ctgaagtatg actacaaaca cgaagaggtc 2700
   attgatttgg cgtcacccaa tccggctatg atctacaaac agaagcagca gcaacgtcaa 2760
   ggtctgactg gccttggagc ccaaaaggct cgaggtggac tcaacggtgg ctcgaacggc 2820
   tccaacttgt tcaacaagcg tcaaagtatt gccgagggat tcaccgagtc tgccaagacg 2880
   ttcgttgaga agcgtacgtg gctgctaccg ctgcgcgctt tcaaccgtat cttcaacttc 2940
   cacgtcatcg cgttccactt cttggcaatg ctcgcgttcg cgaatgagca agagatggac 3000
   ttccaggacg cctgcaagat tatctcgagc actttgatat ctcacttctt gctggacatt 3060
   ttacgtgatg gactcgacat tttcgctgtt tacgacgagc accggaaagt attctcaatg 3120
   gcgcgttccg tgatgcgtgt gtttctgcat ctggctcttg tggtggtcac gtcgatgtta 3180
   tactggtatg cgtgggcgta cggtggtgcc tggtggcagt cgtactacgt gaccgcggtg 3240
   ctattccacg tgccaggcct gattaactgc gtcatgcaag tgatgcctgg tcttaccaac 3300
   tggacacggc gcacggcgtt tgctcctgtt gcatttatcc gtgacattgt gagtccgatg 3360
   aaccgcttat acgtgggtga caacgtgctg gatccggagt cgatgagcgt aggctaccag 3420
   ttcttctgga tgtcgctatt ggcttggaag ttatacttcg gctacgagtt tgagatctac 3480
   ccgcttgtgg tgccgagctt tctgctgtat gctgaccacg tggagaacaa cgtgagcatg 3540
   attacgacag tgttcctcat cttcctaaac tggatgccgt tcttcttggt gttctgcgtt 3600
   gacattacga tttggaactc gatctggatg gcattcacgg gtacgttcgt tggcttttcg 3660
   tcgcgcattg gtgagattcg caacttcacc cgcgttcgat ctgcgttcag tcgtgctgtg 3720
   gatgcattta acgcaaaggt gattgcgcga agctccaaga cgggacttca actctcggac 3780
   agcaatggca cgtcgtatgg atcgacatca gtaggtcacg aggtgcttga tcgtgttgcc 3840
   ggtggtgcgg atccgacgtc ccgcctcctg ttgcagcgcc ggacatcagc ccatgacgac 3900
   gagactccgt tactgtcttt ctcgcgtcgc aaacagacgc ctacggagcg ccaagctgct 3960
   cgtcgccgca agtggttctc gttctctgtg gcctgggaca ctatcatcga tagcatgcgc 4020
   gcggatgatt tgatctcaaa caaggagaaa tctctgcttc atttccaccg tcttgacggc 4080
   taccagcgcg aaatttacct gccgcagttc caacttgctg gttgcttcga gaactttacg 4140
   tcgcacattc ttgatattta ctcgtcgaac aacggcaagg tctcggagcg tgtgctgcaa 4200
   gacaaactac tggaaattct cagtgataac ccaatggtgg aggagtcact tgaagagata 4260
   tgggagcttg cgaactgggt gctggttaat gtgcttggtc cctgtcacgc gaatgatgtg 4320
   aagtacatca catgtgtgct caactcgtgg gccgctcgtg gtgtgttccg tgcgctgaac 4380
   ttgcaaaagg tggctccatg tggccgcgct ttggcgggtt tgatctcgct cttgaaggcc 4440
   aacgtccgag gatggaagag caacgccaag gttatccctg ttcgcaaaga cccatccgac 4500
   tacgcttcgt acgagtttcc gcaacagtct agctcctacc gtccttcgtc ggggcttact 4560
   aagtctgcaa gtacgacagg tctgtcgtcg ttgggtctcg aaccacctcg tcgtagtcgt 4620
   ggctctggtg ttgcgcgtat tgcacgtatg cagcagcaga cgcacaaacc agctgtcaac 4680
   aatggcaagc ttactcattc tatctccagc tctcacatca tgcagattcg cgagcgcgtg 4740
   cgtacgttcc tgaatcttgg aaaggagatt ctggcccacg tccacgagca agaccccgtg 4800
   ttcgctgaaa gcaagggaat ttcggatcgg ttgacgtgga ttcttacaca ggagcgtggt 4860
   tttatgtggg acgataatta tacgggtgaa caaatcactc tcacagcgtt tgagagccac 4920
   accgatgtgg tgttatcgca tctgcacgga cttttgacct tgcagaagat tgatgcggag 4980
   ccccagtcgt acgatgctcg tcgccgcttg ctgttcttcg tgaattcgct gttcatggac 5040
   atgccgcttg ctccgctgct cgaggaaatg aagtcgtgga gcgtcatcac tccgttctat 5100
   gccgaagacg ttctgtactc cagaaaggat ttggaaagca aacaggacgg tctggacgtg 5160
   cacacgctgt tgttcttgca gacgctgtac aagcgagact gggagaactt cttggagcgt 5220
   gtgaagccta agaagaacat ctggaaagac ccggagactg cgatcgagtt gcgtatgtgg 5280
   gcttctctgc gtggccagac actgtcacgt acggtgcagg gtatgatgta cggtgaagct 5340
   gccattcgtt tgctggctga gatcgaacaa gttccccaac agaaacttga ggagttgatc 5400
   aacacaaagt tcacgtacgt ggtggcctgc caaatttatg gacgtcagaa gaagaacaac 5460
   gacccgaagg cgagtgacat tgagtttttg ctgcaccgat tccctaactt gcgcgtggca 5520
   tacatcgatg aggtccgtgt gaactaccaa aaggaacagt catacttctc ggtgctcatc 5580
   aagggcggcg aggaactcgg ctcagttcac gagatctacc gcgtgcgtct gcctggcaat 5640
   cctatcttgg gcgagggcaa acctgagaac cagaacgcag ccattgtttt cactcgcggt 5700
   gaaaatctgc aggctatcga tatgaaccag gatggatatc ttgaagagaa cttgaagatg 5760
   cgaaacctac tcgaagagtt tgacaagggt acggcagacc ggccgtacac gatcgtgggt 5820
   atcccggagc acatattcac gggtagtgtg agctcgctgg caaactacat ggcgctgcag 5880
   gagacgtcgt ttgtgacgct aagtcaacgt acgttggcgc gtccgctgcg tagccgtctg 5940
   cactacggtc atcccgatgt gttcaacaaa cttttcttca taacgcgtgg cggtattagc 6000
   aaggccagta agggtatcaa cctcagtgaa gatatctttg ctggctacaa caattgtatg 6060
   cgtggcggtt ccgtgacttt cccggagtac accaagtgcg gcaagggacg tgatgtggga 6120
   atgcagcaga tctacaagtt cgaggcaaag ttagcgcagg gtgcagctga gcaatcgcta 6180
   tcgcgtgacg tgtaccgtat tagccagcgt ctcgactttt tcaagttgtt gtcgttctac 6240
   tacaaccatg tgggcttcta cctggcgatg tcaatcatca tctggactgt gtactttctg 6300
   ctgtactgca acttattgcg tgcactgctg tcggttgagg gtgttggcgg tcgtgaaccg 6360
   gtattgctaa gtaagctgca gttgatgctt ggatcggtgg cattcttcac tactgcgcca 6420
   ctgctggcga cgatttcagt cgagcgtggc tttaaggcgg cgttgaacga gatcattgtc 6480
   ctgttcgtga ctggaggccc gctgtacttc cttttccaca ttggcacgaa atggttctac 6540
   ttcggacaga cgattcttgc tggtggcgcc aagtatcgtg cgaccggccg tggattcgtg 6600
   acaaagcact cttcttttga tgagctttat cgtttctacg ctagcagcca cctctatgct 6660
   gcagtggaga ttgccattgg gctttccgtt tactacaagt tcacggtcgg caatcagtac 6720
   ttcgcgctga catggtcgct atggcttgtg ttcgtgtcat ggtactggtc gccgttctgg 6780
   ttcaacccac tggcgttcga atggtctgac gttatggagg acttccgtct atggttcaaa 6840
   tggatgcgtg gtgacggtgg taaccctgat caatcgtggg aggcgtggtt caaagaggag 6900
   aacgcgtact tttcgacgct tcgaccgtgg tccaaggcgt gcattacgat caagggcgtg 6960
   ctgttcgcgt tgatcgccgt ctctatctct tcgacgagtg acaaatatca ctcgatcttg 7020
   acggaaacca cgtggcttcc gctgcttatc tgcttatcga tggccgcggt gtatcttagt 7080
   gcagaggctg tcttcttcac ctcgtcgcgt tcgggcgaga ccgggcttgt tcgcttcctg 7140
   aagctccttc tggtgattgt gctgggcgct ggtctgattc tcgctttcat ctacgcggac 7200
   ggtatgtggc agatgctgct gagtatggga tatctcgctg cagctatggg ctgttgggcg 7260
   cttgtgatcc ttggtagcaa ctcgcgcttt gttggaacgc tttacttcgt tcatgacgcc 7320
   gtgctgggtt tggtttcgct gagtctcatt ctgttgctct cggcgctcta cgttccgggc 7380
   aagatccaga catggctact gtacaataat gctttgagtc gtggcgtggt gattgaagat 7440
   attctgcgag ccaactcgag caatgatgaa cgcgatgatg atctgtcagt gcagcagatg 7500
   cgctccatca tccttgagca gcagcgtttc atcaacgctc tgactgctag cggcagcgag 7560
   actgacatcc gcggtgctgg tcctgggaag aaagaagatc taatgcacgc tatgagcgac 7620
   aacacgctga acgcggtact gaggaatatg tcggagtcgg aactcagtgc gctgcaggac 7680
   tcgtcgattc gtctgcaggc catcatgtcg gaggaggagc gcaaggccgc acaacgcaag 7740
   cagcaacaag aagagcagag actcaccgag gcaggaatga actcgtcgtt gtcgcgcacg 7800
   cgtcgtgcct tctccacgag cgatttctcc gccatcccgc tgaatgctag cccctacagt 7860
   cttgctccta ctcaggatgg ttccgctgca cccgtcaacc gccccactaa cggttctgct 7920
   gctcacggag cttatcctga tgtgtag 7947
<210> 36
   <211> 6744
   <212> DNA
   <213> Phytophthora infestans
<400> 36
   atggccacga cgcccggcgg caagatggac cggcgccgcg ccggctccaa ctacttccag 60
   ctcgatgcgg accgcaagcg caacacgtcc aacaaccgca gcaactacgg cgacggccgt 120
   ggcaccttcg gagaccgctt gagcttgatg gaggtgcttc ctcccaacac acagggcggc 180
   aaccagatgc gcggcggtgg cttacagggc gactttgcgg acgaagtgtc gatcgatttt 240
   tgctgcgagg tactgtacaa caagttcggc ttccagtcag gcagtgtgga caaccagcgc 300
   gagcacgtgc tgctgctatt ggccaattcc aaggcgcgtg ccaagcccca ggaccctccg 360
   ggacaccacg tcgtcacgct gcacaaaaag ctcatgagca actacacgga atggtgccag 420
   ttcatcggcg ttcccagcat ctcgtactcg ggacagccac agggagacct caagaaccct 480
   ctgcacatgg acattatgct cttcctgttg ctatggggag aggctggtaa cttgaggcat 540
   atgcctgagt gcctctgcta cttgtaccac cagtcgctaa acttgctgaa ccaggacttc 600
   ctcggtcagc agaaagtacc tgaaggttgg tacttaaggc aggtggtgcg ccccatctgg 660
   aaggaggcgt ctaacatgca gaggaagaac agcttgggca agaacttgga gcacacccaa 720
   gtgcgcaact acgacgatat caacgagtat ttctggaaaa aatactgtct taacgtggat 780
   gtcacgcaga tcggcgagga gctgaccaag aagcacacca agacatacta cgagcaccgc 840
   agtatcttca cgctcgtact gaactactac cgtatttttc agttcaacat gatgttcatg 900
   atggtcctga tggcgatcgg ctttatctcg gccatctcgc ctagcggagg acagcagtgg 960
   ttcgctcagt tcgggtctat gggagaagtg gttgagcctt accagaaaca ggacgttaag 1020
   ctgacctacg tggggattgt gttcgccctc tcctcgatgg gattctgcaa gaccgtcctc 1080
   gaagcgtgtc acggatggca cttactcact gccagtgagt cgtcacagac gtcgtctcgt 1140
   tcgttcaact acggtggtgc ccttgtggtt cgaatgctct ggaatggcgc gttcgctggc 1200
   attttcggct tgatgatcta caccccgttg atcacgagca agaacacaga actgctcgat 1260
   aaggctgcac cggcgtctgt tgcctacatc ctgcctggcg cacttattat cgtcgtgcag 1320
   gcctttgctc catcggttgt aaccaaatcg tttgcggcca agtttatccg tgagggagaa 1380
   acgtgctacg ttggccgcaa catggcgcct ccactgagct accagctcaa gtacatcact 1440
   ttctggatca ttctgtgggc gctgaaggcg ttcgtctcat acttcattct agttcgccca 1500
   ttggttctcc cgtcgctggc catttacgag atggagctcg agtacggtag caatgttgtt 1560
   tcgttccaca acttcggagt catcgctgcg ctgtggctgc ctgtgatctt catcttcaac 1620
   tacgataccc agatttactt taccgtgttc caggctacac ttggtggcgt tcagggtctc 1680
   atcatgaaga ctggcgagat tcacggcatc aaggagatta ccaaggcttt ccgtgtggct 1740
   ccacaactct ttgaccagaa ggttgtgacc aatctggctc gctcgaacga cgctgcggct 1800
   gacggatctg ctgctgcata ccagtcgcaa atgatgctgc gcttcgtggt cgtctggaac 1860
   gagattgtca actcgttccg cgaaggtgac ctggtggacg acaaggaggc tgccatcctg 1920
   cagtatgaca tccagagctc gggcgacgtg ttcgaacctg tgttcctttc ggcgggtaaa 1980
   ctgatggaag ctctggacta cacggttaag attgccaagg aaggtaaggg cgactcgcag 2040
   cttcaagtgt acatggtgca gaaggattgt ctatcggctg tgcgcagctt cttcacagcc 2100
   agcatgtacg tgatggaggc tctgctgggc agtgacgatg cagacattct tgatgcgctg 2160
   cgtcaaatgg aggcgattgc tgcgaacagc agtttcatga gcacgtttga cgccaagagt 2220
   ctagtgcagc tgcgcacagt ctcgatggag ttcctggaag ctgtgatgga tctgcccgac 2280
   ccggatgcgc agtcctcgca catgacatcg tctcgagtgc acaccatggg agttgtgcgt 2340
   aacttcgtga ccaagatgga aaacctgctc aacgctattc gcattttcgc caaccgcccg 2400
   gagctcgctg ccaagttcag caactctaag ttctgctcga gcgccaacgg ctacgtgttt 2460
   gctgctcgtg gcctcgtgaa cctgttccac aacgacactg cgatgggtgc tgctacccgt 2520
   gcgtacctct tgatgtcgct cgagaaggcc gatgctatgc cccgtgtgcc tgaggctcaa 2580
   cgtcgtcttg gttttttcat gaagtctctt ttgatggaca tcccgcagtt gacgtctgtg 2640
   aaggagatgc actcgttctc cgtcgtgacg ccgttctaca gtgaaagtgt gttgatctca 2700
   ttgtcggagc tgaacgatcc gctggccaac cacccggtct tccagaaggt ggaggagaag 2760
   ggcaagaaca ttactattct gaagtatctg attaccatcc accccgagga gtgggagaac 2820
   tttttggaac gtattgatgt gagcactgca gaggaagcac aagccaacta cccgctggaa 2880
   atccgtctgt gggcgtcgta ccgtggtcaa accctggcac gtactgttca aggtatgatg 2940
   ctgtacgagg atgctatcaa gattcttcac tggcttgaga ttggttcaag tcccggcaag 3000
   tcggcggagc agaagcaggc tcaacttgag gacatggtgc gtctgaagtt ctcgtacatt 3060
   tgtgcgtgtc aggtgtacgg taagcatcgc gcagagggca aggcccaggc cgatgatatc 3120
   gactatctgc tcaagacgta cccgaacctg cgtgttgcct acgtcgacac catcgtgatg 3180
   gacggtggca agcagttcga cacggtgttg atcaagagtg aaggcaatga aattgccgaa 3240
   gtttaccgct acgagctgcc tggagacccg attcttggtg aaggtaagcc cgagaaccag 3300
   aacaacgcgc ttccattcac gcgtggcgaa tacctccaga cgattgatat gaaccagcag 3360
   cactacttcg aggagtgtct gaagatgccg cagcttctgg tgactgctga cctgcaccct 3420
   tccaagaaac ctgtgtccat tattggtatg cgtgagcaca ttttcacggg taacgcttca 3480
   tcgctgtcaa agtttaagtc gtggcaggag ctggtgttcg tgacgctgtc tcagcgtgtg 3540
   ctggccgacc cgctgtatgt tcgtatgcac tacggtcacc ccgatatttt cgacaagatc 3600
   attgctatgc ctcgtggtgg agtgtccaag gcttccaagg gcattaactt gtctgaggat 3660
   gtgttcgctg gttttaactc gacgcttcgt ggtggtgtgg tgacgcacgt ggagttcatg      3720
   cagtgtggta agggtcgtga tgtggctctg tcgcagattt ccatgttcga gggtaagctg      3780
   gctaacggtg ctggcgagac gtctctcgct cgtgaggctc atcgtatggg ccagttcatg      3840
   gacttcttcc gtctgaactc catgtactac tcgcacacgg gtttctactt cgccacgtgg      3900
   atgacaattg tcacgacctt cgtgtacatg tactgcaagg tgtacttggc tctagcgggt      3960
   gtgcagcagc agattgtgta cgatatgaac acgaccgctg tgatcaccga gaacatcgca      4020
   aacaacttcg acgggcgtgt gttcaccgat ctgaaggctg tgctgaacac gcagttctac      4080
   atccaagccg gtactttcct catgcttccg ctcatgtgtg tgtacttcgg tgaaggaggc      4140
   ttcgtgcgcg gtatgactcg attcatcgac atgatcatca cgctgggccc tgccttcttc      4200
   gtgttccagg tcggtacgac gatgcactac ttcgacaaca acatcgtgca cggtggcgcc      4260
   aagtatcagg ctacaggtcg tggcttcaag atttcccgtg agacgctggt gctgctgtac      4320
   aaggcgtatg caagctccca ctatcgaaag gcctgggagc tcatcgggtt gtgtcttgtg      4380
   tacatggcgt tcggtaattt ttacatctgt cggaccgatg cggctgccaa cgacaacact      4440
   tttgcgtccg actactgtga gactgctcag gcttacgggg tgcagacgtt ctcggtgtgg      4500
   ttcatctcca tcttgtgggt ggtgggtccg ttcctcttca acagtgacgg tctggactac      4560
   aggaaaacca aggtggatat ccagcagtgg tgcatgtgga tgtttgcccc cgaagactac      4620
   aaggacgacg acccggccaa caagggaggc tgggtaggct ggtggaaggg cgatctggag      4680
   cagctgcacg gctccaacat gatctcgcgc gtcacggtca tcctccgcga gtgtcgccac      4740
   ttcctgctca tgttctacgt ggctacactc gagacgtccg acgtcatgta cgtggcgtac      4800
   tcgttcggtg ctgcggtcgc gacgattgtt ctgcttggcg tgttccatgg ctttggtatg      4860
   ggcatgcgct ccatgagccc ggtgacgcgc gctgtgatct acatggggac cgtcgcagcc      4920
   atcgtgacgg cctacttctt ggccacttgg atcgtgctgg actggaaatt caagtacgcc      4980
   atgtcgctct ggttcgccta cgtggctgcg ctctacggta tcaacgagtg cttccgcatg      5040
   tggagtttcc caagctcgtc gatcgctggc attgctgtgt tccagcagct acagttcctc 5100
   ttcgacttta tcttctgcat tggtatgatc atcccgcttg tggtcatgtc gtgcatcccg 5160
   ttcctgaaca tcatccagac gcgtatgatg tacaacgaag gcttctccaa ggtcatgtct 5220
   gcttcttcgc agtatgcctt ctctctggca gccttcatgg gaatcctggg cggtatcggt 5280
   gtcggctggc tgttcaactt gctgtcaacg ctggagcagt ctgcgagttt cgcaagttac 5340
   gtcgtcacgt acgatggcgt cctaagtgga aacgtgggcg atggcaccac gacgtacatg 5400
   ctctacggtg cctgtgtggt gggtacgatc atcgctggct tcctcaactt cttcctgggt 5460
   cgtcgtctgg ctattgttgc gggtggtctc ttcagtacgc tcggtatggt ggctgtcagt 5520
   gccaacgacg atctccgttc gacgctgctg atgcctggta tcggtctgct tggagcctcg 5580
   tgcggtattc tgctaccgtc gttggccatc tacatcttcg agatctcgac caaggagatg 5640
   cgaggcaaag ccatgttgtt gctgggaatc ggcttcatca tcggcagttt gctgggcggc 5700
   attttcgcga ccgtgaacca gctgggatgg atctggcaga cgtttgctgc ctgcatcgtg 5760
   atcgccctgg tcacgccagt cgtcaatgtg ttcccagaga gtccgtactg ggtgctggat 5820
   cgcaagggct gggacgcctg tgaagcctgt ctcgttatcc tgcgtcgtaa acctgatgtc 5880
   caagaggagc tcaaggtcat gcgagaagag gaaacggcag atgaaggcgg cgccggcgcc 5940
   tacaagttcc tcatcggtct cttcctcatg ctcgtgtcct ctctgactac tggtttcctc 6000
   aacgccttca tcagctacaa gggagcgagc gagtacacgg accaagacca gctgttcgtg 6060
   aacgcgatgg cgctgcagat ctccggtgct gcgatcgcca tcttctacat cgacaagctg 6120
   gaccacaagt cgattctctt cggaacgttg atccctatcg ctatctgtgc tggcattctg 6180
   ggcttcaacg agaactcaga gatgctggga gacaaagagg gctcgggtct gtacctcagt 6240
   cttgtagtca tgctcatgta cttcttcatg gggctgggca cgagctctgc tctgtggtcc 6300
   gcatgcgtcg gcatgttcaa tactcgcggc cgcgcctcgt ccaccaccat gctgttcgct 6360
   atcttcttcc tggcggatat gggctacgtc tacttgcaca cggacgactc gatgatgcag 6420
   aacgaatatt cctatctcta cgccatcgca ggcttcagtg tcgtcgccct ggtgctgtta 6480
   atgggcgcag gcaccaagaa gaacggcgtc atctgtacca agtcagaagc tcagcgcgat 6540
   cgcgaccgta tcgctcgtat gcgtgccgaa cgtgcaagtc gcaacacgcg gacgcctgga 6600
   actgcgcgcc agcggaatct cagtcgcgtg cggtccaagt cgaacgctgg cggccagcgg 6660
   ggcaacgcgg cgcatggcgg tggctaccaa atgtacgaaa cacccgccaa cgctgcgccg 6720
   ccgctcatgc acgcgcgccc gtag 6744
<210> 37
   <211> 2462
   <212> DNA
   <213> Phytophthora infestans
<400> 37
   attcgagaga tttttcgaac tctgacatcg ttgcaaccat aacttgcctg cattaaggtg 60
   ctcctcaact gtacccatgc cgagcaccca tcgactctgc cagtacgatg cttcaccaaa 120
   gttttagtcc acaagaaaag ccgaaacgta catgtcgtaa cggaagccgt gcgacctttt 180
   accgttcgtg cgctggtctc acgtagcttc attctccttc ttccctagcc agcaaaggcg 240
   tctcccaaga tatcccagca attgtctatc ggttaagatc aaggagtaaa atgcactcgg 300
   cgctgttctc gtgctcaact ttggctttga tcacgagttt ggtctcctca gagcgtgccg 360
   actcgatgat caaatctcgg tctggattga gcccctgggt ggacgttgac acccccgaga 420
   gtgcactcaa cgtcacgtcg tcgcgtggag atacgtggac gttggtcatg agcgacgaat 480
   ttaacgttcc tgggcgtaat tttacgcccg gttcggatca catgtggacg gcgttagaaa 540
   tgcccgacgg tgtcaatgca gctctcgaat actacagctt caatatgacc gataccgtaa 600
   cggaatcgga cggtcgtggt gtcttccgga taaagattat ggaagaggac aacatcacct 660
   acacggtgtg gaacacgtac gctaagcctg cgggtttcga gacccatcac atgtactacc 720
   gagccggtat ggtgcaatcc tggaacaaat tctgtttcca aggaggaagg atggaagtgg 780
   tggctcaatt accagcaaca accagctcta gtaaccccga tatgggtgac atcaaaggtc 840
   gcgtcaagac gaacagcttc tatcccacgt ggcctggtat ttggcttcta ggaaatctag 900
   gacgagctct attctcccaa tcgaccagtc ggatgtggcc ttggagttac gacgaatgtg 960
   acgaaaaatt cgaatccagt caaagaatca gtgcgtgtga cggtaatcca ggaagtggat 1020
   tgaatgctca tcaaggacga ggagcgccgg aaatagatct actggaagga ggaggtgtag 1080
   ccatctccac aagtatccag gtcgctcctg gaatgcccga taaattccgt atcattgctc 1140
   ccacggatga taaaagtccc ttctgcgtct tcacggccga gtgtactact attggtgcta 1200
   actttcctgg gatcccagcc aaagcgtacg aagctcgaga ttatgaaagc tggtatcaag 1260
   gactacggta cgctccaaat actctttgta gcccagttgg cagcttgatg caagatccca 1320
   aaacggtgct tgcaaatgcc gagaaaggct tcacttctaa tacgtgtaaa ggagtcaatg 1380
   cgtgtccagc ttcgggtgac ggatactccg atttaggctt gatcgacggg aaagggcctg 1440
   attactgggg ggttaacaaa gaaggcggct gtatgccggt tattaacgga tacacgggag 1500
   ctttcctatg tgaccctgac agttccaaca aaaaatgttc ctctccgtta ggtgcggaag 1560
   aacccaagag taaagttatg gaaccattcg agtatcagat ggacgccctg tccgctaatt 1620
   ggccagttca gctcgcagca tatacaagtt atgtgaaata ccaagtcgaa tgggtcatgg 1680
   gctcacaagg ttacattcgc tggatggtcg aggatattgt gatttttgaa attccagccg 1740
   agtcggtcga gaatgttcca caagatgctg ccaagtccaa ccctaagaaa ctcatgttgg 1800
   aagaacccat gtacgtgatt ttcaacgtcg ctctgtcaac tagttggggt accacgccac 1860
   cgaatccagg gtcgccatgt cgtggagatg ggagcaatgc gcaacataac gctatttgtg 1920
   acggtttccc catgttcatg aagatcgact acattcgtat ctatcaggat ctctcttcca 1980
   actccacgat ggctatcggg tgtgacccgt ctactcatcc gactaaacaa tggatcgagg 2040
   atcacattga tgagtatgag acgacggaga ataaatggat tgaggttcac ggtggagcca 2100
   attgcaagac tgataacgac tgtacggtca gtacttctca cattttgacg gggaaatgta 2160
   gcaagaaaca tcgctgtaga tgcggatctt ccggtgcttg gggtggtcca cgttgcacaa 2220
   ctccactagc tgatacagcg aatggagaag gcttcggacc accaacagtt attacgagct 2280
   tggttggtgc attcgtcatt gtgcttttgg tctttgtggt gtacaagata atggaccaac 2340
   gcagcaagaa ggcgatggtg ggagctggga ttactcaacc agcaattctc tccaagatcg 2400
   agatggacga catcccacgt tcaaataaaa gccagagcgg ctcggaggat aaggtggtgt 2460
   ag 2462
<210> 38
   <211> 942
   <212> DNA
   <213> Phytophthora infestans
<400> 38
   atggctccac tgccatccgc aaccttgacc acacccgacg tggctcctgt cacagctacc 60
   accgcctcgc agcccttcga cgacctagtt gacaaggtcg agagtggcta tgcggctatg 120
   cctaccaccc caaaggcggt ggctcccttc ccaacctcca aaccgcaagg tctccagcgt 180
   ttcgccgttc agagcgtgca cctgcgcgag tgcttcgccg agttcctcgg caccttcgtc 240
   atgatcgtct tcggtatggg cgtcaacaac caagtgacca actcacacga cgccaacggc 300
   acgtggctca gcatcaatat gtgctggggg attggtgtac tcatcggcgt ctactgctcc 360
   gagggcatca gtggtgctaa cctcaacaca gccgtgacgt tggcacattg cgtgtacggc 420
   cgtctaccat ggtggaaagc acccggttac atgatctcac agctattggg cgccttctgt 480
   ggagctttca ttatctacgt catgcagtac cagaacctca acgtcattga cccgtatcgc 540
   gagaccacac agagcagctt ctcgacgtat ccgagcgaca acatctccaa ctacacagcc 600
   ttctacaccg agttcatcgg tactgctatg cttgtgctca gtatctacgc catcacggac 660
   aagcgcaaca gatctgccgg tctcgtgggt tctcccttcg ccttctgtct aatgatcatg 720
   gcgttgggca tggcctttgg catgaacacg ggctacgctg tgaaccctgc acgtgacttc 780
   ggcccccgca tcttcacggc catcgcgggc tggggctcca aggtctttac cactcggaac 840
   tactacttct ggatcccgat cgtggccgat tctatgggcg gaattgctgg tgctggactc 900
   tatcggctgc ttgtggagat ccaccaccca cccctctcgt ag 942
<210> 39
   <211> 1636
   <212> DNA
   <213> Phytophthora infestans
<400> 39
   atgtccgacg accaagtgcc accgttttcg ctcggtacga aacaccttac acgcatgacg 60
   tgcagcgcac agttcagaga tgactaactg gccatgctgc cgctacgtgt atttaccaga 120
   taagccgcgc cacgatactt cgacgtacgt cggtcggtgg cgtaagtttg cggagctcgt 180
   gtcgccaaag tacgtcaaac aaggtagacg atggaaaaag tgtggctcta aacatcatgc 240
   tggggcttta ggtggctctt tctaagctcg gagcagattc agcacgcgac gcagactctg 300
   gaggacttcc gcaacggtaa aatcgcccct ggtcagttca aggatgcgga actttggaat 360
   cttcgccagg gtacgaaaca atgcgacagg atggtaccaa cttgtggttt ttgctgacgt 420
   tttggtgata attgaatcga tatgtagcgt acgaggctgc cgtgcaccct cagactggcg 480
   agacagttcc ggctgtgttt cggctgtcgg ctttcgtgcc tgtgaacatt cccatttgcg 540
   ttggcatgct gctcgctcct gccacggtat gtaaagtctt gctgcacttg taacaatagc 600
   tttattttaa tttaaatgct cacttaaata tgcttacagt tgggaaacac gatcttctgg 660
   caatgggtta accagagcta cagcgccggt ttcaactacg ctaaccgcaa cgccagcagt 720
   gaacaggaca actccaccat cttcagtacg aagttctcga tgctaatggc cttctttcag 780
   cgttctaaca cggctctttg catcgtatca cagagtcgta cgctaccgcg acgctcgtgt 840
   cttgctcgac tgccgttggt ctgggtaaga tggtggagaa agccaagaga ctttctccca 900
   gcacgcgttc cttcctcgga aaggttaaaa ctgctaaatg ccgaatgctg tgctccttat 960
   gtgactgaat ttttgggctg caacttgatc agatggttcc tttcgttgct gtcgcgagcg 1020
   caggtgcttt caacgccgtg tcgatgcgct tcaacgagtt ccagtacgtg tagagtgtct 1080
   tgcactggaa atatgatgct gttgtgctca ttggagtgtg gtgatgcgtt acagagaggg 1140
   aattgatatc atggacgagc acggagatgt tcacgggcgt tcagttgccg ctggacgtca 1200
   gtcgcttggc caagtggcac tcacgcgtat tgcgctaccc atgcccagta cgtcctcgca 1260
   ttgacaagct cttcttgggt gaattatgtc taatattggt ggacttggac agttttgctg 1320
   ctcccgccgt acctgtatga gatcatgaag aaaaccaaca tcatgcccaa ggccaagtac 1380
   ccgaagcttg ctgcggagct cggtacgtgc attatttctg ccatagtccg cggtaatgag 1440
   ctaacttatt gcgtctctgc ctgcatctat gatagtcgtg ttaacgatgt gtctgtgggg 1500
   tgccatgccc agcgctgtgg cgctcttccc gcagttggga acgatttcgg ctgacagcgt 1560
   tgaggaggaa ttccgatctc gggtggatcg caacggccag cccattcgtc acttcatcta 1620
   caacaaggga atctga 1636
<210> 40
   <211> 960
   <212> DNA
   <213> Phytophthora infestans
<400> 40
   atgagcaccg ccaataaaga agcatctccc tttgtggagc ttcgccgtct taactttgcg 60
   ttcgccaaag ttactatcgg tggccaactc atcaatcgct tgcagcatga ccccgctact 120
   gctgacgcca tggaagacga cgatcgagtc ctgcgtgacg tgaatctaac gctgcagtct 180
   ggtcagcgcc tgttggtcgt gggtggtaat ggagctggca aaagcacgct actcagtatc 240
   ttggctggca agcacctgac ggctgacgac acggcgctga tcttcggtcg agacagcttc 300
   cgtgacacga cactcaacgc tttaaggact ttcgttagtg ccgactgggg gcagcgctcg 360
   gtggcgttcg caacgcacgc aatggcttac tcggctgaca tggcggtgga agaaatgatg 420
   acgaaactgc agagtgaaca cccggagcga cgccaaaagt tactgaaggt gttgcgtatt 480
   gacccaaagt ggcgtttaca tcgcctgtcg gacggtcaac gtcgccgcgt tcagctcttt 540
   ctggcgctac tgagaccttc gcagctcatc gtactggacg aagtgctggg aatgctcgac 600
   atcatctcgc gcgaaaacgt actggcattc ctaaaggagg aaacagagac tcgacaagcc 660
   acggtgttgc tagccacgca catcttcgat gggacggacg tctgggcatc tcatgtgctg 720
   tatattcgtc gcggtactgt tgggttctat ggtccgattc agcagtgtac tgatggaggg 780
   aagatcccga tgtataaggt ggtggaacac tggctacgtg aggaattggc cgacgacgac 840
   cgtgtggaat gcgaggcggt gggcccaagt ggtgagttcg acctgggcaa cgcgcagaac 900
   cgcgcaggag ggtatgccga cggtcgactg ggtggtgtcg atgtcaccac atcgttttaa 960
<210> 41
   <211> 510
   <212> DNA
   <213> Phytophthora infestans
<400> 41
   atgagcgacg gtactaagct cattgccgtg atcggagacg aagacaccgt cacgggcttt 60
   atcctcgcgg gtgtcggcca tcgaacggcc gagggaacca actttctcgt cgtcaaaccc 120
   tgtgcgtcgg cattcccgaa aggatttttt taaagcttag attaactact aacacgcgtc 180
   acatacgacg gtgctgcagc tactccaatc tccgctattg aggccagttt ccggacgctc 240
   tcgagccgtg acgatatcgc aattatcctc atcaaccagc acgtacggca tcctagttag 300
   aacccactct gaggtataag cctttatgtt tttctcactc gcttgatatt attgtttggg 360
   caggttgctg aggagatccg tcaccttctc aacacgtacg acaagaccat tcccacggtg 420
   ctagagatcc cgagcaaaga ctcgccttac gacccagcta aggactatat catgaagcgc 480
   gtgaatctca tgcttggagg agagtcgtga 510
<210> 42
   <211> 1491
   <212> DNA
   <213> Phytophthora infestans
<400> 42
   atggtcgctg aagacgcggt gttcacggaa gcgaagacgc ccaaggccgg cgatgtccag      60
   gccggcagat ccgtcccgct ctcgccgacc ttctggttca gcctcgcggt gctgctcctg     120
   ctccccttcc agttcggctg gtcggtcggc cagctcaacc tcacgacctt caacgacgaa     180
   gacgagtgca acgcgcgacc tctagtcgac ggaacgtgcc tcatgtttcc cggtcatagc     240
   agcacggaat ggaccttgat cgtgaacgcg tggattgtag gcggtatgat cgggagtctc     300
   ggttgtggcg ttatctccga acgtttcggt cgcaagaagg tattgctggc gaacgccgtc     360
   gtcatgttgg ccggcgctgt cattcaagcg agtacgtcga gtatctcggt ttttatggtc     420
   gggcgtatcg tcgccggtat cgcgtcgggt tgtgcgacgg gtatggtggg tggctacatc     480
   agtgagatta cacccccgag tctccgtaac tcgtacggca cgttcatgca ggtatctctc     540
   tctgccggta tcttagtagt gaccatcagt tttttcttcg cggataccag tagcggctgg     600
   aggtacatcg ccgcatttcc cgttcttaac gccggtttct tcttggcgtt cgcaccgttc     660
   gttcttgtag agagtcccgc gtggcttttg gaaaagggcg accgggagca tgcggagcgg     720
   gagatcgcca gactttacgg cttcgatttc gtcccagtag cactgacgtg gatggaacca     780
   ggtatcaata ctgacctgga gtcagaggaa ctgtgcggcg aacagcacga aggcggcaca     840
   ttgtcgctgc tgttttcgcc gctctttatc aagcaactgc ttgtggctct tggtgtatca     900
   gctgcgcaac aacttacggg tatcaacgcg gtgtgctatt actcgtccga tatcttctcg     960
   gatgcaggga tgtcggatgg tcgtgtgggt ggcgtcatcg tgtacgtgct gatgttacta    1020
   ccgacgatgg ctgttgcgcg attgtcggag cgattcggca accgacggct tctgcttact    1080
   ggactggccg ggatgtttat tagtgctacg ggtataactt tggcgcttgc gttgtcggtc    1140
   gaggtactct ctatcgtctt catgggtaca ttcgttgcgt tcttctctgc gagcgtgggg 1200
   ccgcttatct agcctatcac ggccgcgctg ttcacggact cggtacgcgc cactgccgtc 1260
   tctatgtgca tctttatcaa ttgggtatgc aacttgatca ttggcgtctg cttcccgtac 1320
   gtctcggatg cgctagacga gtacaagttt gtccccttta tggtgaccac cgctgcgttt 1380
   ttcttcttca ctcagttttg gatcccggag actgcaggta agagcaccga ggagatccaa 1440
   gccacgttcc gatctaggaa agctcagaag ccggtggtgg tgttaagctg a 1491
<210> 43
   <211> 2462
   <212> DNA
   <213> Phytophthora infestans
<400> 43
   ttgtgctact cgagtgcctt cttctccatg acgcagcaag acgttcgcca ggcgctggac 60
   gcccccgcat tagcagccta catccactcg cgactgggtg catcgcacgg cgccatcgcg 120
   tccatccgtc agttccagca tggccagtcc aacccgacgt acctcttgac aatggccggc 180
   tcgaaagcca agtgggtgtt gcgtaagaag ccgcacggtc agatcctgcc gtcagctcac 240
   gccgtagagc gcgaatatcg tgttctagag gctctggagc actcggaggt gcccgttccc 300
   cgcgctgtgt tgctatgtga agaccccaaa gtcattggca cacctttcta cctcatggaa 360
   tacatacatg gtcgtatctt ccaagaccca tcgctgcctg gcattaaacc tatgtatcgc 420
   tacgccatgt acagctcggc agtagacgcg ttggttaagt tgcacgagct ggactacaag 480
   aagatcggac tggccgactt cggtcgcccc gagaagtact gccaccgtgt cgtgacgcgc 540
   tggagtcgac aagtccaaag tggccagaag gtctttagtg aagctggagt gaaggagaac 600
   ccgaagatga cgcagctgca acgttggctg gagcagaacg ccgacgacgc cgagaaggcc 660
   acgaccagtg ccgagggagc gagtatcgtc cacggagact tccgcattga caacatgatc 720
   ttccacccga cggagcctcg tgtgttggcc attctggact gggagctgtg tactatcggc 780
   aatccgttct cggatgtagc cacattggcg tcggcctaca gactgccact ggacaactcg     840
   aacacaatca tgacgcctgg tctctcggat gctccgttga agacactcgg catcccatcg     900
   gagagtgaat tgttgctagg ctactgtcgt cgtgccagac ggttccctct acccactcag     960
   acgtggcatt ttttcatggg gatgatcgta taccgcttcg cagccatctg ccacggtgta    1020
   tacgcccgcg cactgctcgg caacgcgtcg tcggccaacg cagcatgtgc caagacgacg    1080
   atggaccgac tcttggccat gagcgacgac atcatggacg catcggctga tatttacccg    1140
   gagccggagc tgacgcacat tctgccgttc ccgatccgtc ctcatgctct gcagatgtac    1200
   aagaagctgc taaagttttg ccagaaccga gtgtaccccg ctgagtccgt ccacattgct    1260
   cagatcgcca aggcgagaga agaaggacgg gagtggcaga gtgtgcctcc tgttattgaa    1320
   gagctcaaga cggaagccaa ggcgctggga ctctggaacc tcttcctgcc tcagtgtgtg    1380
   gtgccagctc tggatggcaa tggaccagac gtcaactacg gtggagacct gacgaatctc    1440
   gagtacggac tcatgtgtga ggtcatggga cgctccattg tgttggctcc ggaggttttc    1500
   aactgctcag cacccgatac cggcaacatg gagatcttga cgcggttctg cactgtggag    1560
   cagaagcatc agtggcttgt tcctcttctt caaggtgaga tccgatcgtg tttcgcgatg    1620
   acggagaagc gtgtggcttc gtcggatgcg acgaacatcg agacgcgcat tgtacgcgac    1680
   gaacagcgtc aagaatacgt catcaatggc cacaagttct acatctctgg agctggagac    1740
   ccacgatgca agatcattgt gctcatgggc aagcacacgg aacgagccaa ggagagtcca    1800
   ttcaagcagc agtcgatgat cctcgtgcct atggacacac ccggcgtgca ggtcgtgaag    1860
   cccatgcatg tctttggcta cgacgacgca ccgcacggac acatggagat gctgttcaag    1920
   gacgtgcgtg tgccgttcag caacgtgttg ctcggtgaag gtcgcggctt tgagatcgct    1980
   caggctcgtc ttggacctgg ccgtatccac cactgtatgc gagccatcgg agctgctgag    2040
   cgttgtcttg agctgatggt gcagcgagcc aagacacgca cagcgttcaa gcagctcctg    2100
   gccgagaatc cgctcgtgtg ttcgcagatt gccaagtcgc gttgtgaatt ggacagcgcg    2160
   cgcttgttga cgctccaggc tgcacatcag atggacaagc acggcaacaa ggtggcgcaa    2220
   caggccatcg ctatgatcaa gatcgtggcg cctaacatgg cactggacgt ctgcgaccga    2280
   gccatccaga tccacggcgc ttctggcgtg agtcaggact ttgtcctgtc ttacttgtac    2340
   gccgccctgc gcacgctacg tatcgccgac ggaccggatg aagtgcacat gcggaccatc    2400
   gcaaaactcg agctgagcca ctcgaagttg taagagaaat gtaaggcaaa agcaagcgtg    2460
   aa                                                                    2462

## Beschreibung

Die vorliegende Erfindung betrifft eine transgene Pflanze der Art *Solanum tuberosum* mit einer Resistenz gegenüber einem Oomyzet der Gattung *Phytophthora,* transgene Teile einer solchen Pflanze, ein Verfahren zu deren Herstellung sowie ein Mittel zur äußeren Applikation bei Pflanzen.

Die durch *Phytophthora infestans* hervorgerufene Kraut- und Knollenfäule der Kartoffel ist auch heute noch die wichtigste und wirtschaftlich bedeutendste Kartoffelkrankheit.

Weltweit führt das Pathogen zu Ertragsausfällen mit Ernteeinbußen von über 20 Prozent. Dies macht einen aufwendigen Einsatz chemischer Pflanzenschutzmittel notwendig, weil die natürlichen Abwehrmaßnahmen der Kartoffel, mit deren Hilfe *P. infestans* abgewehrt oder die Ausbreitung verzögert und einschränkt werden kann, nicht ausreichend oder nicht von Dauer sind.

Es ist bekannt, dass natürliche pflanzliche Abwehrmechanismen wie die hypersensitive Reaktion an der Infektionsstelle, die Lignifizierung der Zellwand, die Produktion von PR(pathogenesis-related)-Proteinen und die Synthese von Phytoalexinen zwar zur Resistenzsteigerung beitragen, aber stets einen Energie- und somit einen Ertragsverlust der befallenen Pflanze mit sich bringen.
Zu den in Pflanzen vorkommenden natürlichen Abwehrmechanismen zählt auch die Expression von sogenannten Resistenzgenen (R-Gene), deren Genprodukte mit mikrobiellen Avirulenz-Genen (Avr-Gene) interagieren (Gen-für-Gen-Hypothese) und damit eine spezifische Abwehrreaktion induzieren. Allerdings kann diese Resistenz gebrochen werden, wenn ein Erreger wie *P. infestans* auf die Synthese des Avr-Gen verzichten kann und die Erkennung des Pathogens und folglich die spezifische Abwehrreaktion im pflanzlichen Wirt ausbleibt.

Bereits Fire et al. (1998) konnten zeigen, dass doppelsträngige RNA (dsRNA) zum Sequenz-spezifischen Abbau homologer RNA führen kann. Ausgehend von diesen Ergebnissen wurden mittlerweile transgene Pflanzen entwickelt, die mit Hilfe von RNA-Interferenz (RNAi) über Wirtspflanzen-vermitteltes Silencing konservierter und essentieller Gene beispielsweise aus Nematoden oder *Lepidoptera-* und *Coleroptera*-Arten sowohl *in vitro* als auch *in vivo* Resistenz gegen diese Schädlinge zeigen.
Auch auf die Interaktion Wirtspflanze - phytopathogener Pilz läßt sich das Konzept des Wirt-vermittelten Gensilencing (host induced gene silencing: HIGS) zur Resistenzvermittlung anwenden (EP 1 716 238).

Van West et al. (1999) setzten die Methode des Gen-Silencing zunächst in Phytophthora ein, um funktionale Analysen zu diesen Oomyzeten-spezifischen Genen durchzuführen.

In WO 2006/070227 wird erstmals die Verwendung von RNA-Interferenz zur Kontrolle von pilzlichen Pathogenen basierend auf dem Kontakt von dsRNA mit Pilzzellen außerhalb der Pilzzelle beschrieben. Es wird dort ein Verfahren zur Herstellung einer pathogenresistenten Pflanze vorgestellt. Dabei kann die RNA-Interferenz gegen ein oder mehrere Gene eines Pathogens sowie gegen mehrere Pathogene gerichtet sein. *P. infestans* wird als ein mögliches pilzliches Pathogen und Kartoffel als mögliche Wirtspflanze aufgeführt.

Bisherige Arbeiten geben Anlass zur Annahme, dass Wirtspflanzen-vermitteltes Gensilencing nicht für jedes Gen funktioniert und die Auswahl der Zielgene essentiell für ein funktionales Silencing ist. So konnte beispielsweise die Plasmamembran-H+-ATPase PnMA1 in *Phytophthora parasitica* nicht ausreichend über Wirtspflanzen-vermitteltes Gensilencing reduziert werden, um einen effizienten Schutz gegenüber dem Pathogen zu vermitteln (Zhang et al. 2011). Demnach ist die Selektion der Zielgene auch für eine effektive Pathogenabwehr entscheidend (Yin et al. 2011).

Kürzlich wurde ein Screening-System vorgestellt, das die Auswahl geeigneter parasitärer Gene für Silencing-Konstrukte zur Herstellung von pathogenresistenten Pflanzen erleichtern soll (US 2010/0257634). Auch die Identifikation von geeigneten Testkonstrukten zur Vermittlung von Phytophthoraresistenz in Kartoffel wird von den Autoren ausgeführt. Dabei werden Zielgene basierend auf bioinformatorischen Analysen von Genomsequenzen oder basierend auf Sequenzhomologien zu essentiellen Genen oder Virulenzfaktoren aus bekannten Modellorganismen definiert. Ein Hinweis auf die mit der vorliegenden Erfindung offenbarten Gene zur Ausbildung einer Resistenz gegenüber einem Oomyzeten der Gattung *Phytophthora* enthält diese Druckschrift nicht.

Ein Verfahren zur Erzeugung einer Breitband-Resistenz in transgenen Pflanzen gegenüber multiplen Pilzen wird in WO 2009/112270 beschrieben. In einer Ausführungsform des erfindungsgemäßen Verfahrens ist die Breitband-Resistenz gegen *Uncinula necator, Plasmopora viticola*, *Uromyces spec.*, *Phakopsora pachyrhizi*, *Erysiphe sp.* als auch *P*. *infestans* gerichtet.

Darüber hinaus wird die Entwicklung von *P. infestans* resistenten Kartoffelpflanzen durch RNAi-vermitteltes Silencing in WO 2006/047495 dargelegt. Zum einen wurden Pflanzen generiert, die für die RNA Interferenz Gensequenzen der rRNA-Gene aus *P. infestans* tragen. Das in WO 2006/047495 beschriebene gegen die rRNA-Gene von *P. infestans* gerichtete Silencing-Konstrukt umfasst die Basenpaare 1-600 der Accession Nummer AJ854293 und damit 32 bp des kodierenden Bereichs der 18S rRNA sowie den vollständigen kodierenden Bereich der 5.8 S rRNA-Gens des Krautfäuleerregers. Bei der Auswahl von Zielgenen für HIGS-Ansätze ist mit Blick auf die Anwendbarkeit entscheidend, dass es möglichst kurze vorzugsweise keine über 17 aufeinanderfolgende Basenpaare hinausgehenden Homologien zu Gensequenzen von Nichtzielorganismen gibt, da andernfalls die Genexpression der Nichtzielorganismen im Fall des Konsums der transgenen Pflanzen oder ihrer Ernteprodukte gestört werden kann ("off-target" Effekt). Die in WO 2006/047495 beschriebene Sequenz umfasst jedoch 32 bp der *P. infestans* 18S rRNA, die zu 100% identisch mit dem homologen Sequenzabschnitt der 18S rRNA-Gen aus Mensch (*Homo sapiens*), dem Schwein (*Sus scrofa*) und dem Rind (*Bos taurus)* ist. Der menschliche Kartoffelverzehr betrug 2005 in Asien 26 kg, in Nordamerika 58 kg und in Europa 96 kg pro Person (FAOSTAT). Vor dem Hintergrund hoher von Mensch und Tier konsumierten Kartoffelmengen sind die in WO 2006/047495 beschriebenen rRNA Sequenzen von *P. infestans* als HIGS-Zielgene aus Sicherheitsgründen für den Konsumenten ungeeignet.
Zum anderen wurden in WO 2006/047495 Pflanzen erstellt, die für die RNA-Interferenz in einem Doppelkonstrukt Gensequenzen des Kathepsin B-Gen aus *Myzus persicae* und des Elicitin-Gen INF1 aus *P. infestans* tragen und somit Resistenzen gegenüber zwei pflanzlichen Pathogenen aufweisen. Das dort verwendete Zielgen INF1 kodiert für einen Elicitor. Eine auf einem Elicitor als Pathogenitätsfaktor basierende Resistenz ist insofern nachteilig, als dass das Elicitin-Gen INF1 nicht immer für eine Infektion von Kartoffeln durch *P. infestans* notwendig ist (Kamoun et al. 1998).

Aufgabe der vorliegenden Erfindung ist es daher, eine transgene Pflanze der Art *Solanum tuberosum* bereitzustellen, die pathogenresistent gegenüber einem Oomyzeten der Gattung *Phytophthora* und die insbesondere hinsichtlich eines Konsums geeignet ist.

Erfindungsgemäß erfolgt die Lösung der gestellten Aufgabe dadurch, dass in die transgene Pflanze eine doppelsträngige erste und zweite DNA stabil integriert sind, wobei die erste DNA (a) eine Nukleotidsequenz gemäß den SEQ ID NOS: 1, oder (b) ein Fragment von mindestens 25 aufeinanderfolgenden Nukleotiden einer Nukleotidsequenz gemäß den SEQ ID NOS: 1, oder (c) eine Nukleotidsequenz, die komplementär zu einer der Nukleotidsequenzen nach (a) oder (b) ist, oder (d) eine Nukleotidsequenz, die mit einer der Nukleotidsequenzen nach (a), (b) oder (c) unter stringenten Bedingungen hybridisiert, umfasst.

Überraschend wurde gefunden, dass eine solche erste DNA in besonderer Weise geeignet ist, um über einen Wirt-vermittelten Gensilencing-Ansatz eine Pathogenresistenz in Kartoffelpflanzen auszubilden.

Dabei sind die erste und die zweite DNA stabil in das Genom einer transgene Pflanze der Art *Solanum tuberosum* integriert. Vorzugsweise sind die DNAs stabil in ein Chromosom der Pflanze integriert. Sie können aber auch in ein extra-chromosomales Element integriert vorliegen. Der Vorteil einer stabilen Integration besteht darin, dass die DNA an nachfolgende Generationen der transgenen Pflanze weitergegeben werden können.

Die doppelsträngige DNA setzt sich aus einem kodierenden und einem nicht-kodierenden Strang zusammen.

Darüber hinaus ist die Nukleotidsequenz des kodierenden Strangs der zweiten DNA zu der Nukleotidsequenz des kodierenden Strangs der ersten DNA ganz oder teilweise invers komplementär. Unter invers komplementär wird bezogen auf eine Nukleotidsequenz in 5'-3'-Richtung eine Nukleotidsequenz in 3'-5'-Richtung verstanden, dass die Basen entsprechend den Basenpaarungsregeln mit den Basen der ersten DNA korrespondieren und in umgekehrter/gespiegelter Reihenfolge aufgeführt sind. Ist die Nukleotidsequenz des kodierenden Strangs der ersten DNA beispielsweise *atggttc,* so lautet die dazu invers komplementäre Nukleotidsequenz des kodierenden Stranges der zweiten DNA *gaaccat.* Es wird auch von sense und entsprechender antisense (invers komplementärer) Orientierung der Nukleotidsequenzen gesprochen.
Insbesondere kann die Nukleotidsequenz des kodierenden Stranges der zweiten DNA zu der Nukleotidsequenz des kodierenden Stranges der ersten DNA über die gesamte Sequenzlänge invers komplementär sein. Sie kann aber auch nur teilweise invers komplementär sein, d. h., invers komplementär über eine begrenzte Länge. Die Nukleotidsequenz des kodierenden Stranges der zweiten DNA kann auch in mehr als einem Bereich, beispielsweise in zwei oder drei Bereichen ihrer Nukleotidsequenz zu der Nukleotidsequenz des kodierenden Stranges der ersten DNA invers komplementär sein. Ausgehend von den zueinander ganz oder teilweise invers komplementären Nukleotidsequenzen des kodierenden Stranges der ersten und zweiten DNA wird eine doppelsträngige RNA gebildet. Die doppelsträngige Struktur der RNA entsteht durch die Ausbildung von Wasserstoffbrückenbindungen zwischen komplementären Nukleotiden. Es können doppelsträngige RNA-Bereiche über einen einzelnen Nukleinsäurestrang, der zu sich selbst teilweise komplementär ist, oder über zwei verschiedene, diskontinuierlich komplementäre Nukleinsäurestränge gebildet werden. Die Wasserstoffbrückenbildung kann folglich intramolekular als auch intermolekular erfolgen.

Gemäß der Erfindung umfasst die erste DNA eine Nukleotidsequenz gemäß den SEQ ID NO: 1, wobei es sich bei dieser Sequenz um eine Nukleotidsequenz eines ausgewählten Zielgens aus *P. infestans* handelt. Dieses Zielgen umfasst ein essentielles Gen der Aminosäurebiosynthese, insbesondere der Biosynthese der aliphatischen Aminosäuren (Valin, Leucin, Isoleucin) sowie der Glutamatbiosynthese. Beschrieben werden weiterhin die SEQ ID NOs 2 - 43 umfassend Gene der zellulären Regulation und der Signaltransduktion wie der Redoxregulation, des Calciumsignallings, des G-Proteinsignallings, des MAP-Kinasesignallings und Transkriptionsfaktoren, aber auch Gene der Translationskomponenten, Gene mit Funktion in der RNA-Prozessierung, Gene, die für Entwicklungs- und Differenzierungsproteine kodieren wie zum Beispiel mit Funktionen im Zellwandaufbau, sowie Gene, die für Transporter, Kanäle und Membranproteine kodieren. Eine Zusammenstellung dieser Zielgene aus *P. infestans,* die für das Konzept des Wirt-vermittelten Gensilencing genutzt werden, ist in Tabelle 1 aufgeführt.

**Tabelle 1.**

| **ID** | **Zielgen_ID** | **Funktion** | **Kategorie** | **Identifizierung** |
|---|---|---|---|---|
| 1 | PITG_03410 | Acetolactatesynthase | Aminosäurebiosynthese | A |
| 2 | PITG_00375 | Haustorium-spezifisches Membranprotein (Pihmp1) | Entwicklung/Differenzierung | D |
| 3 | PITG_13490 | Urokanase | Glutamatbiosynthese | C |
| 4 | PITG_00146 | Glucose-6-P-Dehydrogenase | Primärmetabolismus | C |
| 5 | PITG_00561 | Ubiquinon-Biosynthese Protein COQ9 | Primärmetabolismus | B |
| 6 | PITG_06732 | Acyl-CoA-Dehyd rogenase | Primärmetabolismus | B |
| 7 | PITG_07405 | Pyruvatkinase | Primärmetabolismus | B |
| 8 | PITG_12228 | NADH-Cytochrom B5 Reduktase | Primärmetabolismus | B |
| 9 | PITG_15476 | Malatdehydrogenase | Primärmetabolismus | B |
| 10 | PITG_18076 | Phosphoglyceratmutase | Primärmetabolismus | B |
| 11 | PITG_19736 | Alkoholdehydrogenase | Primärmetabolismus | B |
| 12 | PITG_20129/ | Acyl-CoA-Dehydrogenase | Primärmetabolismus | B |
| 13 | PITG_00221 | Tryptophansynthase | Aminosäurebiosynthese | A |
| 14 | PITG_05318 | N-(5'-phosphoribosyl)anthranilat-isomerase | Aminosäurebiosynthese | C |
| 15 | PITG_13139 | Threoninsynthase | Aminosäurebiosynthese | C |
| 16 | PITG_00578 | Imidazolonpropionase | Glutamatbiosynthese | C |
| 17 | PITG_15100 | Histidinammoniumlyase | Glutamatbiosynthese | A |
| 18 | PITG_11044 | Proteinphosphatase | Signaltransduktion | B |
| 19 | PITG_21987 | Proteinphosphatase 2C | Signaltransduktion | B |
| 20 | PITG_01957 | Calcineurin-like Katalytische Untereinheit A | Calciumsignalling | C |
| 21 | PITG_02011 | Calcineurin-Untereinheit B | Calciumsignalling | C |
| 22 | PITG_16326 | Calcineurin-like Katalytische Untereinheit A | Calciumsignalling | C |
| 23 | PITG_00708 | Thioredoxin | Redoxregulation | C |
| 24 | PITG_00715 | Thioredoxin | Redoxregulation | C |
| 25 | PITG_00716 | Thioredoxin | Redoxregulation | C |
| 26 | PITG_09348 | Glutaredoxin | Redoxregulation | C |
| 27 | PITG_08393 | PsGPR11 G-Protein gekoppelter Rezeptor | G-Proteinsignalling | D |
| 28 | PITG_10447 | SAPK-Homolog | MAP Kinasesignalling | D |
| 29 | PITG_06748 | Myb-like DNA-Bindeprotein | Transkriptionsfaktor | A |
| 30 | PITG_19177 | C2H2-Transkriptionsfaktor (PsCZF1-Homolog) | Transkriptionsfaktor | D |
| 31 | PITG_06873 | Aspartyl-tRNA-Synthetase | Translation | B |
| 32 | PITG_09442 | 40S Ribosomales Protein S21 | Translation | B |
| 33 | PITG_16015 | Ribonuklease | RNA-Prozessierung | B |
| 34 | PITG_09306 | PnMas2-Homolog | Entwicklung/Differenzierung | D |
| 35 | PITG_03335 | Kallosesynthase (Fks1/2-Homolog) | Zellwandaufbau | D |
| 36 | PITG_05079 | Glycosyltransferase (Fks1/2-Homolog) | Zellwandaufbau | D |
| 37 | PITG_18356 | Beta-Glucansynthese-assoziiertes Protein (KRE6-Homolog) | Zellwand aufbau | D |
| 38 | PITG_09193 | Aquaporin | Kanal | B |
| 39 | PITG_00562 | Mitochondrialer Tricarboxylatcarrier | Transporter | B |
| 40 | PITG_08314 | ABC Superfamilie-Protein | Transporter | B |
| 41 | PITG_12289 | ATPase H- oder Na-translokalisierender F-Typ | Transporter | B |
| 42 | PITG_12999 | MFS Superfamilie-Transporter | Transporter | B |
| 43 | PITG_16478 | Acyl-CoA-Dehydroqenase | Primärmetabolismus | B |

Der Begriff "Gensilencing" oder Silencing beschreibt Prozesse zur Stilllegung von Genen. Silencing kann z. B. auf transkriptioneller Ebene oder post-transkriptioneller Ebene ansetzen. Das Gensilencing umfasst aber auch die antisense-Technologie, RNAi, oder dsRNA.

Die Expression einer Nukleotidsequenz eines Zielgens in *P. infestans* wird durch Gensilencing selektiv inhibiert. Eine Zielnukleotidsequenz kann dabei auch ein unprozessiertes RNA-Molekül, eine mRNA oder eine ribosomale RNA-Sequenz sein.

Die Zielgene wurden über (i) öffentlich verfügbare Expressionsstudien wie beispielsweise Microarray-Daten zu Oomyzeten-Differenzierungs- oder Infektionsprozessen und öffentlich verfügbare Daten zur Untersuchung metabolischer Prozesse während der Oomyzeten-Differenzierung oder Infektion (Grenville-Briggs et al. 2005, Judelson et al. 2009a, Judelson et al. 2009b) (A), (ii) bioinformatorische Vergleichsstudien gekoppelt mit Pedantanalysen (BioMax Bioinformatic Framework) (B), (iii) Analysen von Stoffwechselwegen gekoppelt mit Pedantanalysen (C) sowie (iv) Auswertung öffentlich verfügbarer Daten zur Charakterisierung von homologen Genen in eukaryotischen Organismen (Roemer et al. 1994, Inoue et al. 1995, Mazur et al. 1995, Lesage et al. 2004, Avrova et al. 2008, Wang et al. 2009, Li et al. 2010, Wang et al. 2010) (D) identifiziert.

Bei der Auswahl der Zielgene wurde berücksichtigt, dass die Nukleotidsequenz dieser Gene spezifisch für P. *infestans* ist, um ein unerwünschtes Silencing pflanzlicher und menschlicher Gene auszuschließen. Dazu wurden die ausgewählten Zielgene auf Proteinebene (BlastX) mit dem Proteom von *Solanum tuberosum* und *Solanum lycopersicum* verglichen. Ergänzend wurden die Zielgensequenzen auf Nukleotidebene (BlastN) mit dem Genom von *Solanum tuberosum*, *Solanum lycopersicum* und einem allgemeinen BlastN (Kriterien: BlastN; Database: Human genomic + transcript; Optimize for: Somewhat similar sequences (blastn)) verglichen. Zielgene gelten als sehr geeignet, wenn sie keine Homologien auf Nukleotidebene zu *Solanum tuberosum* und *Solanum lycopersicum* und keine oder nur partielle Homologien im allgemeinen BlastN in nur kurzen Sequenzbereichen (<17 nts) aufweisen, so dass eine Interaktion mit endogenen Pflanzennukleotidsequenzen verhindert wird bzw. nicht erfolgt.

Gemäß der Erfindung können die verwendeten Nukleotidsequenzen unterschiedlich lang sein. So können die Nukleotidsequenzen gemäß der SEQ ID NO: 1 beispielsweise eine Länge zwischen 501 und 735 Nukleotiden aufweisen.

Bei den verwendeten Nukleotidsequenzen kann es sich aber auch um ein oder mehrere Fragmente einer Nukleotidsequenzen gemäß der SEQ ID NO: 1 handeln. Die Fragmente sollten dabei mindestens 25, 30, 40, 50, 60, 70, 80, 90, 100, 150, 200, 250, 300, 350, 400, 450, 500, 550, 600, 650, 700, 750, 800, 850, 900, 950, 1000, 1100, 1200 oder 1300, 1400, 1500, 1600, 1700, 1800, 1900, 2000, 2100, 2200, 2300, 2400 oder 2500 aufeinanderfolgende Nukleotide einer Nukleotidsequenz gemäß der SEQ ID NOS: 1 umfassen. Bei einem besonders geeigneten Fragment handelt es sich um ein Fragment der Nukleotidsequenz gemäß der SEQ ID NO: 1 mit 290 Nukleotiden.

In einer bevorzugten Ausführung der Erfindung werden Kombinationen von zwei, drei, vier, fünf, sechs, sieben, acht, neun, zehn oder mehr Fragmenten derselben Nukleotidsequenz gemäß SEQ ID NO: 1 verwendet. Weiterhin beschrieben wird eine Kombination umfassend Fragmente der Nukleotidsequenzen gemäß den SEQ ID NOS: 4, 23, 27 und 28, deren Gene die Signaltransduktion betreffen. Ebenfalls beschrieben wird eine Kombination umfassend Fragmente von Nukleotidsequenzen gemäß den SEQ ID NOS: 3, 16 und 17, wobei es sich um Gene der Glutamatbiosynthese aus *P. infestans* handelt. Ebenfalls beschrieben werden weitere Kombinationen umfassend Nukleotidsequenzen oder Fragmente von Nukleotidsequenzen von Genen des Zellwandaufbaus (SEQ ID NOS: 25, 36, 37), des Calciumsignalling (SEQ ID NOS: 20, 21, 22), von Genen des Primärmetabolismus (SEQ ID NOS: 5, 6, 7), von Genen der Redoxregulation (SEQ ID NOS: 24, 25, 26), oder solche, die mehrere Nukleotidsequenzen oder Fragmente von Nukleotidsequenzen von Transporter-Gene gemäß SEQ ID NOS: 39, 40, 41, 42 aufweisen. Weiterhin beschrieben werden Kombinationen umfassend Nukleotidsequenzen oder Fragmente von Nukleotidsequenzen unterschiedlicher Zielgengruppen wie beispielsweise Gene des G-Proteinsignalling, des MAP-Kinasesignalling, des Primärmetabolismus und der Redoxregulation (SEQ ID NOS: 27, 28, 4, 23).
Durch die Kombination mehrerer Zielgene wird die Wahrscheinlichkeit vermindert, dass die Resistenz der transgenen Pflanze durch eine natürlich vorkommende Mutation in dem Oomyzet gebrochen wird.

Die in die erfindungsgemäße Kartoffelpflanze eingebrachte doppelsträngige erste DNA kann eine Nukleotidsequenz umfassen, die mit einer der folgenden Nukleotidsequenzen unter stringenten Bedingungen hybridisiert: (a) einer Nukleotidsequenz gemäß den SEQ ID NOS: 1, oder (b) einem Fragment von mindestens 25 aufeinanderfolgenden Nukleotiden einer Nukleotidsequenz gemäß den SEQ ID NOS: 1, oder (c) einer Nukleotidsequenz die komplementär zu einer der Nukleotidsequenzen nach (a)oder (b) ist. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2 7 % SDS, 1 mM EDTA und 1 % BSA für 16 Stunden und anschließendes zweimaliges Waschen mit 2 x SSC und 0,1 % SDS bei 68 °C.

Von der vorliegenden Erfindung erfasst sind insbesondere auch solche Fragmente von Nukleotidsequenzen, die über einige wenige, beispielsweise 1 oder 2 Nukleotide verfügen, die nicht komplementär zu der Zielgensequenz aus *P. infestans* sind. Im Oomyzeten auftretende Sequenzvariationen, die beispielsweise auf einer genetischen Mutation durch z.B. Addition, Deletion oder Substitution oder auf einem Polymorphismus in einem *Phytophthora infestans-*Stamm basieren, und welche in einer Falschpaarung über einen Bereich von 1, 2 oder mehr Nukleotiden führen, können somit toleriert werden, sofern die von der transgenen Kartoffelpflanze gebildete RNA immer noch mit der vom Oomyzet gebildeten Zielgen-RNA interferiert.

Gemäß der Erfindung bildet die transgene Pflanze der Art *Solanum tuberosum* eine Pathogenresistenz gegenüber einem Oomyzeten der Gattung *Phytophthora* aus. Zur Ermittlung der Resistenz wird dabei die transgene Kartoffelpflanze mit einer Kontrollpflanze verglichen, die idealer Weise den identischen Genotyp wie die transgene Pflanze hat und unter identischen Bedingungen angezogen worden ist, welche aber nicht die DNA enthält, die in die transgene Pflanze eingebracht wurde. Die Ermittlung der Resistenz kann über eine optische Bonitur, wobei Boniturnoten von 0 (nicht anfällig) bis 100 (sehr anfällig) vergeben werden, erfolgen. In bevorzugter Weise bildet die transgene Pflanze gemäß der Erfindung eine Resistenz aus, die im Vergleich zu einer Kontrollpflanze zu einer verringerter Ausbreitung der Infektion auf der Pflanzenoberfläche um mindestens 10, 15, 20, 25, 30, 35, 40, 45, 50, 55, 60, 65, 70, 75, 80, 85, 90, 95, 100 Prozent führt (siehe die Bedingungen unter Messung der Resistenz in transgenen Kartoffelpflanzen unter Freilandbedingungen).

Die Gattung *Phytophthora* umfasst verschiedene Arten, beispielsweise die Arten *alni*, *cactorum, capsici, cinnamomi, citrophthora, clandestina, fragariae, hedraiandra, idaei, infestans, ipomoeae, iranica, kernoviae, mirabilis, megakarya, nicotianae, palmivora, parasitica, phaseoli, ramorum, pseuodotsugae, quercina, sojae* oder *tentaculata.*

Nach einer bevorzugten Ausgestaltung der Erfindung weist die transgene Kartoffelpflanze eine Resistenz gegenüber *Phytophthora infestans* auf.

Bereits die Hemmung der Biosynthese der aliphatischen Aminosäuren Valin, Leucin und Isoleucin durch ein gegen die Acetolactatsynthase von *P. infestans* gerichtetes Konstrukt führte zu einer starken Reduktion des Krautfäulebefalls und zu einer drastischen Erhöhung der Blattresistenz unter Labor- und Feldbedingungen. Durch Kombination mehrerer Zielgene, wie beispielsweise Gene aus G-Proteinsignalling, MAP Kinasesignalling, Primärmetabolismus, Aminosäurebiosynthese und Redoxregulation, in einem Konstrukt, kann die Resistenzwirkung noch erhöht werden, da der Pathogen durch die multiple Wirkung der Kombinationskonstrukte an der Nutzung alternativer Signal- und Stoffwechselwege gehindert wird.

Überraschenderweise ist die Abwehrleistung der erfindungsgemäßen Kartoffelpflanzen gegenüber Isolaten unterschiedlicher Aggressivität von *P. infestans* so verändert, dass eine Resistenz erzielt wird, welche die Pflanze effizient und dauerhaft gegenüber diesem wichtigsten Pathogen schützt.

Auch wurde überraschend gefunden, dass die Resistenz keine hohen energetischen Einbußen oder negative Veränderungen der agronomischen Eigenschaften der Kartoffelpflanze bewirkt. Die Anbauversuche unter feldnahen Bedingungen zeigten keine Beeinträchtigungen der Qualität der Pflanze. Durch die sorgsame Auswahl des Zielgens von *P. infestans,* die eine über 17 aufeinanderfolgende Basenpaare hinausgehende Homologie zu Genen von Nichtzielorganismen (Kartoffel, Mensch, Schwein, Rind) ausschloss, gibt es keine Beschränkung der Pflanzen zur Verwendung als Futter- oder Nahrungsmittel sowie keine Einschränkungen bezüglich Aussaat, Anbau, Ernte oder Prozessierung des Ernteguts. Die Pflanzen können uneingeschränkt als Nutz-, Nahrungs- oder Futtermittelpflanze verwendet werden.

Nach einer bevorzugten Ausgestaltung handelt es sich bei der doppelsträngigen RNA um miRNA oder siRNA.
MiRNA beschreibt kurze interferierende RNA und umfasst natürlich produzierte miRNAs und synthetische miRNAs, welche beispielsweise über rekombinante oder chemische Synthese oder über die Prozessierung von Prä-miRNA hergestellt werden können.
SiRNA beschreibt kurze interferierende RNA und umfasst natürlich produzierte siRNAs und synthetische siRNAs, welche beispielsweise über rekombinante oder chemische Synthese oder über Prozessierung von dsRNAs hergestellt werden können.

Die transgene Pflanze produziert ausgehend von der eingebrachten doppelsträngigen DNA dsRNA, die durch endogene RNAi- bzw. Silencing-Mechanismen zu siRNAs und miRNAs prozessiert werden.

Um dsRNA zu erhalten, kann eine doppelsträngige erste DNA mit einer Nukleotidsequenz gemäß einer der SEQ ID NOS: 1 - 43 oder einem Fragment davon in sense und einer doppelsträngigen zweiten DNA in antisense Orientierung verwendet werden, die durch ein Intron getrennt sind, das keine Ähnlichkeit mit den betreffenden Zielgenen hat. Beispielsweise kann die DNA mit einer Nukleotidsequenz gemäß SEQ ID NO: 1 gegen das Acetolactatsynthasegen aus *P. infestans* gerichtet sein. Bei Expression in einer pflanzlichen Zelle wird ein RNA-Transkript gebildet, welches sich aufgrund der Homologie zwischen den sense und antisense Sequenzbereichen zu einer dsRNA zusammenlagern kann. Durch die fehlende Basenpaarung im Bereich des Introns bildet die dsRNA eine hairpin-Struktur aus. Eine dsRNA mit einer hairpin-Struktur kann auch durch eine doppelsträngige DNA mit einer Nukleotidsequenz gemäß einer der SEQ ID NOS: 1 - 43 in sense und einer zweiten in antisense Orientierung unterschiedlicher Länge bereitgestellt werden. Dabei kann die Nukleotidsequenz in sense Orientierung beispielsweise um 190 Nukleotide länger sein als die Nukleotidsequenz in antisense Orientierung oder *vice versa.*

Definierte Sequenzbereiche der ausgewählten Nukleotidsequenzen der Zielgene werden durch PCR amplifiziert und sowohl in sense als auch in antisense Richtung in einen Vektor kloniert, der zur Synthese von hairpin-Strukturen geeignet ist. Dabei können auch mehrere Fragmente mit Sequenzbereichen verschiedenerer Zielgene in einen Vektor kloniert werden, um ein Kombinations-hairpin-Konstrukt zu generieren. Die Vektoren können über in der Pflanzenbiotechnologie bekannte Transformationsverfahren in eine Pflanzenzelle eingebracht werden. Der Fachmann weiss, dass er beispielsweise auch eine ausgewählte Nukleotidsequenz eines Zielgenes in sense Orientierung in einen Vektor und die Nukleotidsequenz des Zielgens in antisense Orientierung in einen zweiten Vektor klonieren kann, welche dann zum Beispiel über Co-Transformation in eine Pflanzenzelle eingebracht werden.

Der Silencing-Mechanismus geht von dsRNA wie beispielsweise hairpin-RNA-Strukturen oder Genduplexen aus. Die dsRNA wird mittels einer dsRNA-spezifischen Endonuklease (Dicer) zu kurzen dsRNAs führen, die bei Einsatz längerer Nukleotidsequenzen zu kurzen dsRNAs von vorzugsweise zu 21 - 25 Basenpaaren prozessiert werden, ein Prozess der sowohl für "stem-loop" (Prä-miRNA) als auch für lange komplementäre dsRNA-Vorstufen ähnlich abläuft. Argonaut-Proteine als zentrale Komponenten des RNA-induzierten Silencing Complexes (RISC) binden und entwinden siRNA und miRNA, so dass der Leitstrang des Duplexes mittels Basenpaarung gezielt an die mRNA bindet und zu deren Abbau führt. RNAi mittels miRNA bezieht sich auf einen vergleichsweise ähnlichen Prozess, mit dem Unterschied, dass die produzierte miRNA auch partiell Regionen umfasst, die nicht identisch zu den Zielgenen sind.

Nach Befall einer Wirtspflanze mit *P. infestans* kann ein Austausch von in der Pflanze gebildeter RNA, welche gegen ein oder mehrere *Phytophthora*-spezifische Zielgensequenzen gerichtet ist, zwischen der Wirtspflanze und dem Oomyzeten stattfinden. In dem Oomyzeten können diese RNAs zu einem Sequenz-spezifischen Gensilencing von einem oder mehreren Zielgenen führen. An diesem Prozess können auch Proteine und Proteinkomplexe wie Dicer, RISC (RNA-induzierter Silencing-Komplex) sowie die RNAabhängige RNA-Polymerase (RdRP) beteiligt sein.

Es ist bekannt, dass in Pflanzen der siRNA-Effekt fortgeführt werden kann, wenn die RdRP neue siRNAs aus den abgebauten mRNA-Fragmenten synthetisiert. Diese sekundäre oder transitive RNAi kann das Silencing verstärken und auch zum Silencing verschiedener Transkripte führen, wenn diese hochkonservierte Sequenzen teilen.

In einer bevorzugten Ausführung sind die erste DNA und die zweite DNA mit mindestens einem Promotor operativ verknüpft.

Ein "Promotor" ist eine nicht-translatierte DNA-Sequenz, typischerweise stromaufwärts einer kodierenden Region, welche die Bindestelle für die RNA-Polymerase beinhaltet und die Transkription der DNA initiiert. Ein Promotor enthält spezielle Elemente, die als Regulatoren der Genexpression fungieren (z.B. *cis*-regulatorische Elemente). Mit operativ verknüpft ist gemeint, dass die DNA, welche die integrierte Nukleotidsequenz umfasst, verbunden ist mit einem Promotor in einer Weise, die eine Expression dieser Nukleotidsequenz erlaubt. Als weitere Komponente kann die integrierte Nukleotidsequenz stromabwärts mit einem Terminator-Signal verknüpft sein.

Der Promotor kann pflanzlichen, tierischen oder mikrobiellen Ursprungs oder synthetisch hergestellt sein und kann beispielsweise aus einer der folgenden Gruppe von Promotoren ausgewählt werden: konstitutiv, induzierbar, entwicklungsspezifisch, zelltypspezifisch, gewebespezifisch oder organspezifisch. Während konstitutive Promotoren unter den meisten Bedingungen aktiv sind, zeigen induzierbare Promotoren Expression infolge eines induzierenden Signals, welches beispielsweise von biotischen Stressoren wie Pathogenen oder abiotischen Stressoren wie Kälte oder Trockenheit oder Chemikalien ausgehen kann.

Als Promotoren kommen beispielsweise der konstitutive CaMV 35S Promotor (Benfey et al., 1990) sowie der in grünen Geweben aktiven C1 Promotor (Stahl et al., 2004) in Betracht.

Die erste und zweite DNA können aber auch mit einem doppelten Promotor operativ verknüpft sein, wie z.B. dem bidirektional aktiven TR1'und TR2'Promotor (Saito et al., 1991).

Des Weiteren können die erste und die zweite DNA mit jeweils einem Promotor operativ verknüpft sein.

Die Verwendung von zwei Promotoren, die jeweils das 3'- und das 5'-Ende des Nukleinsäuremoleküls flankieren, ermöglicht die Expression des jeweiligen individuellen DNA-Strangs, wobei zwei komplementäre RNAs gebildet werden, die hybridisieren und eine dsRNA bilden. Zudem können die zwei Promotoren derart eingesetzt werden, dass der eine Promotor auf die Transkription einer ausgewählten Nukleotidsequenz und der zweite Promotor auf die Transkription einer zu der ersten Nukleotidsequenz komplementären Nukleotidsequenz gerichtet ist. Sofern beide Nukleotidsequenzen transkribiert werden, entsteht eine dsRNA.

Ferner kann ein bidirektionaler Promotor eingesetzt werden, der eine Expression von zwei Nukleotidsequenzen in zwei Richtungen ermöglicht, wobei eine Nukleotidsequenz in 3'-Richtung und eine zweite Nukleotidsequenz in 5'-Richtung abgelesen wird. Sofern die beiden Nukleotidsequenzen komplementär zueinander sind, kann eine dsRNA gebildet werden.

Die vorliegende Erfindung betrifft auch Teile einer transgenen Pflanze der Art *Solanum tuberosum.*

Im Sinne dieser Anmeldung sind mit "Teilen" der transgenen Pflanze insbesondere Samen, Wurzeln, Blätter, Blüten sowie Zellen der erfindungsgemäßen Pflanze gemeint. Dabei sind unter "Zellen" beispielsweise isolierte Zellen mit einer Zellwand oder Aggregate davon oder Protoplasten zu verstehen. Bei den transgenen Teilen der transgenen Pflanze handelt es sich auch um solche, die geerntet werden können, wie etwa die Kartoffelknollen.

Die vorliegende Erfindung betrifft weiterhin ein Verfahren zur Herstellung einer transgenen Pflanze der Art *Solanum tuberosum,* die eine Resistenz gegenüber einem Oomyzeten der Gattung *Phytophthora* aufweist.

Geeignete Verfahren zur Transformation von Pflanzenzellen sind in der Pflanzenbiotechnologie bekannt. Jedes dieser Verfahren kann genutzt werden, um eine ausgewählte Nukleinsäure vorzugsweise in einem Vektor in eine Pflanzenzelle einzubringen, um eine transgene Pflanze gemäß der vorliegenden Erfindung zu erhalten. Transformationsverfahren können direkte und indirekte Verfahren der Transformation beinhalten und sind für dikotyle und zumeist auch für monokotyle Pflanzen anwendbar. Geeignete direkte Transformationsverfahren schließen PEG-induzierte DNA-Aufnahme, Liposomen-vermittelte Transformation, biolistische Methoden mittels Particle Bombardement, Elektroporation oder Mikroinjektion ein. Zu den indirekten Verfahren zählen beispielsweise die Agrobakterium-vermittelte Transformationstechnik oder die virale Infektion mittels viraler Vektoren.

Ein bevorzugt eingesetztes Verfahren ist der Agrobakterium-vermittelte DNA-Transfer unter Verwendung binärer Vektoren. Nach der Transformation der Pflanzenzellen werden die Zellen auf einen oder mehrere Marker selektiert, die mit der DNA der Erfindung in die Pflanze transformiert wurden und Gene umfassen, die vorzugsweise Antibiotika-Resistenz vermitteln, wie z.B. das Neomycinphosphotransferase II-Gen *NPTII*, welches Kanamycinresistenz vermittelt oder das Hygromycinphosphotransferase II-Gen *HPTII*, welches Hygromycinresistenz vermittelt.

Im Anschluss werden die transformierten Zellen zu vollständigen Pflanzen regeneriert. Nach dem DNA-Transfer und der Regeneration können die erhaltenen Pflanzen beispielsweise über quantitative PCR auf das Vorhandensein der DNA der Erfindung überprüft werden. Resistenzprüfungen dieser Pflanzen gegenüber *P*. *infestans in vitro* und im Gewächshaus schließen sich an. Weitere phänotypische Untersuchungen können im Gewächshaus oder im Freiland routinemäßig von entsprechend geschulten Personen durchgeführt werden. Die auf diese Weise untersuchten transformierten Pflanzen können direkt angezogen werden.

Das erfindungsgemäße Verfahren zur Herstellung einer transgenen Pflanze der Art *Solanum tuberosum,* die eine Resistenz gegenüber einem Oomyzeten der Gattung *Phytophthora* aufweist, umfassent die Schritte:
(i) Herstellen einer transformierten ersten Elternpflanze enthaltend eine doppelsträngige erste DNA , die stabil in das Genom der Elternpflanze integriert ist und die (a) eine Nukleotidsequenz gemäß den SEQ ID NOS: 1 - 43, oder (b) ein Fragment von mindestens 15 aufeinanderfolgenden Nukleotiden einer Nukleotidsequenz gemäß den SEQ ID NOS: 1 - 43, oder (c) eine Nukleotidsequenz, die komplementär zu einer der Nukleotidsequenzen nach (a) oder (b) ist, oder (d) eine Nukleotidsequenz, die mit einer der Nukleotidsequenzen nach (a), (b) oder (c) unter stringenten Bedingungen hybridisiert, umfasst,
(ii) Herstellen einer transformierten zweiten Elternpflanze enthaltend eine doppelsträngige zweite DNA, die stabil in das Genom der Elternpflanze integriert ist, wobei die Nukleotidsequenzen des kodierenden Stranges der ersten und zweiten DNA zueinander ganz oder teilweise invers komplementär sind,
(iii) Kreuzen der ersten Elternpflanze mit der zweiten Elternpflanze,
(iv) Auswählen einer solchen Pflanze, in deren Genom die erste DNA und die zweite DNA zur Ausbildung einer Pathogenresistenz gegenüber einem Oomyzeten der Gattung *Phytophthora* integriert sind, so dass ausgehend hiervon eine doppelsträngige RNA gebildet werden kann.

Erfindungsgemäß handelt es sich um eine Nukleotidsequenz oder ein Fragment einer Nukelotidsequenz gemäß der SEQ ID NO: 1 aus *P. infestans.*

In einer bevorzugten Ausgestaltung des Verfahrens kann es sich bei der doppelsträngigen RNA um miRNA oder siRNA handeln.

Die Erfindung betrifft auch ein Mittel zur äußeren Applikation bei Pflanzen.

Dieses Mittel wird zur äußeren Applikation bei Pflanzen bereitgestellt. Es enthält doppelsträngige RNA, wobei ein Strang dieser RNA dem Transkript einer doppelsträngigen DNA umfassend (a) eine Nukleotidsequenz gemäß der SEQ ID NOS: 1, oder (b) ein Fragment von mindestens 25 aufeinanderfolgenden Nukleotiden einer Nukleotidsequenz gemäß der SEQ ID NO: 1, oder (c) eine Nukleotidsequenz, die komplementär zu einer der Nukleotidsequenzen nach (a) oder (b) ist, oder (d) eine Nukleotidsequenz, die mit einer der Nukleotidsequenzen nach (a), (b) oder (c) unter stringenten Bedingungen hybridisiert, entspricht.

Doppelsträngige RNA zur Herstellung des erfindungsgemäßen Mittels kann *in vitro* über dem Fachmann bekannte Methoden produziert werden. Beispielsweise kann die Synthese der doppelsträngigen RNA synthetisch erfolgen, wobei die RNA *in vitro* direkt gebildet wird.

Die doppelsträngige RNA kann auch ausgehend von einer doppelsträngigen DNA über die Bildung eines mRNA-Transkriptes, welches dann z. B. eine haipin-Struktur ausbildet, synthetisiert werden.

Das erfindungsgemäße Mittel kann als Fungizid für eine Pflanze oder deren Saatgut verwendet werden. Dabei ist das Mittel zur Kontrolle des Pathogenwachstums, zur Eindämmung der Pathogenausbreitung oder zur Behandlung von befallenen Pflanzen einzusetzen. Beispielsweise kann das Mittel als Fungizid durch Aufsprühen in Form eines Sprays oder weiteren dem Fachmann geläufigen Wegen der äußeren Applikation auf das Pflanzengewebe oder durch Aufsprühen oder Mischen mit dem Aufzuchtsubstrat vor oder nach Aufgang der Pflanzen eingesetzt werden.
In einer weiteren Anwendung kommt das erfindungsgemäße Mittel zur Vorbehandlung des Saatguts zum Einsatz. Hierzu wird das Mittel zunächst mit einer Trägersubstanz verbunden und in einer Kombination, welche die doppelsträngige RNA und die Trägersubstanz umfasst, auf das Saatgut aufgebracht, wobei die Trägersubstanz beispielsweise RNAstabilisiernde Wirkung hat. So kann die RNA-Stabilität und damit ihre Wirkung gegenüber den ausgewählten Zielgenen von *P. infestans* zum Beispiel über chemische Modifikationen, wie den Austausch von Ribose gegen eine Hexose, erhöht werden. Als RNA-Stabilisatoren können auch Liposomen, welche die RNA-Moleküle verkapseln, eingesetzt werden. Idealer Weise handelt es sich bei den mit dem Mittel behandelten Pflanzen um solche der Art *Solanum tuberosum.*

Die obigen Ausführungen zur erfindungsgemäßen Pflanze sowie zu dem erfindungsgemäßen Verfahren gelten entsprechend auch für dieses Mittel.

Die vorliegende Erfindung wird nachfolgend mit Bezug auf die Figuren und Sequenzen beschrieben:

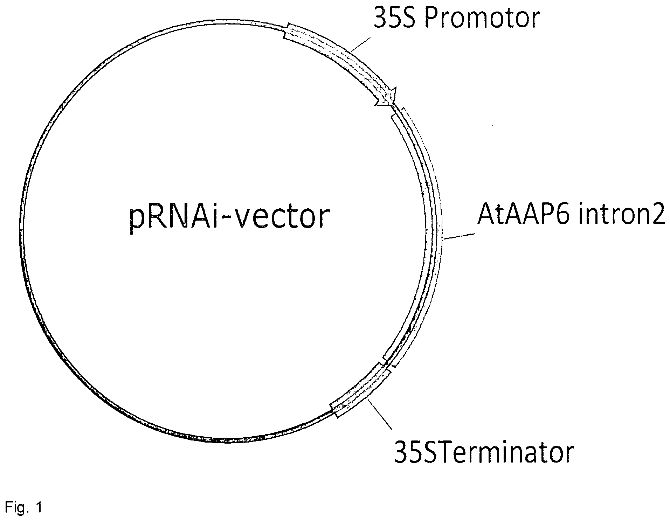

Fig. 1: Plasmid pRNAi als beispielhafte Darstellung eines Vektors, der zur Bildung von hairpin-Konstrukten gegen ein Zielgen genutzt werden kann. Dieser Vektor enthält einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

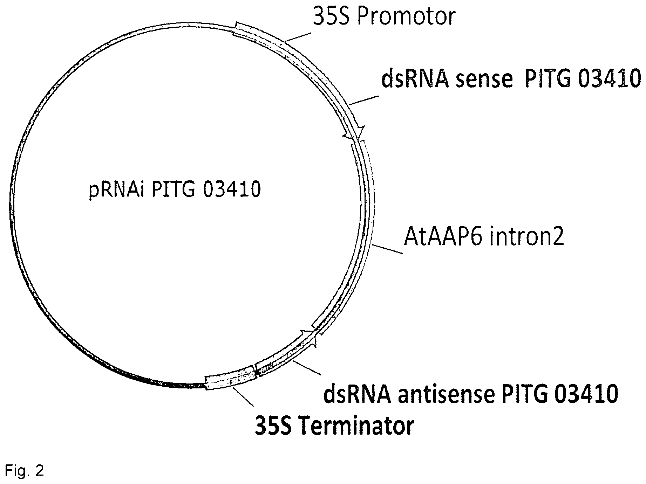

Fig. 2: Plasmid pRNAi_PITG_03410 als beispielhafte Darstellung eines Vektors, der ein sense-Intron-antisense-Fragment zur Bildung von dsRNA gegen ein Zielgen (hier PITG_03410) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

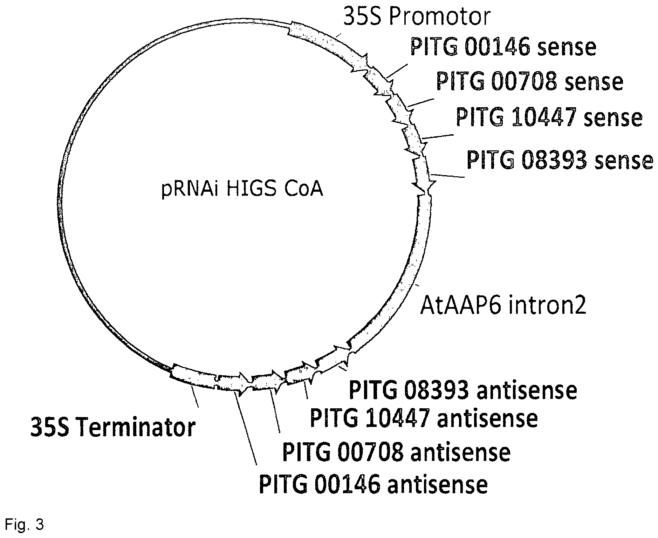

Fig. 3: Plasmid pRNAi_HIGS_CoA als beispielhafte Darstellung eines Vektors, der verschiedene definierte Sequenzen im sense-Intron-antisense-Fragment enthält, das zur Bildung von dsRNA gegen diverse Zielgene führen soll. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

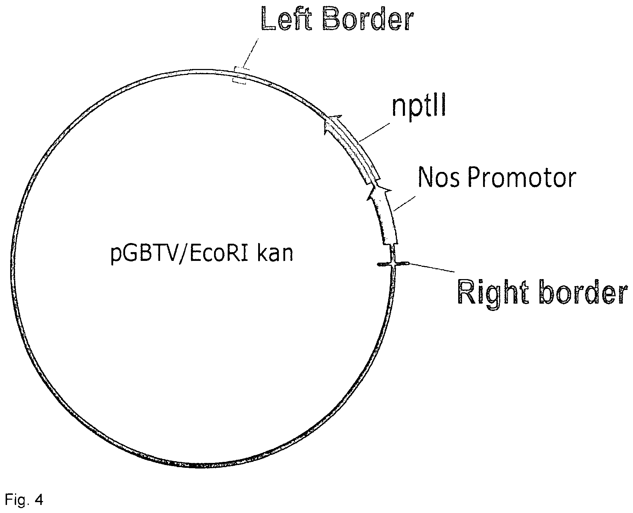

Fig. 4: Plasmid pGBTV/EcoRI_kan. Binäres Ti-Plasmid, das als Klonierungsvektor eingesetzt wurde.

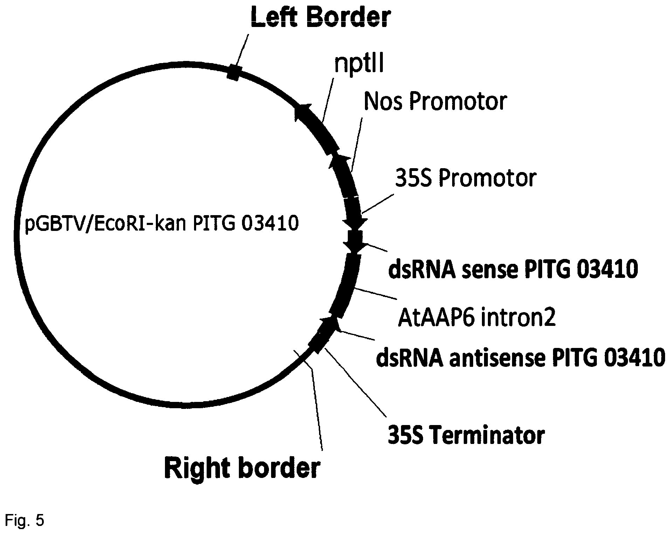

Fig. 5: Plasmid pGBTV/EcoRI_kan_PITG_03410. Binäres Ti-Plasmid, das für die Agrobakterien-vermittelte Transformation eingesetzt wurde.

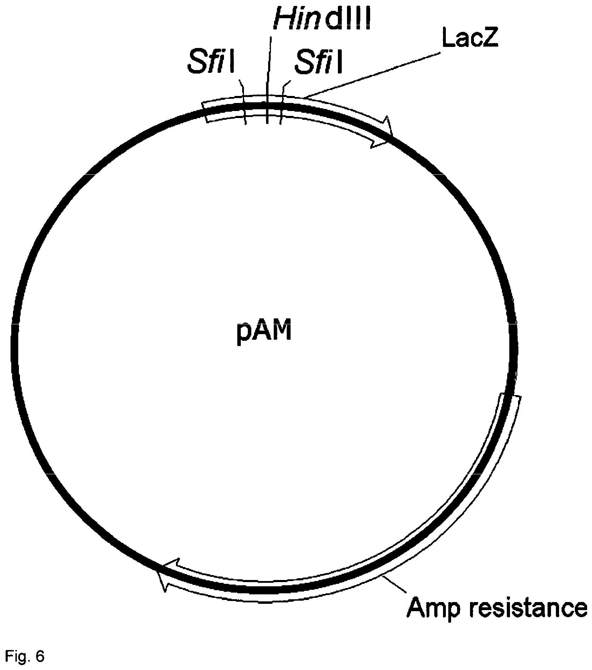

Fig. 6: Plasmid pAM, das als Klonierungsvektor eingesetzt wurde.

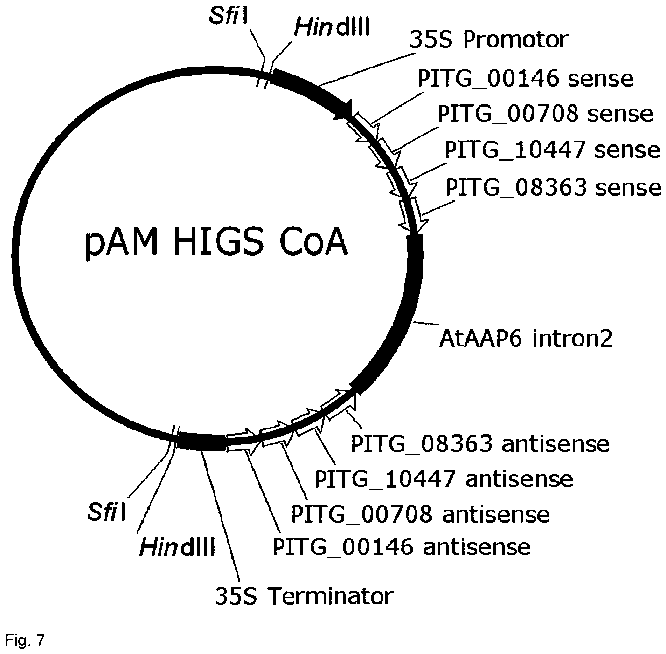

Fig. 7: Plasmid pAM_HIGS_CoA, als beispielhaftes Plasmid, das als Klonierungsvektor eingesetzt wurde.

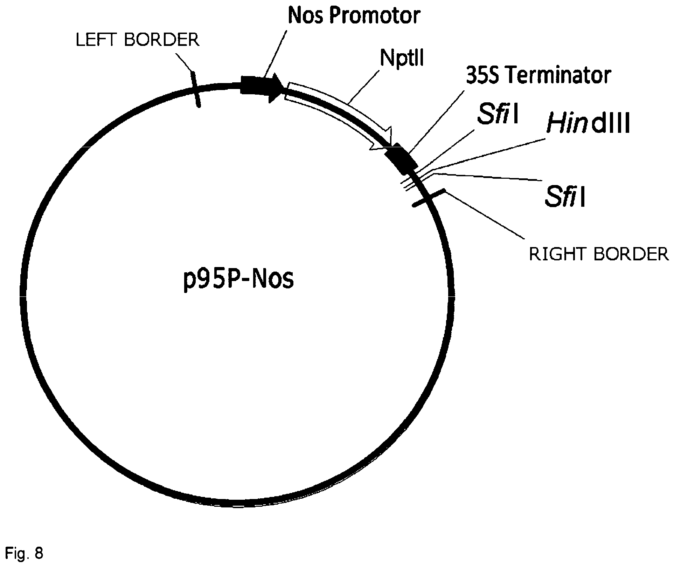

Fig. 8: Plasmid p95P-Nos. Binäres Ti-Plasmid, das als Klonierungsvektor eingesetzt wurde.

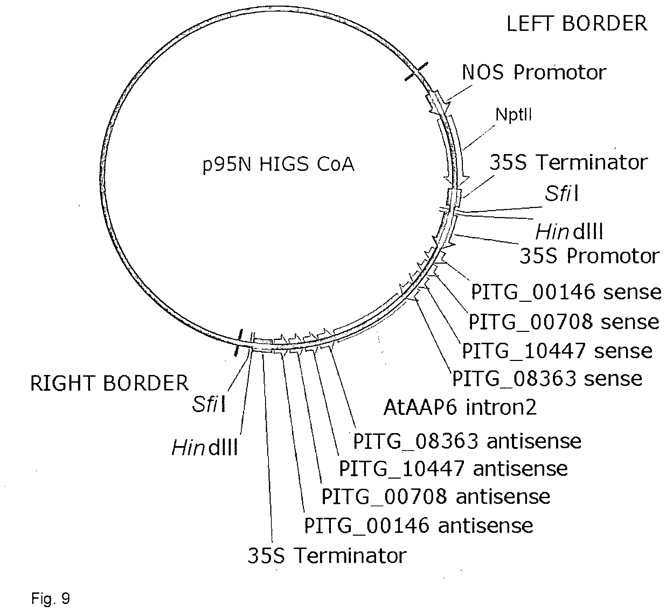

Fig. 9: Plasmid p95N_HIGS_CoA. Binäres Ti-Plasmid, das für die Agrobakterien-vermittelte Transformation eingesetzt wurde.

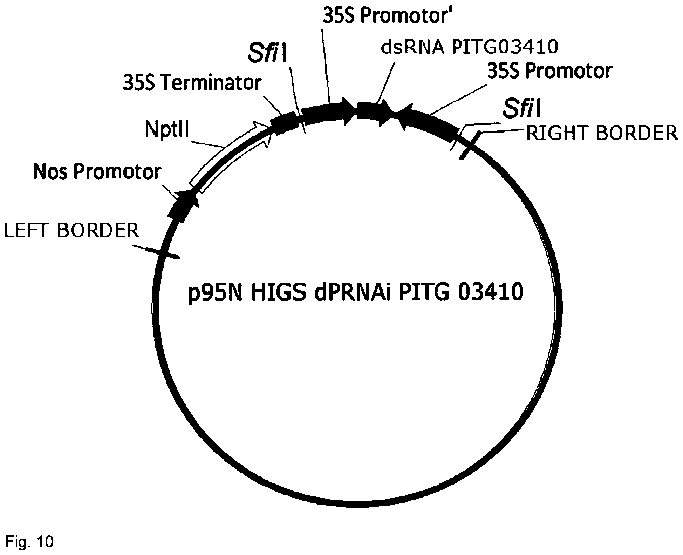

Fig. 10: Plasmid p95N_HIGS_dPRNAi_PITG_03410 als beispielhafte Darstellung eines binären Vektors zur Bildung von dsRNA gegen ein Zielgen (hier PITG_03410) unter Verwendung von zwei CaMV 35S Promotoren, die jeweils das 3'- und das 5'-Ende des Nukleinsäuremoleküls flankieren.

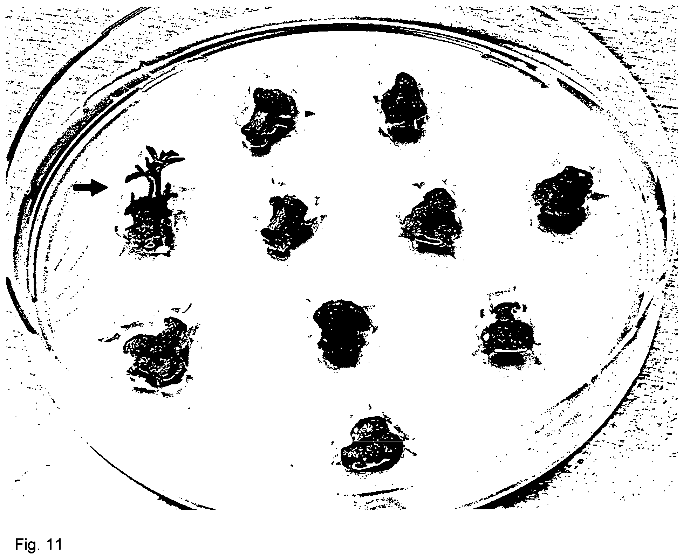

Fig. 11: Transgene Kartoffel-Sprosse auf Selektionsmedium nach der Transformation im Stadium der Regeneration.

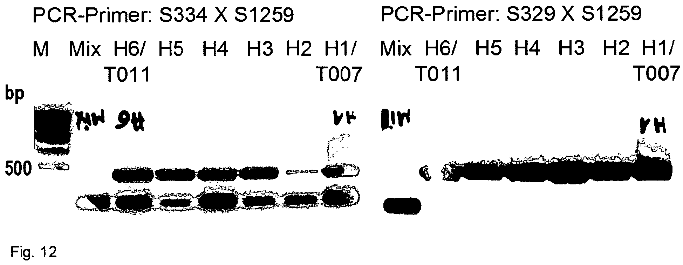

Fig. 12: Diagnostische PCR zur Überprüfung der Transgenität der Kartoffeln (PR-H4) nach Transformation mit dem binären Vektor pGBTV/EcoRI_kan_PITG_03410. Nachweis des sense Fragments (370 bp) (Primer S334 5'-ATCCCACTATCCTTCGCAAG-3' x S1259 5'-TTGATATCGCGGAAGGCGAGAGACATCG-3') und des antisense Fragments (450 bp) (S 329 5'-CTAAGGGTTTCTTATATGCTCAAC-3' x S1259 5'-TTGATATCGCGGAAGGCGAGAGACATCG-3'). Mix: PCR-MasterMix, PCR-Kontrolle. Marker: Tracklt™ 1 Kb DNA Ladder.

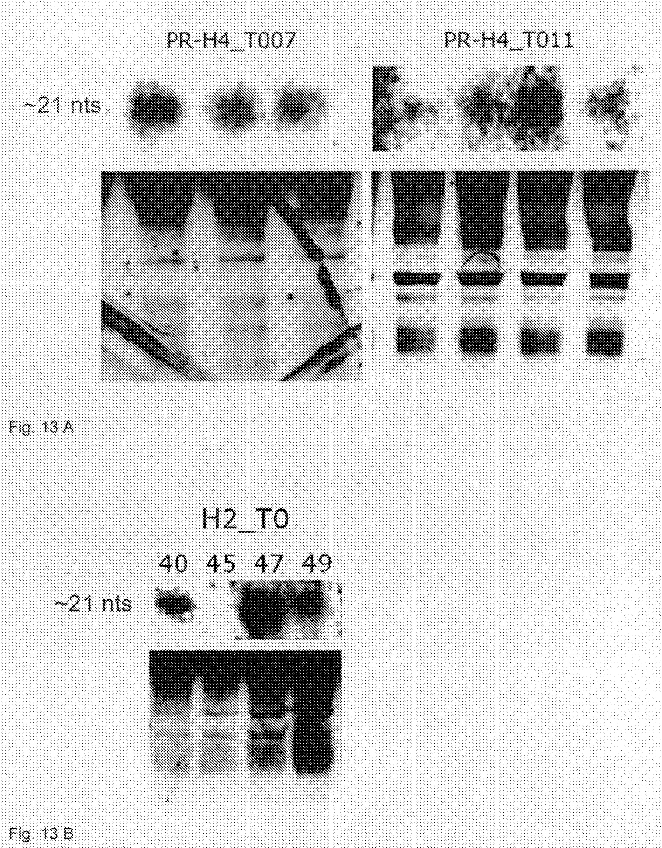

Fig. 13: Detektion siRNAs in transgenen Kartoffelpflanzen nach Transformation mit dem binären Vektor pGBTV/EcoRI_kan_PITG_03410 (A) und dem binären Vektor p95N_HIGS_PITG_00375 (B). Der Nachweis erfolgte mittels Hybridisierung der Northern Blots mit der radioaktiv markierten Sonde dsRNA_PITG03410 (A) bzw. mit der radioaktiv markierten Sonde dsRNA_PITG00375 (B). A: Mehrfache Auftragung verschiedener Proben der Linien PR-H4_T007 und T011. B: Einfache Auftragung der Proben der Linien PR-H2_T040, T045, T047 und T049.

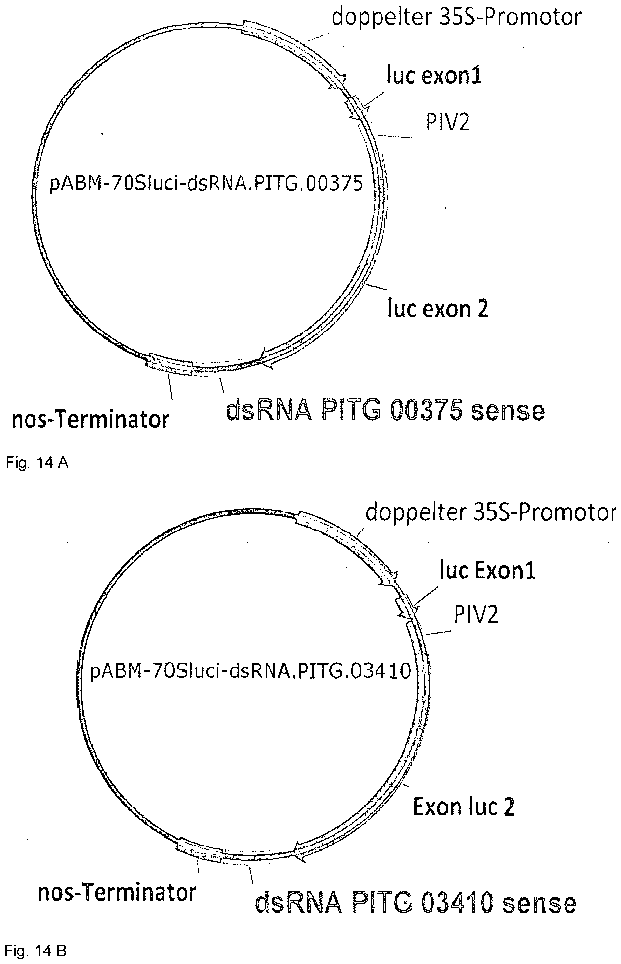

Fig. 14 A: Plasmid pABM-70Sluci_dsRNA.PITG_00375 als beispielhafte Darstellung eines Vektors, der ein Fusion-Konstrukt bestehend aus einem Luciferase-Reportergen und dem zu testenden HIGS-Zielgen-Fragment PITG_00375 enthält. Der Vektor enthält zusätzlich einen doppelten CaMV 35S-Promotor, eine multiple Klonierungsstelle, die kodierende Sequenz des Gens *luc* aus *Photinus pyralis*, das für eine Luciferase kodiert, getrennt von einem modifizierten Intron PIV2 aus dem Kartoffel-Gen *St-LS1* (Eckes et al. 1986, Vancanneyt et al. 1990), eine weitere multiple Klonierungsstelle sowie einen Nos-Terminator des Nopalin Synthase-Gens aus *Agrobacterium tumefaciens.*
   Fig. 14 B: Plasmid pABM-70Sluci_dsRNA.PITG_03410 als beispielhafte Darstellung eines Vektors, der ein Fusion-Konstrukt bestehend aus einem Luciferase-Reportergen und dem zu testenden HIGS-Zielgen-Fragment PITG_03410 enthält.

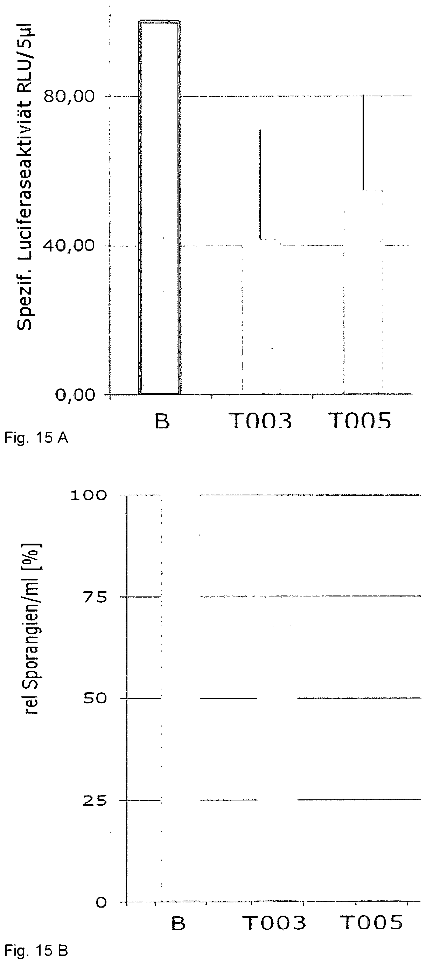

Fig. 15 A: Relative Luciferaseaktivität in transgener Kartoffellinien des Genotyps Baltica mit stabiler Integration des HIGS_RNAi-Konstrukts gegen das Gen PITG_03410 aus *P*. *infestans* nach Beschuss mit dem Vektor pABM-70Sluci_dsRNA.PITG_03410. B: Baltica (nicht transgene Kontrolle), T003, T005 transgene HIGS-Kartoffellinien.
   Fig. 15 B: Relative Sporangienproduktion von *P. infestans* auf transgenen HIGS-Linien. Die Kartoffellinien der Sorte Baltica wurden mit einem RNAi-Konstrukt zur Bildung von dsRNA gegen das *P. infestans-Gen* PITG_03410 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). B: Baltica (nicht transgene Kontrolle), T003, T005: transgene HIGS-Kartoffellinien.

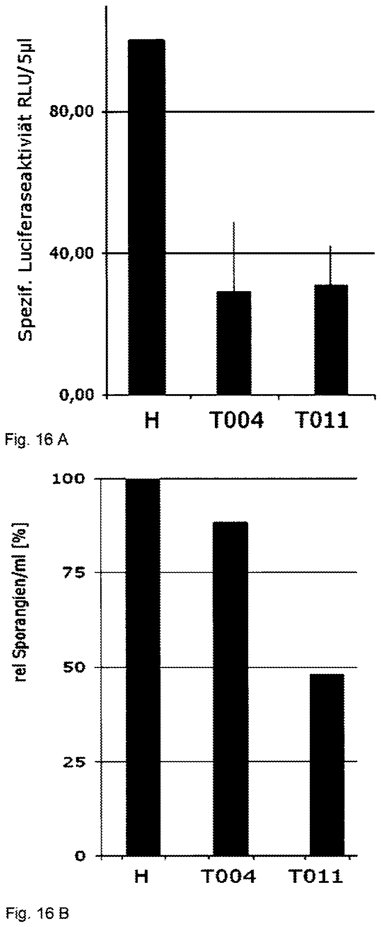

Fig. 16 A: Relative Luciferaseaktivität in transgener Kartoffellinien des Genotyps Hermes mit stabiler Integration des HIGS_RNAi-Konstrukts gegen das Gen PITG_03410 aus *P. infestans* nach Beschuss mit dem Vektor pABM-70Sluci_dsRNA.PITG_03410. H: Hermes (nicht transgene Kontrolle), T004, T011: transgene HIGS-Kartoffellinien.
   Fig. 16 B: Relative Sporangienproduktion von *P. infestans* auf transgenen HIGS-Linien. Die Kartoffellinien der Sorte Hermes wurden mit einem RNAi-Konstrukt zur Bildung von dsRNA gegen das *P. infestans-Gen* PITG_03410 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). H: Hermes (nicht transgene Kontrolle), T004, T011: transgene HIGS-Kartoffellinien.

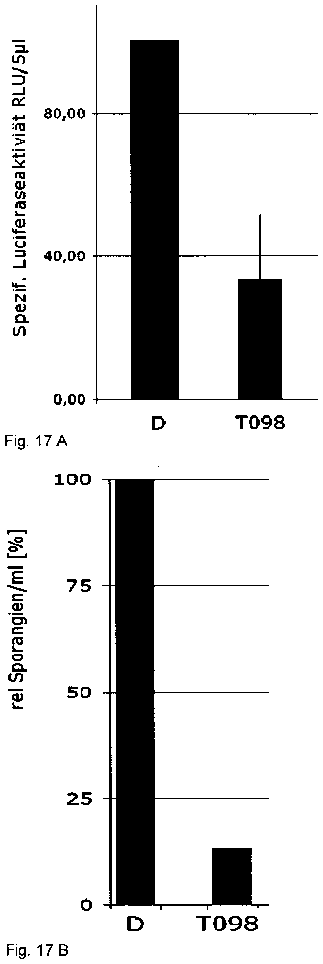

Fig. 17 A: Relative Luciferase-Aktivität transgener Kartoffellinien des Genotyps Desiree mit stabiler Integration des HIGS_RNAi-Konstrukts gegen das Gen PITG_03410 aus *P. infestans* nach Beschuss mit dem Vektor pABM-70Sluci_dsRNA.PITG_03410. D: Desiree (nicht transgene Kontrolle), T098: transgene HIGS-Kartoffellinie.
   Fig. 17 B: Relative Sporangienproduktion von *P. infestans* auf transgenen HIGS-Linien. Die Kartoffellinien der Sorte Desiree wurden mit einem RNAi-Konstrukt zur Bildung von dsRNA gegen das *P. infestans-Gen* PITG_03410 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). D: Desiree, (nicht transgene Kontrolle), T098: transgene HIGS-Kartoffellinie.

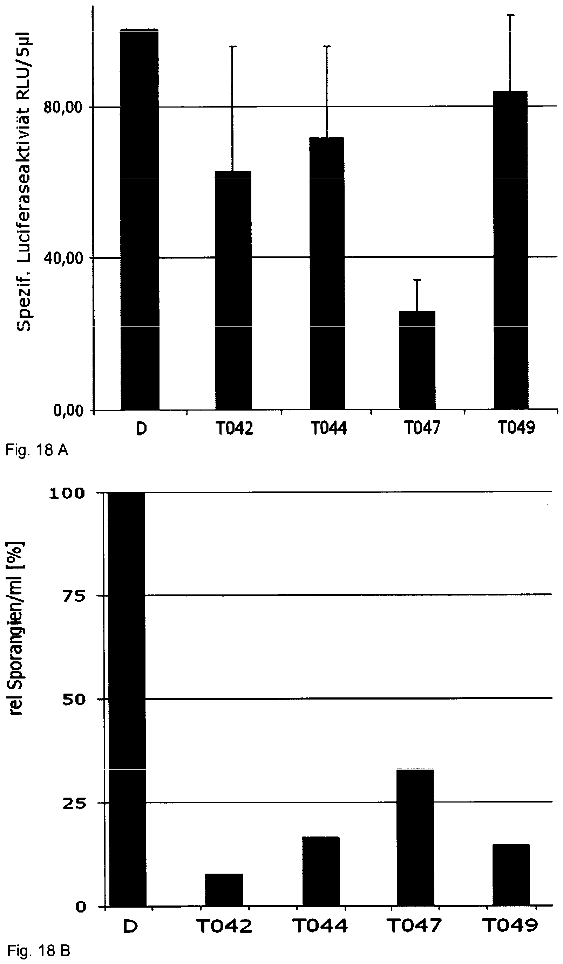

Fig. 18 A: Relative Luciferase-Aktivität transgener Kartoffellinien des Genotyps Desiree mit stabiler Integration des HIGS_RNAi-Konstrukts gegen das Gen PITG_00375 aus *P. infestans* nach Beschuss mit dem Vektor pABM-70Sluci_dsRNA.PITG_00375. D: Desiree, (nicht transgene Kontrolle), T042, T044 T047, T049: transgene HIGS-Kartoffellinien.
   Fig. 18 B: Relative Sporangienproduktion von *P. infestans* auf transgenen HIGS-Linien. Die Kartoffellinien der Sorte Desiree wurden mit einem RNAi-Konstrukt zur Bildung von dsRNA gegen das *P. infestans-Gen* PITG_00375 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). D: Desiree, (nicht transgene Kontrolle), T042, T044 T047, T049: transgene HIGS-Kartoffellinien.

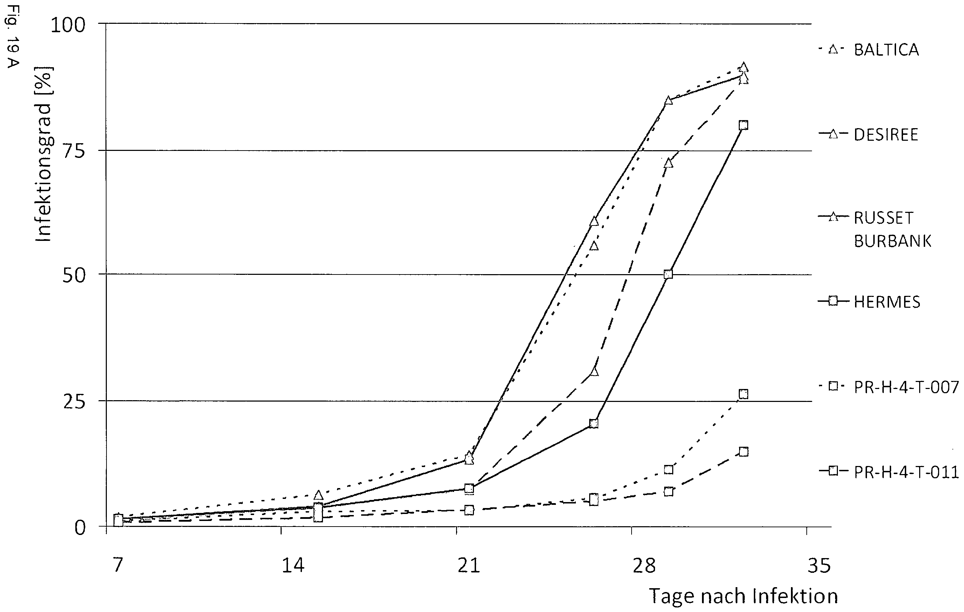

Fig. 19 A: Infektionsgrad transgener Kartoffellinien des Genotyps Hermes mit stabiler Integration des HIGS_RNAi-Konstrukts gegen das Gen PITG_03410 aus *P. infestans* nach Infektion der Pflanzen unter Freiland-ähnlichen Bedingungen mit *P. infestans.* Graue Linien mit Dreieck: Baltica, Desiree, und Russet Burbank (nicht transgene Kontrollen), Schwarze Linien mit Quadrat: Pflanzen des Genotyps Hermes: durchgezogene Linie: Hermes (nicht transgene Kontrolle), gestrichelte Linie: PR-H-4-7 & gepunktete Linie: PR-H-4-11: transgene HIGS-Kartoffellinien.

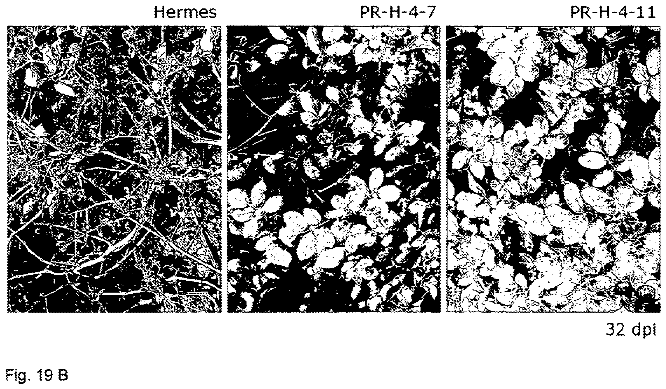

   Fig. 19 B: Photographische Dokumentation des Infektionsgrads der transgenen Kartoffellinien PR-H-4-7 und PR-H-4-11 des Genotyps Hermes mit stabiler Integration des HIGS_RNAi-Konstrukts gegen das Gen PITG_03410 aus *P. infestans* nach Infektion der Pflanzen unter Freiland-ähnlichen Bedingungen mit *P. infestans* im Vergleich zur nicht transgenen Kontrolle Hermes. Photographie 32 Tage nach Infektion.

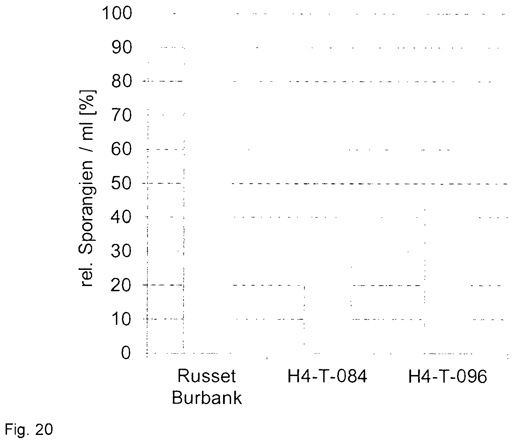

Fig. 20: Relative Sporangienproduktion von *P. infestans* auf transgenen HIGS-Linien. Die Kartoffellinie der Sorte Russet Burbank wurden mit einem RNAi-Konstrukt zur Bildung von dsRNA gegen das *P. infestans-Gen* PITG_03410 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-4-T084, H-4-T096: transgene HIGS-Kartoffellinien.

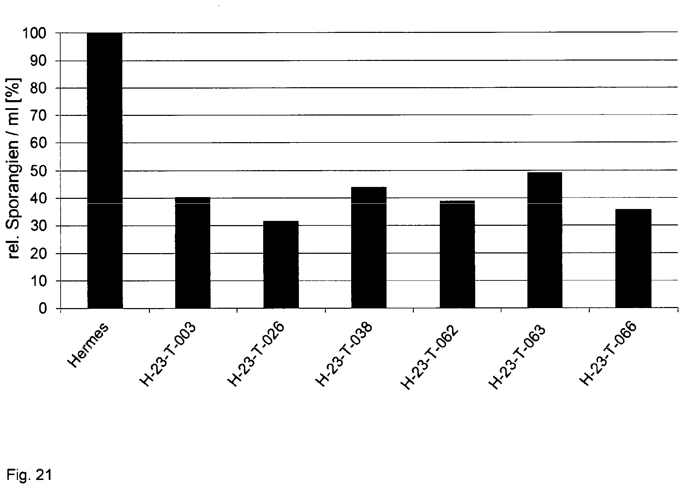

Fig. 21: Relative Sporangienproduktion von *P. infestans* auf transgener HIGS-Linien. Die Kartoffellinien der Sorte Hermes wurden mit einem RNAi-Konstrukt zur Bildung von dsRNA mittels eines Doppel-Promotor-Konstruktes HIGS_dPRNAi_PITG_03410 gegen das *P. infestans*-Gen PITG_03410 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Hermes (Mittelwert aus 3 biologischen Replikaten). Hermes (nicht transgene Kontrolle); H-23-T0003, H-23-T026, H-23-T038, H-23-T062, H-23-T063, H-23-T066: transgene HIGS-Kartoffellinien.

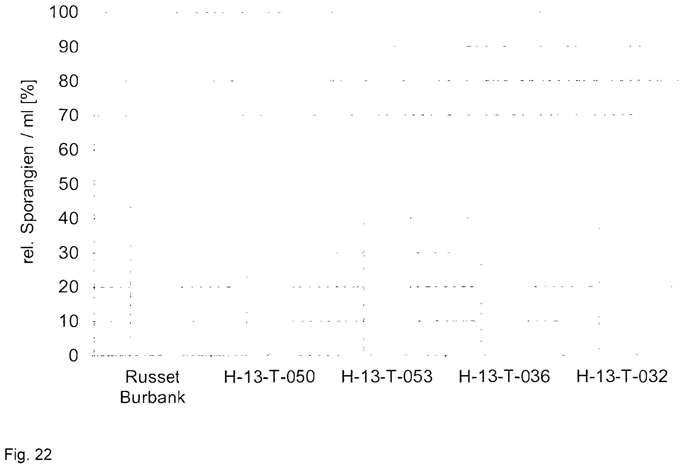

Fig. 22: Relative Sporangienproduktion von *P. infestans* auf transgener HIGS-Linien. Die Kartoffellinien der Sorte Russet Burbank wurden mit einem RNAi-Konstrukt HIGS_CoA zur Bildung von dsRNA gegen die Gene PITG_00146, PITG_08393, PITG_10447 und PITG_00708 aus *P. infestans* transformiert. Diese Linien zeigen nach Infektion mit P. *infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-13-T050, H-13-T053, H-13-T036, H-13-T032: transgene HIGS-Kartoffellinien.

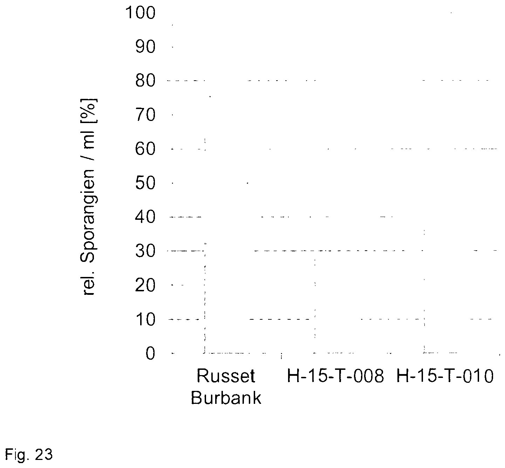

Fig. 23: Relative Sporangienproduktion von *P. infestans* auf transgenen HIGS-Linien. Die Kartoffellinien der Sorte Russet Burbank wurden mit einem RNAi-Konstrukt zur Bildung von dsRNA gegen das *P. infestans*-Gen PITG_06748 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-15-T008, H-15-T010: transgene HIGS-Kartoffellinien.

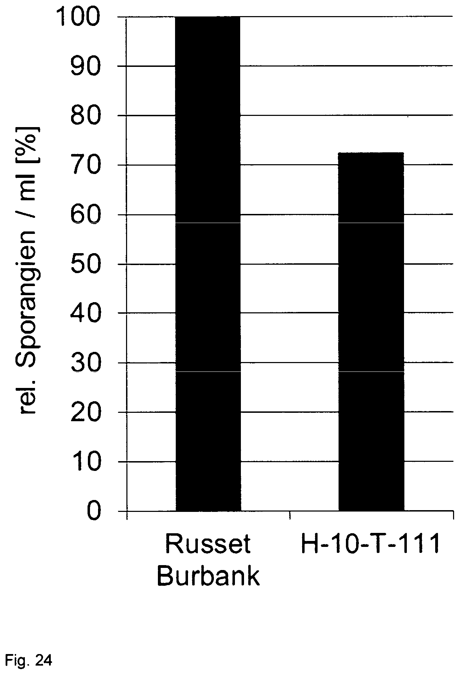

Fig. 24: Relative Sporangienproduktion von *P. infestans* auf transgener HIGS-Linie. Die Kartoffellinie der Sorte Russet Burbank wurden mit einem RNAi-Konstrukt zur Bildung von dsRNA gegen das *P. infestans*-Gen PITG_09306 transformiert. Diese Linie zeigt nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-10-T111: transgene HIGS-Kartoffellinie.

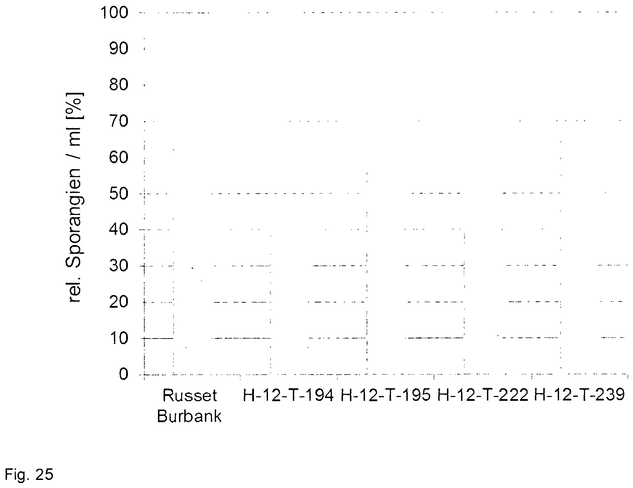

Fig. 25: Relative Sporangienproduktion von *P. infestans* auf transgenen HIGS-Linien. Die Kartoffellinien der Sorte Russet Burbank wurden mit einem RNAi-Konstrukt zur Bildung von dsRNA gegen das *P. infestans*-Gen PITG_09193 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-12-T194, H-12-T195, H-12-T222, H-12-T239: transgene HIGS-Kartoffellinien.

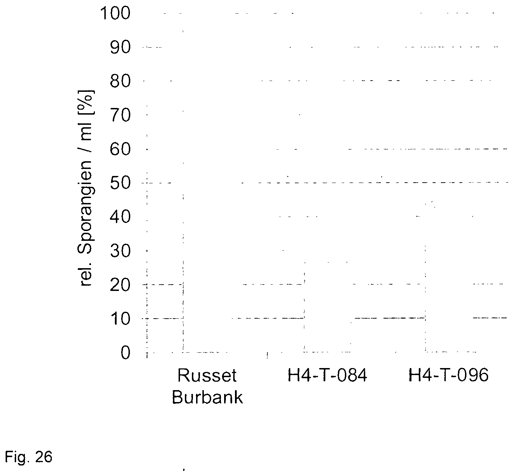

Fig. 26: Relative Sporangienproduktion von *P. infestans* auf transgenen HIGS-Linien. Die Kartoffellinien der Sorte Desiree wurden mit einem RNAi-Konstrukt zur Bildung von dsRNA gegen das *P. infestans*-Gen PITG_09193 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Desiree (Mittelwert aus 3 biologischen Replikaten). Desiree (nicht transgene Kontrolle); H-12-T187, H-12-T216, H-12-T237, H-12-T245: transgene HIGS-Kartoffellinien.

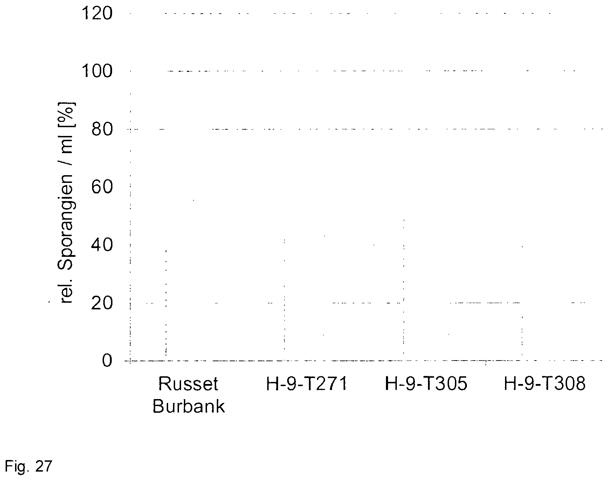

Fig. 27: Relative Sporangienproduktion von *P. infestans* auf transgenen HIGS-Linien. Die Kartoffellinien der Sorte Russet Burbank wurden mit einem RNAi-Konstrukt zur Bildung von dsRNA gegen das *P. infestans*-Gen PITG_19177 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-9-T271, H-9-T305, H-9-T308: transgene HIGS-Kartoffellinien.

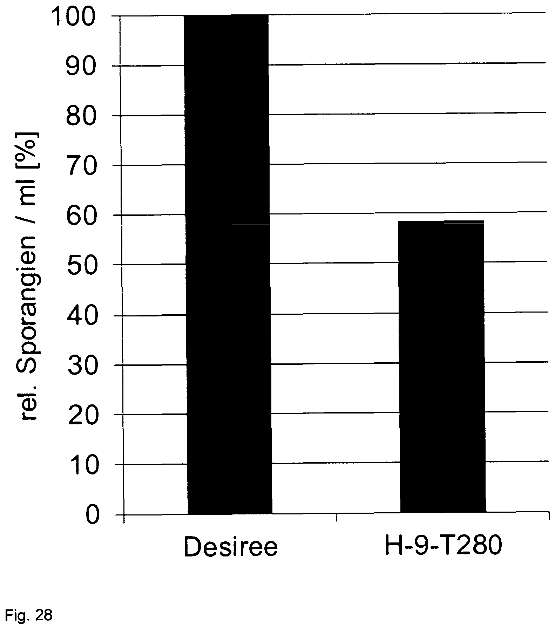

Fig. 28: Relative Sporangienproduktion von *P. infestans* auf transgenen HIGS-Linien. Die Kartoffellinie der Sorte Desiree wurden mit einem RNAi-Konstrukt zur Bildung von dsRNA gegen das *P. infestans*-Gen PITG_19177 transformiert. Diese Linie zeigt nach Infektion mit *P. infestans* im detached leaf assay (Einzelblatt-Biotestsystem) eine verminderte Sporangienproduktion im Vergleich zur nicht-transgenen Sorte Desiree (Mittelwert aus 3 biologischen Replikaten). Desiree (nicht transgene Kontrolle); H-9-T280: transgene HIGS-Kartoffellinie.

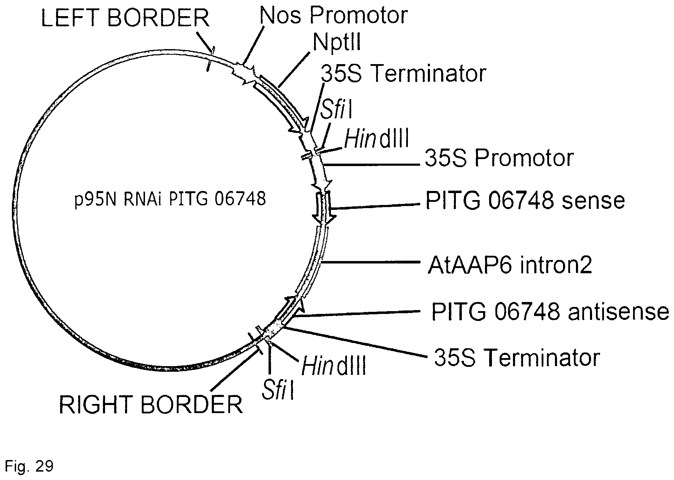

Fig. 29: Plasmid p95N_RNAi PITG_06748 als beispielhafte Darstellung eines Vektors, der ein sense-Intron-antisense-Fragment zur Bildung von dsRNA gegen ein Zielgen (hier PITG_06748) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

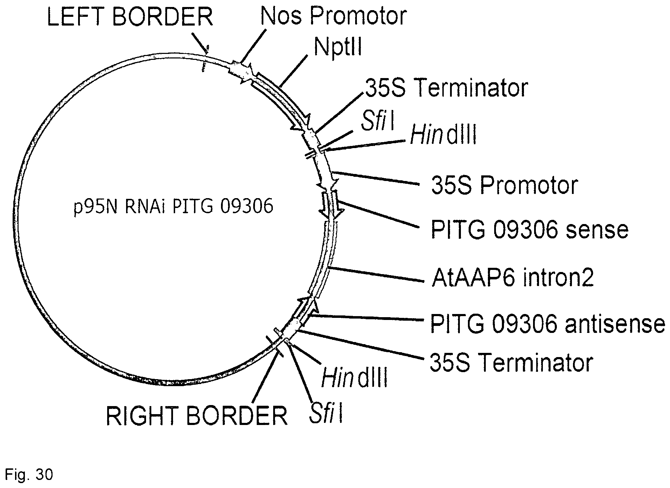

Fig. 30: Plasmid p95N_RNAi_PITG_09306 als beispielhafte Darstellung eines Vektors, der ein sense-Intron-antisense-Fragment zur Bildung von dsRNA gegen ein Zielgen (hier PITG_09306) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

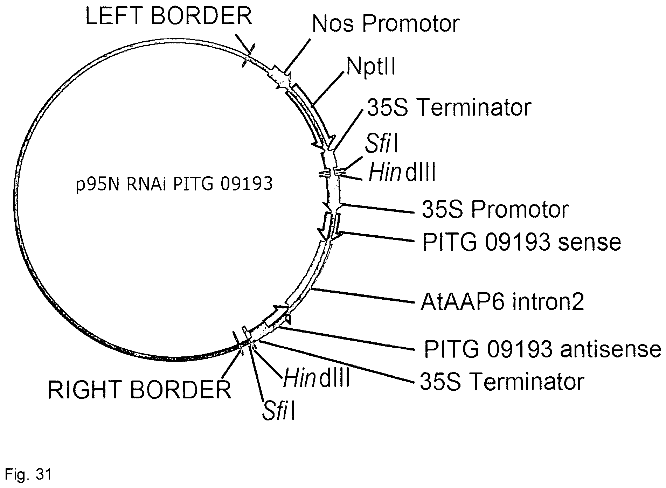

Fig. 31: Plasmid p95N_RNAi_PITG_09193 als beispielhafte Darstellung eines Vektors, der ein sense-Intron-antisense-Fragment zur Bildung von dsRNA gegen ein Zielgen (hier PITG_09193) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

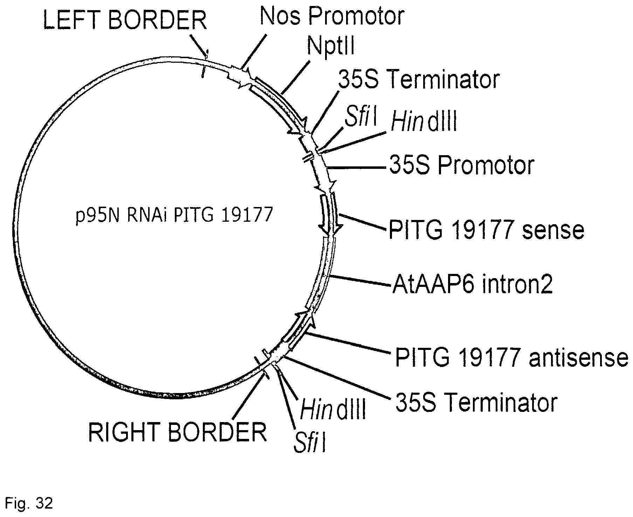

Fig. 32: Plasmid p95N_RNAi_PITG_19177 als beispielhafte Darstellung eines Vektors, der ein sense-Intron-antisense-Fragment zur Bildung von dsRNA gegen ein Zielgen (hier PITG_19177) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

### Ausführungsbeispiele

### Bereitstellung der Konstrukte

Definierte Sequenzbereiche der ausgewählten Zielgene wurden durch PCR amplifiziert und sowohl in sense- als auch in antisense-Richtung in einen Vektor pRNAi kloniert, der zur Synthese von hairpin-Strukturen geeignet ist (Fig. 2). Dabei können auch mehrere Fragmente mit Sequenzbereichen verschiedener Zielgene in einen Vektor kloniert werden, um ein Kombinations-hairpin-Konstrukt zu generieren (Fig. 3).
Ausgehend von genomischer DNA von *P. infestans* wurde mittels PCR ein Sequenzbereich von 290 bp aus dem kodierenden Bereich des Gens PITG_03410 amplifiziert, über die durch die Primersequenzen angehängten Restriktionsenzymschnittstellen XhoI und SmaI ausgeschnitten und in den Vektor pRNAi kloniert (Primer 1: cgctcgaggctggatctcgcgctgaggt, Primer 2: ttgatatcgcggaaggcgagagacatcg). Dieser Vektor enthält einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator. Dieser wurde mit XhoI und Ecl136II geschnitten und der 4.098 kb große Vektoranteil über Agarosegelelektrophorese aufgetrennt und anschließend isoliert. Der Ligationsansatz wurde in *E. coli* Stamm XL1-blue (Stratagene, LaJolla, CA) transformiert. In das Plasmid pRNAi_PITG_03410_sense wurde das gleiche PITG_03410-Fragment nun in antisense Orientierung kloniert. Dazu wurde das Fragment erneut aus genomischer DNA von *P*. *infestans* wurde mittels PCR amplifiziert, über die durch die Primersequenzen angehängten Restriktionsenzymschnittstellen XhoI und SmaI geschnitten und in den mit SmaI - SalI geschnittenen und damit folglich linearisierten Vektor pRNAi_PITG_03410_sense ligiert (Fig. 1). Das Genfragment sense-Intron-antisense (RNAi-PITG_03410) wurde aus dem Vektor pRNAi- geschnitten und in den Vektor pGBTV/EcoRI_kan kloniert (Fig. 4). Dazu wurden sowohl pGBTV/EcoRI_kan als auch pRNAi_PITG_03410 mit HindIII geschnitten und ligiert, so dass das Plasmid pGBTV/EcoRI_kan_PITG_03410 entsteht (Fig. 5). Alternativ wurden HIGS-RNAi-Konstrukte wie beispielsweise HIGS-CoA zunächst in den Vektor pAM (DNA Cloning Service e.K., Hamburg) kloniert (Fig. 6). Dazu wurden sowohl pAM als auch pRNAi_PITG_03410 mit HindIII geschnitten und ligiert, so dass das Plasmid pAM_HIGS_CoA entsteht (Fig. 7). Aus dem Vektor pAM wurde das HIGS_CoA-Fragment mittels Sfil-Verdau und Ligation in der Vektor p95P-Nos (DNA Cloning Service e.K., Hamburg) integriert (Fig. 8), so dass das Plasmid p95N_HIGS_CoA entsteht (Fig. 9), das zur Kartoffeltransformation genutzt wurde.

Alternativ zu einem oben beschriebenen Vektor, der zur Synthese von hairpin-Strukturen geeignet ist, kann ein Abschnitt des kodierenden Zielgenbereiches in der Kartoffelpflanze mit Hilfe zweier gegenläufig (invers) orientierter Promotoren zur Expression gebracht werden (Fig. 10). Außerdem kann ein Gen-Silencing auch über artifizielle microRNA-Konstrukte (amiRNA) gemäß dem Protokoll des Web microRNA Designers (WMD3) erzielt werden. Artifizielle miRNAs sind 21mer einzelsträngige RNAs, die synthetisiert werden können, um gewünschte Gene in Pflanzen gezielt negativ zu regulieren. Die Regulation erfolgt - wie bei siRNAs- über mRNASpaltung. Diese RNAi-Konstrukte werden dann in einen binären Vektor kloniert und durch *Agrobacterium tumefaciens* vermittelte Transformation in Kartoffeln transformiert.

### Transformation und Regeneration

Die Transformation der Kartoffeln erfolgte nach einem modifizierten Protokoll von Pel et al. (2009) unter Verwendung des Antibiotikums Kanamycin. Die Anzucht des Spendermaterials erfolgte in 80 ml MS(D) (25°C; 16 h Tag / 8 h Nacht; 2000 Lux) für 3-4 Wochen. Für die Transformation (C1) wurden aus dem Spendermaterial die Internodien in ca. 0,5 cm große Explantate geschnitten. Diese wurden in Petrischalen mit 10 ml MS(D) (15-20 Explantate/Schale) mit 70 µl einer über Nacht bei 28°C angezogenen *Agrobacterium tumefaciens*-Kultur, die zuvor mit dem in die Pflanzen zu transformierenden HIGS-RNAi Konstrukt als Teil eines binären Vektors wie beispielsweise p95N transformiert wurde, bei 25°C für 2 Tage im Dunkeln inkubiert. Anschließend wurden die Explantate auf Filterpapier getrocknet und in Petrischalen auf MSW-Medium mit Selektionsantibiotikum (400 mg/l Timentin+ 75 Kanamycin mg/l) gesetzt, welche luftdicht abgeklebt und für 2 Wochen (25°C; 16 h Tag / 8 h Nacht; 2000 Lux) kultiviert wurden (C2). Dieser Selektionsschritt wurde im 2-wöchentlichen Rhythmus bis zur Sprossregeneration (ab C3) wiederholt. Regenerierte Sprosse (Fig. 11) wurden zur Bewurzelung auf MS (30 g/l Saccharose) mit Selektionsantibiotikum (250 Timentin mg/l + 100 Kanamycin mg/l) inkubiert und mittels PCR auf die Integration des zu transformierenden Konstruktes getestet und so auf das Vorhandensein der Nukleinsäuren der Erfindung überprüft. Die Verwendung der Primer Bo2299 (5'-GTGGAGAGGCTATTCGGTA-3') und Bo2300 (5'-CCACCATGATATTCGGCAAG-3') führte zu der Amplifikation eines 553 bp großen DNA-Fragments aus dem bakteriellen *NPTII*-Gen, das für die Neomycinphosphotransferase kodiert. Außerdem wurden noch das sense sowie das antisense Fragment mittel PCR nachgewiesen, um die Vollständigkeit des Konstruktes zu gewährleisten (Fig. 12). Die PCR wurde unter Verwendung von 10 ng genomischer DNA, einer Primerkonzentration von 0,2 µM bei einer Annealingtemperatur von 55°C in einem Multicycler PTC-200 (MJ Research, Watertown, USA) durchgeführt. Die Vermehrung der nach PCR positiv-getesteten Sprosse erfolgte auf MS + 30 g/l Saccharose + 400 mg/l Ampicillin.

### Detektion der prozessierten doppelsträngigen RNA und siRNAs

In den transformierten Pflanzen werden die exprimierten hairpin- oder doppelsträngigen RNAs über die natürlichen pflanzlichen RNAi-Mechanismen so prozessiert, dass diese RNA-Moleküle zu kleinen einzelsträngigen RNAs abgebaut werden. Diese siRNAs lagern sich an die mRNA des entsprechenden Zielgens im Oomyzeten und bewirken so ein Silencing dieses Gens. Die Pflanze wird somit in die Lage versetzt, sich selbst gegen angreifende Pathogene zu schützen.
Durch dieses Konzept können transgene Kartoffelpflanzen erstellt werden, die eine erhöhte Resistenz gegen *P. infestans* aufweisen.

Die Transformation von Kartoffelpflanzen mit Konstrukten zur Expression von hairpin- oder doppelsträngigen RNAs soll dazu führen, dass die daraus resultierenden dsRNA über die natürlichen pflanzlichen RNAi-Mechanismen zu siRNAs prozessiert werden. Um die Fragmentierung der dsRNA zu messen, wurde Gesamt-RNA aus den transgenen Pflanzen mittels der Trizol-Methode (Chomczynski und Sacchi, 1987) isoliert. 15 µg Gesamt-RNA / Probe wurden mit Formamid versetzt, denaturiert und in einem 1% Agarosegel mit 10% Formaldehyd in 1 X MOPS-Puffer (0.2 M MOPS (Natriumsalz), 0.05 M NaOAc, 0.01 M EDTA in DEPC dH₂O. pH 7.0 mit NaOH) elektrophoretisch aufgetrennt. Die aufgetrennte RNA wurde aus dem Gel mittels des Northern Blot-Verfahrens in 20 x SSC-Puffer (Saline-Sodiumcitrat Puffer) auf eine Nylon-Membran (positiv geladen) transferiert. Diese wurde mit einer radioaktiv markierten Sonde, die komplementär zur Sequenz des Zielgenfragments ist, welches in sense bzw. in antisense Richtung in dem in die Pflanzen transformierten Konstrukt vorliegt, hybridisiert. So konnten RNA-Fragmente, die komplementär zur Sequenz des dsRNA-Fragments sind, markiert und mittels eines Phosphoimagers detektiert werden.

Die Transformation von Kartoffelpflanzen mit Konstrukten zur Expression von hairpin- oder doppelsträngigen RNAs soll dazu führen, dass die daraus resultierenden dsRNA über die natürlichen pflanzlichen RNAi-Mechanismen zu siRNAs prozessiert werden. Um die Fragmentierung der dsRNA zu siRNAs zu messen, wurde Gesamt-RNA aus den transgenen Pflanzen mittels der Trizol-Methode (Chomczynski und Sacchi, 1987) isoliert. 15 µg Gesamt-RNA / Probe wurden mit Formamid versetzt, denaturiert und in einem Polyacrylamid-Gel mit 15% Tris/Borsäure/EDTA (TBE) und Harnsäure in 0,5 x TBE elektrophoretisch aufgetrennt. Die aufgetrennte RNA wurde aus dem Gel mittels des Tankblot-Verfahrens in 0,5 x TBE auf eine Nylon-Membran (neutral) transferiert. Diese wurde mit einer radioaktiv markierten Sonde, die komplementär zur Sequenz des Zielgenfragments ist, welches in sense bzw. in antisense Richtung in dem in die Pflanzen transformierten Konstrukt vorliegt, hybridisiert. So konnten siRNAs, die komplementär zu Abschnitten der Sequenz des dsRNA-Fragments sind, markiert und mittels eines Phosphoimagers detektiert werden.
In verschiedenen transgenen Kartoffellinien wie beispielsweise PR-H4-Linien oder PR-H2-Linien konnten solche siRNAs nachgewiesen werden (Fig. 13A, B). Dies zeigt, dass die in die Pflanzen transformierten Konstrukte durch pflanzliche RNAi-Mechanismen so erkannt und prozessiert werden, dass siRNAs gegen HIGS-Zielgene aus *P. infestans* entstehen können, die ein Silencing dieser Gene im Pathogen bewirken sollen.

### Messung der Resistenz in transgenen Kartoffelpflanzen im Einzelblatt-Biotestsystem

Für den Resistenztest der transgenen Kartoffelblätter wurden die transgenen Pflanzen aus *in vitro* Pflanzen im Gewächshaus in 5 l -Töpfen angezogen. Nach 6-8 Wochen wurden 2 Fieder pro Pflanze abgeschnitten und in einer geschlossenen Plastikbox, so auf eine feuchte Grodanmatten gelegt, dass der Blattstiel im feuchten Grodanmaterial steckt. Eine hohe Luftfeuchtigkeit ist damit gewährleistet. Die Boxen wurden bei 18°C mit Tag/Nacht-Rhythmus (Sonnenlicht, Februar-September) inkubiert. Die Fiederblättchen wurden mit Tropfen einer Zoosporensuspension (10 µl; 10⁴ Zoosporen / ml) von *P. infestans* inokuliert. Nach 24 h wurden die Deckel der Boxen etwas geöffnet, um einen leichte Luftzirkulation in den Boxen zu ermöglichen. Die optische Bonitur sowie die Quantifizierung der Zoosporen erfolgte nach 6 Tagen. Die optische Bonitur bewertet den Infektionsgrad und die Zersetzung des Fiederblattes durch *P*. *infestans.* Die Auszählung der Sporangien ermöglicht die Quantifizierung der Reproduktionsfähigkeit des Pathogens auf der Pflanze. Dabei wurden die zuvor infizierten Blättchen eines Fieders in 5 ml Wasser in Falconröhrchen auf einem Schüttler für 2 h inkubiert, so dass sich die Sporangien vom Blatt lösen. Die Sporangien wurden dann mit Hilfe der Thoma-Zählkammer unter dem Mikroskop ausgezählt.

In verschiedenen HIGS-Kartoffellinien, die durch Transformationen unterschiedlicher Kartoffelgenotypen entstanden sind, konnte eine reduzierte Sporangienzahl nach Infektion mit *P. infestans* (6 dpi) bestimmt werden (Fig. 15B -18B). Dies weist darauf hin, dass die Reproduktionsfähigkeit des Pathogens auf den transgenen Pflanzen beeinträchtigt ist.

Das Gen PITG_03410 wurde als Zielgen für HIGS im genetischen Hintergrund der Kartoffelsorten Baltica, Hermes und Desiree als auch der Sorte Russet Burbank unter Verwendung eines Vektors nach Fig. 5 getestet. Das Einzelblatt-Biotestsystem angewendet auf diese transgenen Pflanzen der Sorte Russet Burbank zeigt, dass die Reproduktionsfähigkeit des Pathogens auf den transgenen Pflanzen im Vergleich zu nicht-transgenen Kontrollpflanzen beeinträchtigt ist (Fig. 20).
Alternativ zu Vektoren, die zur Synthese von hairpin Strukturen geeignet sind, wurde ein Vektor nach Fig. 10 mit dem Zielgen PITG_03410 in Kartoffelpflanzen der Sorte Hermes eingebracht und die transgenen Linien im Einzelblatt-Biotestsystem untersucht (Fig. 21). Auch hier zeigt sich, dass die Reproduktionsfähigkeit von *P. infestans* auf den transgenen Pflanzen im Vergleich zu nicht-transgenen Kontrollpflanzen beeinträchtigt ist.
Es wurden auch Kartoffelpflanzen der Sorte Russet Burbank, die mit einem Kombinationsvektor gegen die Gene PITG_00146, PITG_00708, PITG_10447 und PITG_08363 nach Fig. 3 transformiert wurden, im Einzelblatt-Biotestsystem getestet. Es zeigt sich, dass die Reproduktionsfähigkeit von *P. infestans* auf diesen transgenen Pflanzen im Vergleich zu nicht-transgenen Kontrollpflanzen beeinträchtigt ist (Fig. 22). Ebenso zeigt sich im Einzelblatt-Biotestsystem, dass die Reproduktionsfähigkeit von *P. infestans* auf transgenen Pflanzen der Sorte Russet Burbank beeinträchtigt ist, die mit einem Vektor nach Fig. 29, nach Fig. 30, nach Fig. 31 oder nach Fig. 32 transformiert wurden, der gegen die Gene PITG_06748 (Fig. 23), PITG_09306 (Fig. 24), PITG_09193 (Fig. 25) bzw. PITG_19177 (Fig. 27) gerichtet ist.
Auch auf transgenen Pflanzen der Sorte Desiree, welche mit einem Vektor nach Fig. 31 oder nach Fig. 32 transformiert wurden, der gegen das Gen PITG_09193 (Fig. 25) bzw. PITG_19177 (Fig. 27) gerichtet ist, zeigte sich im Einzelblatt-Biotestsystem, dass die Reproduktionsfähigkeit von *P. infestans* im Vergleich zu nicht-transgenen Kontrollpflanzen beeinträchtigt ist.

### Transientes Testsystem für die RNAi-Vektoren in Kartoffelblättern

Es wurde ein transientes Testsystem nach Birch et al. (2010) entwickelt, um die Funktionalität der RNAi-Vektoren gegen ausgewählte Zielgensequenzen von *P*. *Infestans* zu überprüfen. Mittels Co-Bombardement wurden ein auf ein Zielgen abzielender RNAi-Vektor zusammen mit einem Fusion-Konstrukt bestehend aus einem Luciferase-Reportergen und dem zu testenden Zielgen-Fragment transient in Kartoffelblättern exprimiert. Wird das im RNAi-Vektor kodierte dsRNA-Konstrukt prozessiert, sollte die Bildung von dsRNA und daraus resultierend die Bildung von siRNAs gewährleistet sein. Diese siRNAs sollen nicht nur den Abbau des Zielgen-Fragment-Transkripts sondern auch des daran fusionierten Reportergen-Transkripts bewirken, so dass bei einem funktionalen RNAi-Konstrukt eine Reduktion der Luciferaseaktivität beobachtet werden kann. Das Plasmid pABM-70Sluci umfasst einen doppelten CaMV 35S-Promotor, eine multiple Klonierungsstelle, die kodierende Sequenz des Gens *luc* aus *Photinus pyralis*, das für eine Luciferase kodiert, getrennt von einem modifizierten Intron PIV2 aus dem Kartoffel-Gen *St-LS1* (Eckes et al. 1986, Vancanneyt et al. 1990), eine weitere multiple Klonierungsstelle sowie einen Nos-Terminator des Nopalin Synthase-Gens aus Agrobacterium tumefaciens. In dieses Plasmid pABM-70Sluci wurde das mittels PCR amplifizierte Fragment des kodierenden Sequenzbereiches beispielsweise des PITG_03410-Gens kloniert, das auch zur Herstellung des dsRNA-Konstruktes in den pRNAi-Vektor kloniert wurde (Fig. 14 A) Dieses transiente Testsystem kann nicht nur zur Validierung der generellen Funktionalität der RNAi-Konstrukte dienen, sondern außerdem genutzt werden, um verschiedene Sequenzbereiche eines Genes auf deren Silencing-Effekt zu untersuchen und schließlich die besten Sequenzbereiche eines Gens für ein optimales Silencing auszuwählen. Außerdem kann der Beschuss transgener stabil mit einem RNAi-Konstrukt transformierter Pflanzen dazu dienen, die Silencing-Effizienz der einzelnen transgenen HIGS-Kartoffellinien zu bestimmen, die sich beispielsweise abhängig vom Integrationsort des Konstruktes stark unterscheiden kann. In verschiedenen transgenen HIGS-Kartoffellinien, die durch Transformationen in unterschiedliche Kartoffelgenotypen entstanden sind, und die eine reduzierte Sporangienzahl nach Infektion mit *P. infestans* zeigten, konnte auch eine Reduktion der Luciferaseaktivität gemessen werden (Fig. 15A - 18A). Dies zeigt die Funktionalität der zu siRNAs prozessierten HIGS-Konstrukte in Bezug auf ein Silencing der Zielgensequenz in den transgenen Pflanzen.
Wenn die kodierenden Gensequencen, die von einem Kombinationskonstrukt wie beispielsweise pRNAi_HIGS-CoA gesilenced werden sollen (Zielgene: PITG_08393, PITG_00146, PITG_10447, PITG_00708), jeweils in den Vektor pABM_70Sluci hinter die kodierende Sequenz des Gens *luc* aus *Photinus pyralis* kloniert werden (pABM_70Sluci_PITG_08393, pABM_70Sluci_PITG_00146, pABM_70Sluci_PITG_10447, pABM_70Sluci_PITG_00708) und zusammen mit dem Vektor pRNAi_HIGS_CoA in Kartoffelblättern transient exprimiert werden, kann die Silencing-Effizienz des Kombinationskonstrukts auf die verschiedenen Zielgene analysiert werden. Dies ist auch möglich durch den Beschuss transgener stabil mit dem RNAi-Kombinationskonstrukt transformierter Pflanzen mit den einzelnen Fusion-Konstrukten bestehend aus dem Luciferase-Reportergen und den zu testenden kodierenden Sequenzen der verschiedenen Zielgene.
Die Luciferase-Aktivitätsbestimmungen wurden mit Hilfe des Dual Luciferase® Reporter Assays (Promega, Mannheim) durchgeführt (Schmidt et al. 2004).

### Messung der Resistenz in transgenen Kartoffelpflanzen unter Freilandbedingungen

Für den Resistenztest der transgenen Kartoffelpflanzen unter Freilandbedingungen wurden die transgenen Pflanzen im Frühjahr (März) zunächst aus *in vitro* Pflanzen im Gewächshaus für 3 Wochen in Multitopfplatten angezogen. Danach wurden diese Pflanzen in ein Gewächshaus mit Maschendrahtdach in gewachsenem Boden ausgepflanzt, so dass die Pflanzen Umweltbedingungen beeinflusst beispielsweise durch Temperatur, Sonneneinstrahlung, Niederschlag und Luftfeuchtigkeit, welche mit natürlichen Feldbedingungen vergleichbar sind, ausgesetzt waren. Die Pflanzen wurden in je 3 Parzellen mit jeweils 6 Pflanzen ausgepflanzt. Nach 8 Wochen wurde jeweils ein Fieder von 2 Pflanzen einer Parzelle mittels Sprühinokulation (750 µl; 10⁴ Zoosporen / ml) von *P*. *infestans* inokuliert. Über diese Fieder wurden Plastiktüten gestülpt, um eine hohe Luftfeuchtigkeit zu gewährleisten, die die Infektion begünstigt. Nach zwei Tagen wurden diese Tüten entfernt. Die Ausbreitung der Krautfäule durch *P. infestans* im Gewächshaus wurde wöchentlich optisch bonitiert und photographisch dokumentiert. Die Kriterien zur Bonitur der Infektion beziehen sich zunächst nur auf das infizierte Fiederblatt (0: keine Infektion, 1: leichte Infektion (1/2 Anzahl der infizierten Fiederblättchen befallen), 2: Infektion auf mehr als 1/2 Blättchen des Fieders, 3: Infektion auf allen Blättchen des Fieders) und bewerten dann die Ausbreitung der Infektion auf der Pflanze und in der gesamten Parzelle (4: Infektion breitet sich auch auf einige andere Blätter der Pflanze aus, 6: Infektion breitet sich auch auf andere Pflanzen aus, 8: Infektion breitet sich auch auf andere Pflanzen stark aus, 10: 10% der Pflanze infiziert / zerstört, 20: 20% der Pflanzen infiziert / zerstört, 100: 100% der Pflanze infiziert / zerstört).
In verschiedenen transgenen HIGS-Kartoffellinien (PR-H-4-7, PR-H-4-11), die durch Transformationen in den Kartoffelgenotypen Hermes entstanden sind, konnte eine stark reduzierter Infektionsgrad dieser Pflanzen im Verlauf der Infektion mit *P. infestans* im Vergleich zum Transformationsgenotyp Hermes, der als Kontrolle genau wie die transgenen Pflanzen angezogen, ausgepflanzt und infiziert worden war, bestimmt werden. Der reduzierte Infektionsgrad spiegelte sich zunächst durch eine stark verringerte Infektionsfähigkeit des Pathogens auf den inokulierten Fiedern (Boniturnoten 21 Tage nach Infektion: PR-H-4-7: 3,3; PR-H-4-11: 3,2; Hermes: 7,6) und zu späteren Zeitpunkten durch eine deutlich verringerte Ausbreitungsfähigkeit des Pathogens auf diesen Pflanzen wider (Boniturnoten 32 Tage nach Infektion: PR-H-4-7: 26; PR-H-4-11: 15; Hermes: 80) (Fig. 19 A, B).

Durch die beschriebenen Versuche konnte nicht nur die Prozessierung der HIGS-Konstrukte in transgenen Kartoffelpflanzen zu siRNAs, sondern auch die Funktionalität dieser Konstrukte in Bezug auf das Silencing der Zielgensequenz in diesen transgenen Pflanzen sowie eine erhöhte Resistenz dieser Pflanzen gegenüber *P. infestans* quantifizierbar durch eine reduzierte Sporangienproduktion des Pathogens auf diesen Wirtspflanzen demonstriert werden. Da die Identifizierung funktionaler HIGS-Zielgene nicht ohne sorgfältige Überprüfung ihrer Funktionalität möglich ist, eignen sich diese hier beschriebenen ausführlichen Analysen besonders gut, um für das HIGS-Konzept wirksame Gene zu definieren und resistente HIGS-Pflanzen zu generieren.

### Referenzen

Avrova AO, Boevink PC, Young V, Grenville-Briggs LJ, van West P, Birch PR, Whisson SC (2008) A novel Phytophthora infestans haustorium-specific membrane protein is required for infection of potato. Cell Microbiol. 10(11):2271-84.
Birch RG, Shen B, Sawyer BJ, Huttner E, Tucker WQ, Betzner AS (2010) Evaluation and application of a luciferase fusion system for rapid in vivo analysis of RNAi targets and constructs in plants. Plant Biotechnol J. May 1;8(4):465-75. Epub 2010 Jan 19
Blackman LM, Arikawa M, Yamada S, Suzaki T, Hardham AR (2011) Identification of a mastigoneme protein from Phytophthora nicotianae. Protist. 162(1):100-14.
Benfey, P. N., Ren, L., and Chua, N.-H. (1990). Combinatorial and synergistic properties of CaMV 35S enhancer subdomains. EMBO J. (9), 1685-1696.
Chomczynski P, Sacchi N. (1987) Single-step method of RNA isolation by acid guanidinium thiocyanate-phenol-chloroform extraction. Anal Biochem.162(1):156-9
Eckes P., Rosahl S., Schell J., Willmitzer L. (1986) Isolation and characterization of a lightinducible, organ-specific gene from potato and analysis of its expression after tagging and transfer into tobacco and potato shoots. Molecular and General Genetics 205 (1) 14-22, DOI: 10.1007/BF02428027
Fire A, Xu S, Montgomery MK, Kostas SA, Driver SE, Mello CC. (1998) Potent and specific genetic interference by double-stranded RNA in Caenorhabditis elegans. Nature Feb 19;391(6669):806-11
Grenville-Briggs LJ, Avrova AO, Bruce CR, Williams A, Whisson SC, Birch PR, van West P (2005) Elevated amino acid biosynthesis in Phytophthora infestans during appressorium formation and potato infection. Fungal Genet Biol. 42(3):244-56.
Inoue SB, Takewaki N, Takasuka T, Mio T, Adachi M, Fujii Y, Miyamoto C, Arisawa M, Furuichi Y, Watanabe T (1995) Characterization and gene cloning of 1,3-beta-D-glucan synthase from Saccharomyces cerevisiae. Eur J Biochem 231(3):845-54.
Judelson HS, Narayan RD, Ah-Fong AM, Kim KS (2009a) Gene expression changes during asexual sporulation by the late blight agent Phytophthora infestans occur in discrete temporal stages. Mol Genet Genomics. 281(2): 193-206.
Judelson HS, Tani S, Narayan RD (2009b) Metabolic adaptation of Phytophthora infestans during growth on leaves, tubers and artificial media. Mol Plant Pathol. 10(6):843-55.
Kamoun S, van West P, Vleeshouwers VG, de Groot KE, Govers F. (1998) . Resistance of nicotiana benthamiana to phytophthora infestans is mediated by the recognition of the elicitor protein INF1. Plant Cell. Sep;10(9):1413-26.
Lesage G, Sdicu AM, Menard P, Shapiro J, Hussein S, Bussey H (2004) Analysis of beta-1,3-glucan assembly in Saccharomyces cerevisiae using a synthetic interaction network and altered sensitivity to caspofungin. Genetics. May; 167(1):35-49
Li A, Wang Y, Tao K, Dong S, Huang Q, Dai T, Zheng X, Wang Y (2010) PsSAK1, a Stress-Activated MAP Kinase of Phytophthora sojae, Is Required for Zoospore Viability and Infection of Soybean. Mol Plant Microbe Interact. 23(8):1022-31.
Mazur P, Morin N, Baginsky W, el-Sherbeini M, Clemas JA, Nielsen JB, Foor F (1995) Differential expression and function of two homologous subunits of yeast 1,3-beta-D-glucan synthase. Mol Cell Biol. 15(10):5671-81.
Pel MA, Foster SJ, Park TH, Rietman H, van Arkel G, Jones JDG, Van Eck HJ, Jacobsen E, Visser RGF, Van der Vossen EAG (2009) Mapping and cloning of late blight resistance genes from Solanum venturii using an interspecific candidate gene approach. MPMI 22:601-615
Roemer T, Paravicini G, Payton MA, Bussey H (1994) Characterization of the yeast (1-->6)-beta-glucan biosynthetic components, Kre6p and Skn1p, and genetic interactions between the PKC1 pathway and extracellular matrix assembly. J Cell Biol. 127(2):567-79.
Saito, K., Yamazaki, M., Kaneko, H., Murakoshi, I., Fukuda, Y., and van Montagu, M. (1991). Tissue-specific and stress-enhancing expression of the TR promoter for mannopine synthase in transgenic medicinal plants. Planta 184, 40-46.
Schmidt K., Heberle B., Kurrasch J., Nehls R., Stahl D.J. (2004) Suppression of phenylalanine ammonia lyase expression in sugar beet by the fungal pathogen Cercospora beticola is mediated at the core promoter of the gene. Plant Mol. Biol., 55: 835-852.
Stahl D. J., Kloos, D. U., and Hehl, R. (2004). A sugar beet chlorophyll a/b binding protein void of G-box like elements confer strong and leaf specific reporter gene expression in transgenic sugar beet. BMC Biotechnology 4;31: 12
Vancanneyt G., Schmidt R., O'Connor-Sanchez A., Willmitzer L., Rocha-Sosa M. (1990) Construction of an intron-containing marker gene: splicing of the intron in transgenic plants and its use in monitoring early events in Agrobacterium-mediated plant transformation. Mol Gen Genet. 220(2):245-50
Van West P, Kamoun S, van 't Klooster JW, Govers F (1999) Internuclear gene silencing in Phytophthora infestans. Mol Cell. Mar;3(3):339-48.
Wang Y, Dou D, Wang X, Li A, Sheng Y, Hua C, Cheng B, Chen X, Zheng X, Wang Y (2009) The PsCZF1 gene encoding a C2H2 zinc finger protein is required for growth, development and pathogenesis in Phytophthora sojae. Microb Pathog. 47(2):78-86.
Wang Y, Li A, Wang X, Zhang X, Zhao W, Dou D, Zheng X, Wang Y (2010) GPR11, a putative seven-transmembrane G protein-coupled receptor, controls zoospore development and virulence of Phytophthora sojae. Eukaryot Cell 9(2):242-50.
Yin C, Jurgenson J E, Hulbert S H (2011) Development of a Host-Induced RNAi System in the Wheat Stripe Rust Fungus Puccinia striiformis f. sp. Tritici. MPMI 24(5): 554-561. doi:10.1094/MPMI -10-10-0229. © 2011 The American Phytopathological Society
Zhang M, Wang Q, Xu K, Meng Y, Quan J, et al. (2011) Production of dsRNA Sequences in the Host Plant Is Not Sufficient to Initiate Gene Silencing in theColonizing Oomyzete Pathogen Phytophthora parasitica. PLoS ONE 6(11): e28114.
EP 1716238 (Bayer S.A.S.) METHOD FOR MODIFYING GENE EXPRESSION OF A PHYTOPATHOGENIC FUNGUS
WO 2006/070227 (Devgen N.V.) METHOD FOR DOWN-REGULATING GENE EXPRESSION IN FUNGI
WO 2009/112270 (Leibniz-Institut für Pflanzengenetik und Kulturpflanzenforschung) METHOD FOR CREATING BROAD-SPECTRUM RESISTANCE TO FUNGI IN TRANSGENIC PLANTS
US 2010/0257634 (Venganza Inc.) BIOASSAY FOR GENE SILENCING CONSTRUCTS
WO 2006/047495 (Venganza Inc.) METHODS AND MATERIALS FOR CONFERRING RESISTANCE TO PESTS AND PATHOGENS OF PLANTS

## Patentansprüche

1. Transgene Pflanze der Art *Solanum tuberosum* oder deren Teile, in deren Genom eine doppelsträngige erste DNA und eine doppelsträngige zweite DNA zur Ausbildung einer Pathogenresistenz gegenüber einem Oomyzeten der Gattung *Phytophthora* stabil integriert sind, wobei die Nukleotidsequenzen des kodierenden Stranges der ersten und zweiten DNA zueinander ganz oder teilweise invers komplementär sind, so dass ausgehend hiervon eine doppelsträngige RNA gebildet werden kann, **dadurch gekennzeichnet, dass** die erste DNA
(a) eine Nukleotidsequenz gemäß der SEQ ID NO: 1, oder
(b) ein Fragment von mindestens 25 aufeinanderfolgenden Nukleotiden einer Nukleotidsequenz gemäß der SEQ ID NO: 1, oder
(c) eine Nukleotidsequenz, die komplementär zu einer der Nukleotidsequenzen nach (a) oder (b) ist, oder
(d) eine Nukleotidsequenz, die mit einer der Nukleotidsequenzen nach (a), (b) oder (c) unter stringenten Bedingungen hybridisiert,
umfasst.

2. Transgene Pflanze nach Anspruch 1 **dadurch gekennzeichnet, dass** die Pflanze eine Resistenz gegenüber *Phytophthora infestans* aufweist.

3. Transgene Pflanze nach Anspruch 1, **dadurch gekennzeichnet, dass** es sich bei der doppelsträngigen RNA um miRNA oder siRNA handelt.

4. Transgene Pflanze nach Anspruch 1, **dadurch gekennzeichnet, dass** die erste DNA und die zweite DNA mit mindestens einem Promotor operativ verknüpft sind.

5. Teile einer Pflanze nach Anspruch 1, **dadurch gekennzeichnet, dass** es sich um Samen oder Zellen handelt.

6. Transgene Pflanze nach Anspruch 1 hergestellt durch ein Verfahren umfassend die Schritte:
i. Herstellen einer transformierten ersten Elternpflanze enthaltend eine erste doppelsträngige DNA, die stabil in das Genom der Elternpflanze integriert ist und die
(a) eine Nukleotidsequenz gemäß der SEQ ID NO: 1, oder
(b) ein Fragment von mindestens 25 aufeinanderfolgenden Nukleotiden einer Nukleotidsequenz gemäß der SEQ ID NO: 1, oder
(c) eine Nukleotidsequenz, die komplementär zu einer der Nukleotidsequenzen nach (a) oder (b) ist, oder
(d) eine Nukleotidsequenz, die mit einer der Nukleotidsequenzen nach (a), (b) oder (c) unter stringenten Bedingungen hybridisiert, umfasst;
ii. Herstellen einer transformierten zweiten Elternpflanze enthaltend eine zweite doppelsträngige DNA, die stabil in das Genom der Elternpflanze integriert ist, wobei die Nukleotidsequenzen des kodierenden Stranges der ersten und zweiten DNA zueinander ganz oder teilweise invers komplementär sind;
iii. Kreuzen der ersten Elternpflanze mit der zweiten Elternpflanze;
iv. Auswählen einer solchen Pflanze, in deren Genom die erste DNA und die zweite DNA zur Ausbildung einer Pathogenresistenz gegenüber einem Oomyzeten der Gattung Phytophthora integriert sind, so dass ausgehend hiervon eine doppelsträngige RNA gebildet werden kann.

7. Mittel zur äußeren Applikation bei Pflanzen zur Erzeugung einer Resistenz gegenüber einem Oomyzeten der Gattung *Phytophthora,* insbesondere gegenüber *Phytophthora infestans* enthaltend doppelsträngige RNA, wobei ein Strang dieser RNA dem Transkript einer doppelsträngigen DNA umfassend
(a) eine Nukleotidsequenz gemäß der SEQ ID NO: 1, oder
(b) ein Fragment von mindestens 25 aufeinanderfolgenden Nukleotiden einer Nukleotidsequenz gemäß der SEQ ID NO: 1, oder
(c) eine Nukleotidsequenz, die komplementär zu einer der Nukleotidsequenzen nach (a) oder (b) ist, oder
(d) eine Nukleotidsequenz, die mit einer der Nukleotidsequenzen nach (a), (b) oder (c) unter stringenten Bedingungen hybridisiert,
entspricht.

## Claims

1. A transgenic plant of the species *Solanum tuberosum* or parts thereof, which has a first double-stranded DNA and a second double-stranded DNA stably integrated into the genome thereof in order to provide a pathogen resistance to an oomycete of the genus *Phytophthora,* wherein the nucleotide sequences of the coding strand for the first and second DNA are complete or partial reverse complements of each other, so that a double-stranded RNA can be formed therefrom, **characterized in that** the first DNA comprises:
(a) a nucleotide sequence with SEQ ID NO: 1, or
(b) a fragment of at least 25 successive nucleotides of a nucleotide sequence according to SEQ ID NO: 1, or
(c) a nucleotide sequence which is complementary to one of the nucleotide sequences of (a) or (b), or
(d) a nucleotide sequence which hybridizes with one of the nucleotide sequences of (a), (b) or (c) under stringent conditions.

2. The transgenic plant as claimed in claim 1, **characterized in that** the plant exhibits a resistance to *Phytophthora infestans.*

3. The transgenic plant as claimed in claim 1, **characterized in that** the double-stranded RNA is miRNA or siRNA.

4. The transgenic plant as claimed in claim 1, **characterized in that** the first DNA and the second DNA are operatively linked to at least one promoter.

5. Parts of a plant as claimed in claim 1, **characterized in that** they are seeds or cells.

6. The transgenic plant as claimed in claim 1, produced by a method comprising the steps of:
(i) producing a transformed first parent plant comprising a first double-stranded DNA which is stably integrated into the genome of the parent plant and which comprises:
(a) a nucleotide sequence with SEQ ID NO: 1, or
(b) a fragment of at least 25 successive nucleotides of a nucleotide sequence with SEQ ID NO: 1, or
(c) a nucleotide sequence which is complementary to one of the nucleotide sequences of (a) or (b), or
(d) a nucleotide sequence which hybridizes with one of the nucleotide sequences of (a), (b) or (c) under stringent conditions;
(ii) producing a transformed second parent plant comprising a second double-stranded DNA which is stably integrated into the genome of the parent plant, wherein the nucleotide sequences of the coding strand for the first and second DNA are complete or partial reverse complements of each other;
(iii) crossing the first parent plant with the second parent plant;
(iv) selecting a plant that has a genome into which the first DNA and the second DNA have been integrated in order to provide a pathogen resistance to an oomycete of the genus *Phytophthora,* so that a double-stranded RNA can be formed therefrom.

7. An agent for external application to plants in order to provide a resistance to an oomycete of the genus *Phytophthora,* in particular to *Phytophthora infestans* containing double-stranded RNA, wherein a strand of said RNA corresponds to the transcript of a double-stranded DNA comprising:
(a) a nucleotide sequence with SEQ ID NO: 1, or
(b) a fragment of at least 25 successive nucleotides of a nucleotide sequence with SEQ ID NO: 1, or
(c) a nucleotide sequence which is complementary to one of the nucleotide sequences of (a) or (b), or
(d) a nucleotide sequence which hybridizes with one of the nucleotide sequences of (a), (b) or (c) under stringent conditions.

## Revendications

1. Plante transgénique de type *Solanum tuberosum* ou ses parties, dans le génome de laquelle sont intégrés de manière stable un premier ADN à double brin et un deuxième ADN à double brin, pour générer une résistance pathogénique à l'encontre d'un oomycète de l'espèce *Phytophthora,* les séquences nucléotidiques du brin codé du premier et deuxième ADN étant totalement ou en partie inversement complémentaires l'une de l'autre, de sorte à pouvoir créer à partir de là un ARN à double brin, **caractérisée en ce que** le premier ADN
(a) comprend une séquence nucléotidique selon la SEQ ID NO : 1, ou
(b) comprend un fragment d'au moins 25 nucléotides d'une séquence nucléotidique selon la SEQ ID NO : 1, ou
(c) comprend une séquence nucléotidique qui est complémentaire à l'une des séquences nucléotidiques selon (a) ou (b), ou
(d) comprend une séquence nucléotidique qui s'hybride dans des conditions stringentes avec l'une des séquences nucléotidiques selon (a), (b) ou (c).

2. Plante transgénique selon la revendication 1 **caractérisée en ce que** la plante fait preuve d'une résistance à l'encontre de *Phytophthora infestans (mildiou de la pomme de terre).*

3. Plante transgénique selon la revendication 1, **caractérisée en ce que** l'ARN à double brin est un miARN (microARN) ou un siARN (petit ARN interférent).

4. Plante transgénique selon la revendication 1, **caractérisée en ce que** le premier ADN et le deuxième ADN sont fonctionnellement liés avec au moins un promoteur.

5. Parties d'une plante selon la revendication 1, **caractérisée en ce qu'**il s'agit de semences ou de cellules.

6. Plante transgénique selon la revendication 1, fabriquée à l'aide d'un procédé comprenant les étapes :
i. de la production d'une première plante parentale transformée, contenant un premier ADN à double brin, qui est intégré de manière stable dans le génome de la plante parentale et qui
(a) comprend une séquence nucléotidique selon la SEQ ID NO : 1, ou
(b) comprend un fragment d'au moins 25 nucléotides successifs d'une séquence nucléotidique selon la SEQ ID NO : 1, ou
(c) comprend une séquence nucléotidique qui est complémentaire à l'une des séquences nucléotidiques selon (a) ou (b), ou
(d) comprend une séquence nucléotidique, qui s'hybride, dans des conditions stringentes avec l'une des séquences nucléotidiques selon (a), (b) ou (c) ;
ii. de la production d'une deuxième plante parentale transformée, contenant un deuxième ADN à double brin, qui est intégré de manière stable dans le génome de la plante parentale, les séquences nucléotidiques du brin codé du premier et deuxième ADN étant totalement ou en partie inversement complémentaires l'une de l'autre ;
iii. du croisement de la première plante parentale avec la deuxième plante parentale ;
iv. de la sélection d'une plante dans le génome de laquelle sont intégrés le premier ADN et le deuxième ADN, pour générer une résistance pathogénique à l'encontre d'un oomycète de l'espèce Phytophthora de sorte à pouvoir créer à partir de là un ARN à double brin.

7. Produit, destiné à l'application externe sur des plantes pour générer une résistance à l'encontre d'un oomycète de l'espèce *Phytophthora,* notamment à l'encontre de *Phytophthora infestans* contenant des ARN à double brin, un brin desdits ARN correspondant à la transcription d'un ADN à double brin, comprenant
(a) une séquence nucléotidique selon la SEQ ID NO : 1, ou
(b) un fragment d'au moins 25 nucléotides successifs d'une séquence nucléotidique selon la SEQ ID NO : 1, ou
(c) une séquence nucléotidique qui est complémentaire à l'une des séquences nucléotidiques selon (a) ou (b) ou
(d) une séquence nucléotidique, qui s'hybride dans des conditions stringentes avec l'une des séquences nucléotidiques selon (a), (b) ou (c).
